# EUROPEAN PATENT APPLICATION

(11) **EP 4 809 959 A1**
(43) Date of publication of application: **23.09.2026**
(21) Application number: 24911073.5
(22) Date of filing: 23.12.2024
(51) Int. Cl.: A61B 34/35, A61B 34/00, A61B 17/34

(54) **MASTER HAND CONTROL APPARATUS FOR ROBOT, PUNCTURE APPARATUS, AND ROBOT**

(30) Priority: 25.12.2023 CN 202311811277; 25.12.2023 CN 202311798581
(71) Applicant: Wuhan United Imaging Surgical Co., Ltd., Wuhan, Hubei 430206 (CN)
(72) Inventor: LU, Zhuangzhuang, Wuhan, Hubei 430206 (CN); WEI, Fang, Wuhan, Hubei 430206 (CN); ZHAI, Mingchun, Wuhan, Hubei 430206 (CN); ZHAO, Zhuo, Wuhan, Hubei 430206 (CN); YANG, Xin, Wuhan, Hubei 430206 (CN); XU, Lihong, Wuhan, Hubei 430206 (CN)
(74) Representative: Wang, Bo
(86) International application number: PCT/CN2024/141630
(87) International publication number: WO 2025/140146

(57) **Abstract**

Embodiments of the present disclosure provide a main hand control device for a robot, a puncture device, and a robot. The main hand control device includes: a puncture needle execution component, the puncture needle execution component including a rod structure; and an attitude adjustment execution component, the attitude adjustment execution component being configured to obtain an attitude of the rod structure; wherein the rod structure is capable of moving relative to the attitude adjustment execution component along an axial direction of the rod structure. The puncture device includes: a puncture needle drive module, including an advance-retreat drive mechanism, the advance-retreat drive mechanism being connected to a puncture needle; and an attitude control module, the advance-retreat drive mechanism being disposed on the attitude control module, and the advance-retreat drive mechanism being configured to drive the puncture needle to move relative to the attitude control module along an axial direction of the puncture needle. The robot includes the main hand control device and the puncture device, and the puncture needle drive module of the puncture device drives the puncture needle to move in response to a puncture execution signal from the puncture needle execution component of the main hand control device.

## Description

### CROSS-REFERENCE TO RELATED APPLICATIONS

This application claims priority to Chinese Patent Application No. 202311811277.8 and Chinese Patent Application No. 202311798581.3, filed on December 25, 2023, the contents of each of which are hereby incorporated by reference.

### TECHNICAL FIELD

The present disclosure generally relates to a field of medical equipment technology, and in particular to a main hand control device for a robot, a puncture device for a robot, and a robot.

### BACKGROUND

A master-slave teleoperation robot-assisted puncture surgery mode is a relatively advanced surgical method. By remotely operating a slave-end puncture device to perform a puncture operation through a main hand control device of a master-slave teleoperation robot, medical personnel can be effectively prevented from being exposed to radiation (e.g., X-ray radiation) during the surgical procedure. Simultaneously, real-time guidance of the puncture using medical images (e.g., CT images, MR images, or the like) allows for control over the entire puncture operation, thereby greatly improving puncture accuracy and success rate. In actual clinical operation, an operator controls an end effector to perform a puncture through the main hand control device. The closer the main hand control device is to a puncture needle structure and the closer the control of the puncture operation is to the actual puncture scenario when an operator holds the puncture needle, the higher the success rate of the puncture surgery. However, current main hand control devices cannot simulate the actual puncture scenario when an operator holds the puncture needle during the puncture surgery process, which has a certain impact on the success rate of the puncture surgery.

### SUMMARY

One or more embodiments of the present disclosure provide a main hand control device for a robot. The main hand control device includes a puncture needle execution component. The puncture needle execution component includes a rod structure. The main hand control device includes an attitude adjustment execution component. The attitude adjustment execution component is configured to obtain an attitude of the rod structure. The rod structure is capable of moving relative to the attitude adjustment execution component along an axial direction of the rod structure.

One or more embodiments of the present disclosure provide a puncture device for a robot. The puncture device includes a puncture needle drive module. The puncture needle drive module includes an advance-retreat drive mechanism. The advance-retreat drive mechanism is connected to a puncture needle. The puncture device includes an attitude control module. The advance-retreat drive mechanism is disposed on the attitude control module. The advance-retreat drive mechanism is configured to drive the puncture needle to move relative to the attitude control module along an axial direction of the puncture needle.

One or more embodiments of the present disclosure provide a robot. The robot includes the main hand control device and the puncture device. The puncture needle drive module of the puncture device drives the puncture needle to move in response to a puncture execution signal from the puncture needle execution component of the main hand control device.

### BRIEF DESCRIPTION OF THE DRAWINGS

The present disclosure is further described by way of exemplary embodiments. These exemplary embodiments are described in detail with reference to the accompanying drawings. These embodiments are not limiting. In these embodiments, identical reference numerals denote identical structures, wherein:
FIG. 1A is a structural block diagram of a main hand control device according to some embodiments of the present disclosure.
FIG. 1B is a schematic structural diagram of a main hand control device according to some embodiments of the present disclosure.
FIG. 2 is a schematic structural diagram of a connection between a puncture needle execution component and a portion of an attitude adjustment execution component according to some embodiments of the present disclosure.
FIG. 3 is a schematic diagram of the structure shown in FIG. 2 from another angle.
FIG. 4 is a schematic diagram of the structure shown in FIG. 2 from yet another angle.
FIG. 5 is an enlarged schematic structural diagram of a position detection module in FIG. 4.
FIG. 6 is a schematic diagram of the structure shown in FIG. 2 from other angles.
FIG. 7 is an enlarged schematic structural diagram of a position detection module in FIG. 6.
FIG. 8A is a schematic structural diagram of a force feedback component according to some embodiments of the present disclosure.
FIG. 8B is an enlarged schematic structural diagram of the force feedback component shown in FIG. 8A from another angle.
FIG. 8C is a detailed schematic structural diagram of the force feedback component shown in FIG. 8A.
FIG. 8D is an enlarged schematic structural diagram of a dashed box M in FIG. 8C.
FIG. 8E is an enlarged schematic structural diagram of a dashed box N in FIG. 8C.
FIG. 9 is a schematic structural diagram of a main hand control device according to other embodiments of the present disclosure.
FIG. 10 is an enlarged schematic structural diagram of a dashed box A in FIG. 9 of the present disclosure.
FIG. 11 is a schematic diagram of a working principle of a main hand control device according to some embodiments of the present disclosure.
FIG. 12 is a comparative schematic diagram of a rod structure before and after moving along a first degree of freedom according to some embodiments of the present disclosure.
FIG. 13 is a comparative schematic diagram of a rod structure before and after moving along a second degree of freedom according to some embodiments of the present disclosure.
FIG. 14 is a comparative schematic diagram of a rod structure before and after moving along a third degree of freedom according to some embodiments of the present disclosure.
FIG. 15 is another schematic structural diagram of a main hand control device according to some embodiments of the present disclosure.
FIG. 16 is a schematic diagram of a working principle of an attitude adjustment execution component according to some embodiments of the present disclosure.
FIG. 17 is a cross-sectional schematic diagram of a dashed box in FIG. 15.
FIG. 18 is a schematic diagram of the main hand control device shown in FIG. 15 from another angle.
FIG. 19 is an enlarged schematic structural diagram of a dashed box C in FIG. 18.
FIG. 20 is an enlarged schematic structural diagram of a dashed box D in FIG. 18.
FIG. 21A is a structural block diagram of a puncture device according to some embodiments of the present disclosure.
FIG. 21B is a schematic structural diagram of a puncture device according to some embodiments of the present disclosure.
FIG. 22 is an enlarged schematic structural diagram of a dashed box F in FIG. 21B.
FIG. 23 is a schematic structural diagram of a puncture device according to other embodiments of the present disclosure.
FIG. 24 is an enlarged schematic structural diagram of a dashed box G in FIG. 23.
FIG. 25 is a schematic structural diagram illustrating a connection between a puncture device and a mobile device according to some embodiments of the present specification.
FIG. 26 is a comparative schematic diagram illustrating a puncture needle before and after moving along a first degree of freedom according to some embodiments of the present specification.
FIG. 27 is a comparative schematic diagram illustrating a puncture needle before and after moving along a third degree of freedom according to some embodiments of the present specification.
FIG. 28 is a workflow diagram of a robot according to some embodiments of the present specification.

Reference Signs: 100, main hand control device; 110, puncture needle execution component; 111, rod structure; 1110, interaction handle; 1111, operation portion; 11111, operation region; 11112, first control region; 11113, first enable button; 11114, second control region; 11115, second enable button; 11116, third enable button; 11117, finger placement region; 1112, sliding portion; 1113, first limit structure; 1114, second limit structure; 112, first position detection module; 1121, first linear scale; 1122, first linear scale reading member; 1123, linear scale reading head mounting seat; 113, resistance transmission member; 1131, rack; 1132, mounting seat; 1133, first connection rope; 1134, second connection rope; 114, angle detection component; 1141, encoder; 1142, encoder reading member; 120, attitude adjustment execution component; 1211, first connecting rod; 1212, first rotating ring; 1213, second angle sensor; 1221, second connecting rod; 1222, second rotating ring; 1223, third angle sensor; 1224, second connection shaft; 1225, second support seat; 1226, second shaft end cover; 1227, third brake; 1228, second motor; 12281, second homing angle sensor; 1229, second reducer; 123, base; 125, second damper; 1251, second damper mounting seat; 126, passive degree-of-freedom connection component; 1261, passive degree-of-freedom mounting seat; 1262, first passive degree-of-freedom bearing; 1263, second passive degree-of-freedom bearing; 1264, bearing locking seat; 130, force feedback component; 131, force output unit; 1311, force feedback motor; 132, force transmission unit; 1321, coupling; 1322, gear; 1323, first angle sensor; 1324, first brake; 1325, force feedback mounting seat; 1326, pulley; 140, linear guiding component; 141, linear rail; 142, slider; 200, puncture device; 210, puncture needle drive module; 211, puncture needle; 212, rotary drive mechanism; 2121, rotary drive motor; 2122, fourth angle sensor; 2123, fourth brake; 2124, first force sensor; 213, advance-retreat drive mechanism; 2131, advance-retreat drive motor; 2132, fifth angle sensor; 2133, fifth brake; 2134, linear rail; 2135, slider; 2136, mounting seat; 2137, transmission mechanism; 21371, ball nut; 21372, ball screw; 2138, second coupling; 2139, second position detection module; 21391, second linear scale; 21392, second linear scale reading member; 2140, mounting base; 21401, slewing bearing; 220, attitude control module; 221, guiding structure; 222, attitude adjustment base; 223, attitude adjustment drive module; 2231, first passive ring; 2232, second passive ring; 2233, first coupling rod; 2234, second couping rod; 2235, first attitude adjustment drive mechanism; 2236, second attitude adjustment drive mechanism; 22361, seventh brake; 22362, second reducer; 22363, second attitude adjustment drive motor; 22364, seventh angle sensor; 2237, second attitude adjustment support seat; 2238, second attitude adjustment end cover; 2239, first attitude adjustment support seat; 300, mobile device; 310, trolley body; 320, transmission connection component; 321, telescopic portion; 322, first connection portion; 323, second connection portion; 330, caster.

### DETAILED DESCRIPTION

In order to more clearly illustrate the technical solutions of the embodiments of the present disclosure, the accompanying drawings to be used in the following description of the embodiments are briefly introduced. It is evident that the accompanying drawings in the following description are merely some examples or embodiments of the present disclosure. For a person skilled in the art, the present disclosure may also be applied to other similar scenarios based on the accompanying drawings without inventive efforts. Unless obviously obtained from the context or the context illustrates otherwise, the same numeral in the drawings refers to the same structure or operation.

It should be understood that the terms 'system', 'device', 'unit', and/or 'module' as used herein are a manner for distinguishing different components, elements, parts, portions, or assemblies at different levels. However, if other words can achieve the same purpose, then the words may be replaced by other expressions.

As shown in the present disclosure, unless the context clearly indicates otherwise, words such as 'a', 'an', 'one', and/or 'the' do not exclusively refer to the singular form, and may also include the plural form. Generally speaking, the terms 'comprising' and 'including' merely indicate the inclusion of explicitly identified steps and elements, and these steps and elements do not constitute an exhaustive list. A manner or a device may also include other steps or elements.

In recent years, medical imaging technologies (such as CT, MR, or the like) have made great progress, whether in terms of basic technologies or new clinical applications. Various components of the medical imaging technologies (such as CT, MR, or the like), such as X-ray tubes, detectors, slip rings, data acquisition systems, and algorithms, have made great progress. For example, with CT, since the advent of spiral CT and multi-slice CT, a plurality of new clinical applications have emerged, which have advantages such as fast scanning time and clear images, or the like, and may be used for examination of a plurality of diseases. After more than 30 years of development, the medical imaging technologies (such as CT, MR, or the like) have once again become one of the most exciting diagnostic approaches in the field of medical imaging. Nowadays, the medical imaging technologies (such as CT, MR, or the like) no longer exist merely as a simple imaging examination. Driven by a plurality of diversified manners, such as modern medical science continuously breaking disciplinary boundaries, mutual dependence, and joint exploration, or the like, the medical imaging technologies (such as CT, MR, or the like) also cooperate with various clinical departments to achieve a plurality of examinations and treatments, and have achieved significant medical effects. Percutaneous puncture guided by medical imaging technologies (such as CT, MR, or the like) is one of the currently widely clinically applied technologies. It refers to a technology that, under the accurate guidance of medical images, accurately punctures a puncture needle connected to a robot into a lesion in the body and obtains diseased tissue.

Puncture surgery is a manner of inserting surgical tools (such as a puncture needle, or the like) provided on a puncture device that performs a puncture operation at a distal end, into a patient's body to complete a biopsy or resection of a lesion. Traditional puncture surgeries are all blind punctures. A puncture surgery is completed by an operator based on clinical experience without accurately knowing a position of the lesion. This approach generally has a low success rate, may easily cause a plurality of injuries to a patient, and places high demands on the operator of the puncture surgery. Puncture surgery guided by medical images, under the premise of medical imaging (medical images of human tissues and puncture instruments), can determine a puncture direction in real time and make timely adjustments, thereby greatly improving a surgical success rate, reducing surgical risks, and improving a patient's recovery speed and quality of life. However, medical imaging equipment all use X-rays, gamma rays, or the like to complete work. Completing surgery on the medical imaging equipment side may expose medical staff to a radiation environment for a long time, posing a great threat to body health. Based on this, master-slave teleoperation robots came into being.

Through a master-slave teleoperation robot-assisted puncture system (hereinafter referred to as a 'robot'), a robotic arm can be remotely operated through a master operator outside a medical imaging room to complete a puncture surgery, thereby avoiding a doctor suffering X-ray radiation exposure during the surgery. Simultaneously, medical images guide the puncture surgery in real time, improving a success rate of the puncture surgery. However, some robots currently cannot simulate the actual puncture conditions when an operator holds a needle during a puncture surgery. An operator cannot experience the clinical puncture sensation, which to a certain extent reduces the success rate of the puncture surgery.

Based on the above reasons, the present disclosure provides a main hand control device that can completely simulate puncture needle insertion at a master end. The main hand control device may be disposed in an operating room, and an operator controls a puncture process of a clinical slave end and an attitude adjustment process of a puncture needle of the slave end by operating a needle handle at the master end (i.e., the rod structure described later herein), so that the operator can complete a puncture surgery under the real-time guidance of medical imaging, thereby improving a success rate of the puncture. In some embodiments, the main hand control device includes an attitude adjustment execution component and a puncture needle execution component, wherein the attitude adjustment execution component is configured to allow an operator to adjust an attitude of a puncture needle, so that the puncture needle can advance along a correct path. The puncture needle execution component is configured to execute a puncture process after an operator determines the attitude of the puncture needle, wherein the puncture needle will follow a movement of a needle handle at a master end of the puncture needle execution component of the main hand control device, thereby completing the entire process of master-slave attitude adjustment and puncture, and achieving master-slave teleoperation puncture surgery guided by medical images. It has been found that a relatively large difference in structure and size between a main hand control device and an end effector (For example, a puncture needle) may affect a puncture effect. Therefore, in embodiments of the present disclosure, a structure of a needle handle at a master end is set to be closer to an actual puncture needle structure at a slave end. When an operator holds the needle handle at the master end for puncture or attitude adjustment, it can simulate actual puncture conditions when an operator holds a puncture needle during a puncture surgery, allowing the operator to experience the clinical puncture sensation as much as possible, and improving a success rate of the puncture surgery.

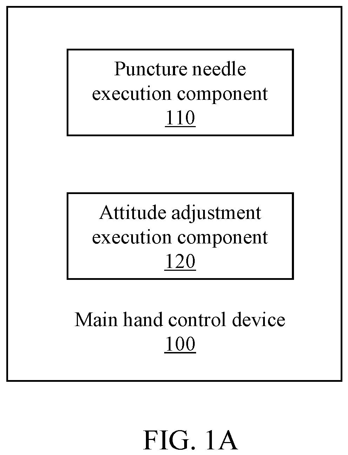

In some embodiments, referring to FIGs. 1A-5, a main hand control device 100 includes a puncture needle execution component 110 and an attitude adjustment execution component 120. The puncture needle execution component 110 includes a rod structure 111. The attitude adjustment execution component 120 is configured to obtain an attitude of the rod structure 111. The rod structure 111 can move relative to the attitude adjustment execution component 120 along an axial direction of the rod structure 111.

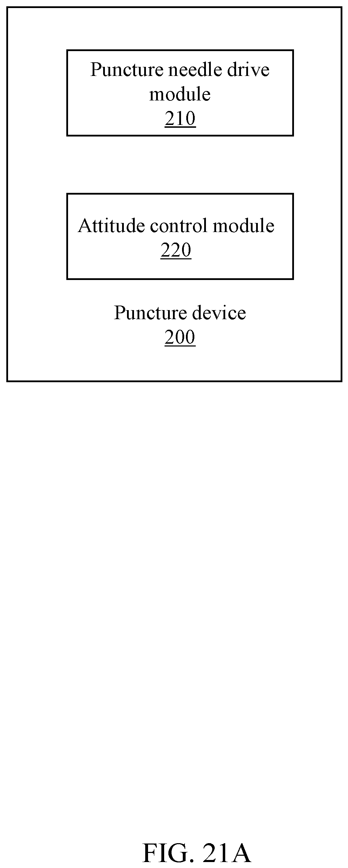

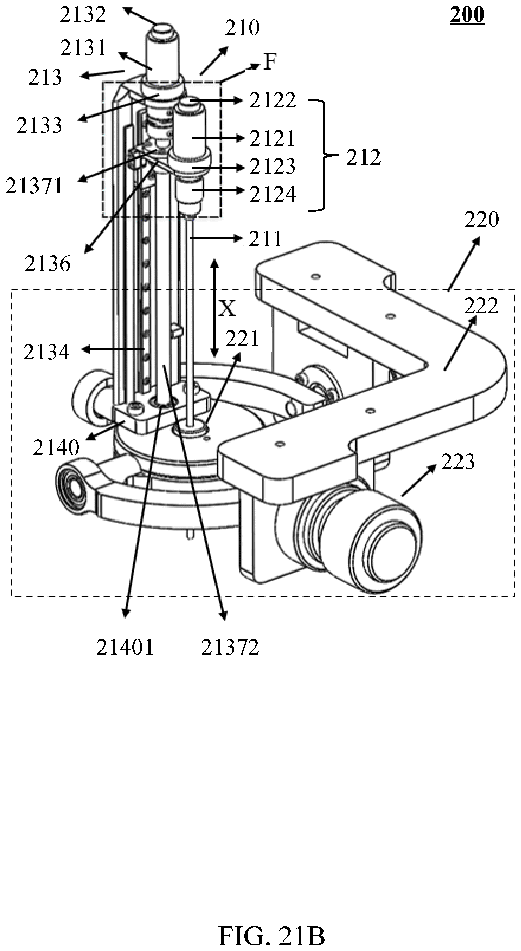

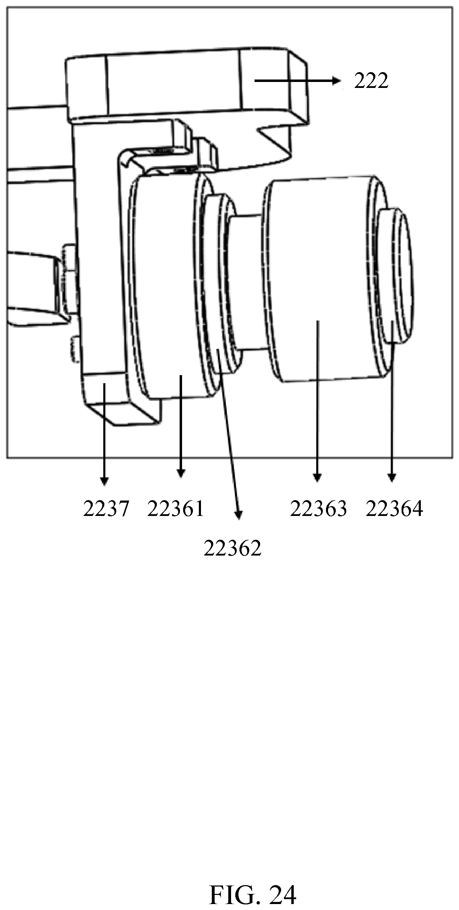

The puncture needle execution component 110 is a main structure of the main hand control device 100 for controlling a puncture needle to perform an advance operation or a retreat operation. The advance operation refers to a puncture needle puncturing into a patient's body. The retreat operation refers to a puncture needle retreating from a patient's body. In some embodiments, the main hand control device 100 can communicate with/be connected to a processor of a robot (not shown in the figures). When the puncture needle execution component 110 moves, a movement of the puncture needle execution component 110 can be fed back to the processor in real time, and then the processor can control the robot to drive a puncture needle to perform a puncture operation based on the movement of the puncture needle execution component 110. For relevant content regarding the puncture needle, please refer to relevant descriptions in FIGs. 21A-24 later herein.

The attitude adjustment execution component 120 is a main structure of the main hand control device 100 for adjusting an attitude of a puncture needle. Obtaining the attitude of the rod structure 111 refers to obtaining rotation angle information of an axial direction of the rod structure 111 relative to the attitude adjustment execution component 120. Merely by way of example, when an attitude of a puncture needle needs to be adjusted, an operator may control the rod structure 111 to swing relative to the attitude adjustment execution component 120, wherein the attitude adjustment execution component 120 can detect rotation angle information of the rod structure 111 relative to the attitude adjustment execution component 120 (For example, a rotation angle of a first connecting rod 1211 relative to a base 123 described later herein, and a rotation angle of a second connecting rod 1221 relative to the base 123 described later herein), and feed the rotation angle information back to the processor of the robot. The processor can adjust an attitude of a puncture needle based on the rotation angle information of the rod structure 111 relative to the attitude adjustment execution component 120, to achieve a purpose of adjusting the attitude of the puncture needle, so that the puncture needle can align with a target puncture target, ensuring an accuracy of a puncture operation.

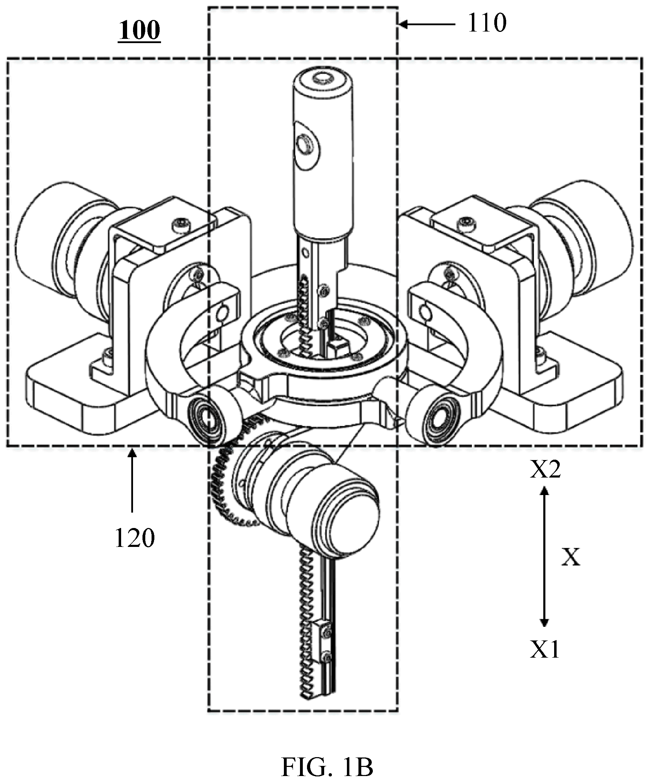

The rod structure 111 is a structure of the main hand control device 100 for an operator to hold and operate. The rod structure 111 may move along the axial direction of the rod structure 111 for an operator to perform an advance operation or a retreat operation, thereby realizing control of a linear movement of a puncture needle, and may swing relative to the attitude adjustment execution component 120 for the operator to perform attitude adjustment, thereby realizing control of an attitude of the puncture needle. The rod structure 111 can perform a linear movement, after which the linear movement is fed back to the processor of the robot, the processor can control a puncture needle to perform an advance operation based on a distance of the linear movement of the rod structure 111, so that the puncture needle can smoothly penetrate a target puncture target. After a puncture surgery is completed, the main hand control device 100 performs a reverse movement of the advance operation, to realize a puncture needle retreating from a patient's body, wherein a principle thereof is substantially the same as the advance operation. In some embodiments, an axial direction of the rod structure 111 may be represented by an arrow X in FIG. 1B. When the rod structure 111 moves downwards along a direction of the arrow X (For example, along a direction X1 in FIG. 1B), a puncture needle performs an advance operation. When the rod structure 111 moves upwards along the direction of the arrow X (For example, along a direction X2 in FIG. 1B), the puncture needle performs a retreat operation. In some cases, since a shape of the rod structure 111 will be closer to an actual shape of a puncture needle, it can ensure that when an operator holds the rod structure 111 to move along its axial direction, it can better simulate a linear movement generated when holding the puncture needle during a puncture surgery, thereby improving puncture accuracy.

It should be noted that a working process of the main hand control device 100 provided in the present disclosure may include a puncture process and an attitude adjustment process. In the puncture process, the rod structure 111 moves along its own axial direction. In the attitude adjustment process, the rod structure 111 swings relative to the attitude adjustment execution component 120. The terms 'during puncture' and 'puncture mode' mentioned in the present disclosure both correspond to the puncture process, and the terms 'during attitude adjustment' and 'attitude adjustment mode' mentioned in the present disclosure both correspond to the attitude adjustment process. In some embodiments, the puncture process and the attitude adjustment process are decoupled from each other, meaning that attitude adjustment cannot be realized during the puncture process, and puncture cannot be realized during the attitude adjustment process, to ensure safety of a patient.

It also needs to be noted that a distance of linear movement of a puncture needle and a distance of linear movement of the rod structure 111 may have a proportional mapping relationship. For example, a ratio of the distance of linear movement of the puncture needle to the distance of linear movement of the rod structure 111 may be 1:1, 1:1.2, 1:1.5, 1:2, 2:1, or 1.5:1, or the like. In some embodiments, a ratio of the distance of linear movement of the puncture needle to the distance of linear movement of the rod structure 111 is 1:1, so that an operator can control a puncture needle to move a preset distance when operating the rod structure 111 to output a preset distance, allowing the operator to experience the clinical puncture sensation as much as possible, improving an operating experience of the operator, and increasing a success rate of the puncture surgery.

In some embodiments, the main hand control device 100 further includes a first signal transmission component (not shown in the figures), wherein the first signal transmission component communicates with/is connected to the puncture needle execution component 110 and the attitude adjustment execution component 120. The first signal transmission component can receive various signals fed back by the puncture needle execution component 110 and the attitude adjustment execution component 120 (For example, rotation angle information mentioned above or below, and puncture force feedback information mentioned below), and output corresponding control signals based on the received signals, to meet use requirements of different scenarios (For example, an attitude adjustment scenario and a puncture scenario).

In some embodiments, the first signal transmission component may communicate with/be connected to the processor of the robot, for establishing signal transmission between the main hand control device 100 and the robot, and realizing information interaction between the main hand control device 100 and the robot. In some embodiments, the first signal transmission component may include, but is not limited to, Ethernet, a serial port, wireless, a CAN bus, an EtherCAT bus, or the like. For example, the first signal transmission component can realize information interaction through Ethernet.

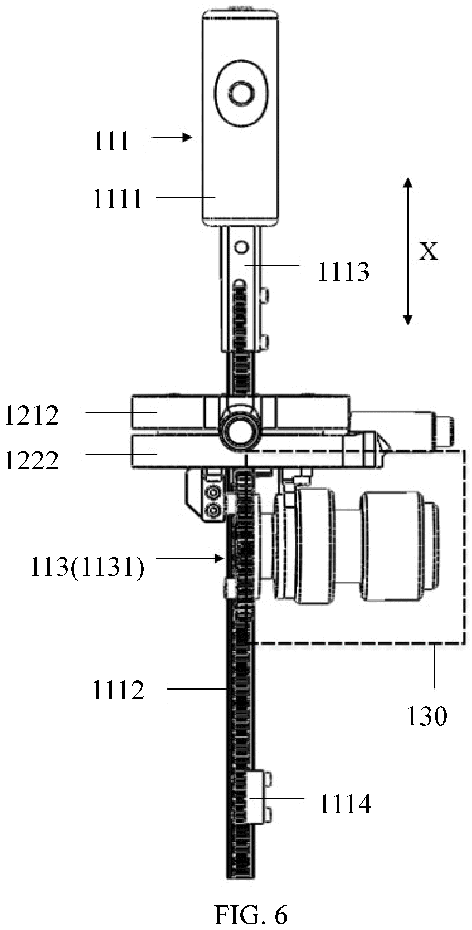

In some embodiments, the main hand control device 100 further includes a force feedback component 130, wherein the force feedback component 130 is configured to apply a puncture motion resistance along the axial direction of the rod structure 111 to the rod structure 111, to provide an operator with force feedback simulating puncture of a puncture needle during master-slave teleoperation, and further simulate a linear movement generated when a doctor holds a needle for puncture during a puncture surgery, to make an operation process safer and more efficient, and can improve puncture accuracy. In some embodiments, the force feedback component 130 can obtain puncture force feedback information of a puncture needle, and then apply a motion resistance to the rod structure 111 based on the puncture force feedback information of the puncture needle. For relevant descriptions of the force feedback component, reference may be made to FIGs. 6-7 and embodiments thereof.

In some embodiments, the main hand control device 100 further includes a resistance transmission member 113 disposed on the rod structure 111, wherein the force feedback component 130 applies a puncture motion resistance to the rod structure 111 via the resistance transmission member 113. For example, the resistance transmission member 113 may include a sliding portion 1112 disposed on the rod structure 111. The resistance transmission member 113 may be connected to the force feedback component 130, and is configured to transmit a puncture motion resistance generated by the force feedback component 130 along the axial direction of the rod structure 111 to the rod structure 111.

In some embodiments, referring to FIGs. 6-7, the force feedback component 130 includes a force output unit 131 and a force transmission unit 132, wherein the force transmission unit 132 is drivingly connected to the resistance transmission member 113, and the force output unit 131 outputs a puncture motion resistance based on puncture force feedback information during puncturing of a puncture needle. The force output unit 131 refers to a member that outputs a motion resistance. The force transmission unit 132 refers to a member that transmits the motion resistance output by the force output unit 131 to the resistance transmission member 113. Puncture force feedback information refers to resistance information received by a puncture needle advancing or retreating, when the rod structure 111 is performing puncture (performing linear movement along its own axial direction), wherein the puncture needle is disposed on a puncture device. For example, the resistance received by the puncture needle from a patient's tissue during the process of the puncture needle puncturing into the patient's tissue. In some embodiments, the resistance received by the puncture needle can be obtained by a force sensor disposed on the puncture needle (For example, a first force sensor described later herein), and then fed back to the force output unit 131 via a signal transmission component (such as the first signal transmission component and a second signal transmission component). The force output unit 131 then outputs a puncture motion resistance to the rod structure 111 via the resistance transmission member 113, the puncture motion resistance being equivalent to the resistance received by the puncture needle during a puncture process. In this manner, when an operator performs a puncture operation, the operator can feel the resistance received by a puncture needle through the puncture motion resistance fed back by the force feedback component 130, to truly simulate a scenario of holding a needle for puncture. In some embodiments, the force output unit 131 may include a motor (For example, a force feedback motor 1311 described later herein), a hydraulic cylinder, a pneumatic cylinder, or the like.

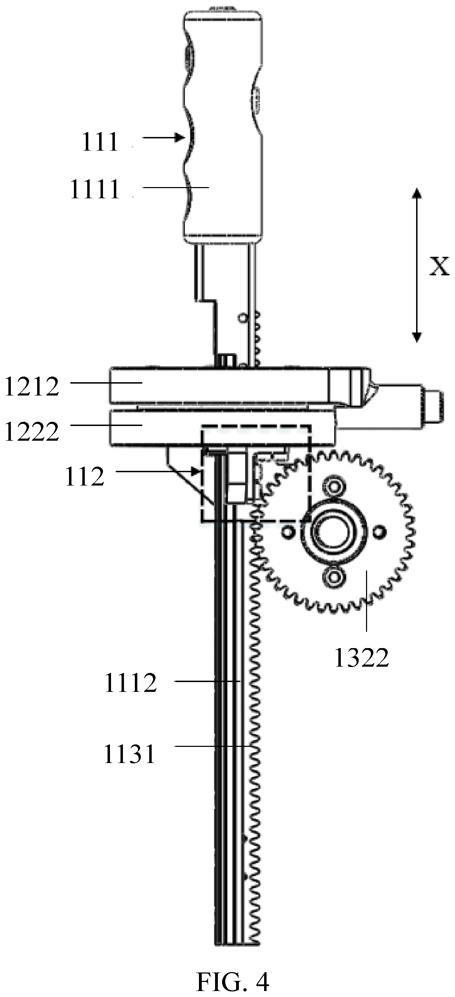

In some embodiments, a specific structure of the resistance transmission member 113 may be adjusted based on a specific structure of the force feedback component 130. For example, the resistance transmission member 113 may include a worm (wherein a corresponding force transmission unit 132 may include a worm wheel meshing with the worm), a rack (wherein a corresponding force transmission unit 132 may include a gear meshing with the rack), a connection rope (wherein a corresponding force transmission unit 132 may include a pulley wound by the connection rope), or a synchronous belt (wherein a corresponding force transmission unit 132 may include a belt pulley meshing with the synchronous belt), or the like. For more details about the resistance transmission member 113 and the force feedback component 130, reference may be made to descriptions of FIGs. 4-5 and embodiments thereof later herein.

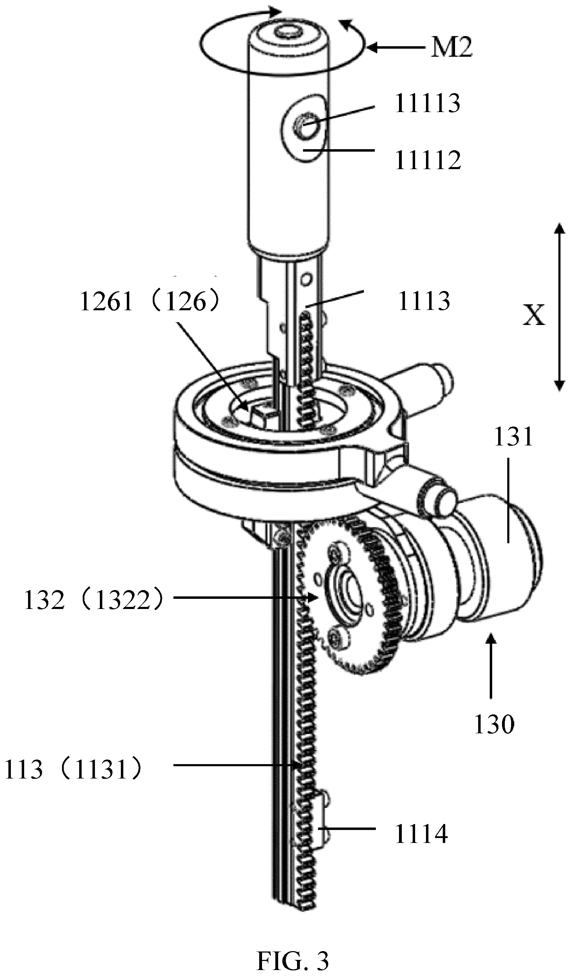

In some embodiments, as shown in FIGs. 3-7, the force output unit 131 includes a force feedback motor 1311, and the force transmission unit 132 includes a gear 1322 connected to an output end of the force feedback motor 1311. The resistance transmission member 113 may include a rack 1131 meshing with the gear. The rack 1131 is disposed on the rod structure 111, and an arrangement direction of the rack 1131 is parallel to the axial direction of the rod structure 111.

When the rod structure 111 moves along its own axial direction, the rack 1131 moves relative to the gear 1322. After the force feedback motor 1311 outputs a torque, the torque can hinder the relative rotation between the gear 1322 and the rack 1131, thereby causing the operator to feel a motion resistance. The greater the torque generated by the force feedback motor 1311, the greater the torque required for the gear 1322 to rotate, and the more significant the motion resistance felt by the operator when holding the rod structure 111. The smaller the torque generated by the force feedback motor 1311, the smaller the torque required for the gear 1322 to rotate, and the weaker the motion resistance felt by the operator.

In some embodiments, the force transmission unit 132 further includes a coupling 1321. The coupling 1321 is fixedly connected to an output shaft of the force feedback motor 1311 and the gear 1322, respectively. The torque output by the force feedback motor 1311 is the source of the motion resistance. The coupling 1321 connects the output shaft of the force feedback motor 1311 to the rotating shaft of the gear 1322, thereby transmitting the output torque of the force feedback motor 1311 to the gear 1322.

In this embodiment, the force feedback motor 1311 can generate a torque based on puncture force feedback information. The generated torque can be transmitted to the gear 1322 through the coupling 1321. The torque can then be transmitted from the gear 1322 to the resistance transmission member 113, and finally, the torque can be transmitted to the rod structure 111 through the resistance transmission member 113, which is connected to the rod structure 111. This enables the operator to feel a motion resistance along the axial direction of the rod structure 111 when holding the rod structure 111.

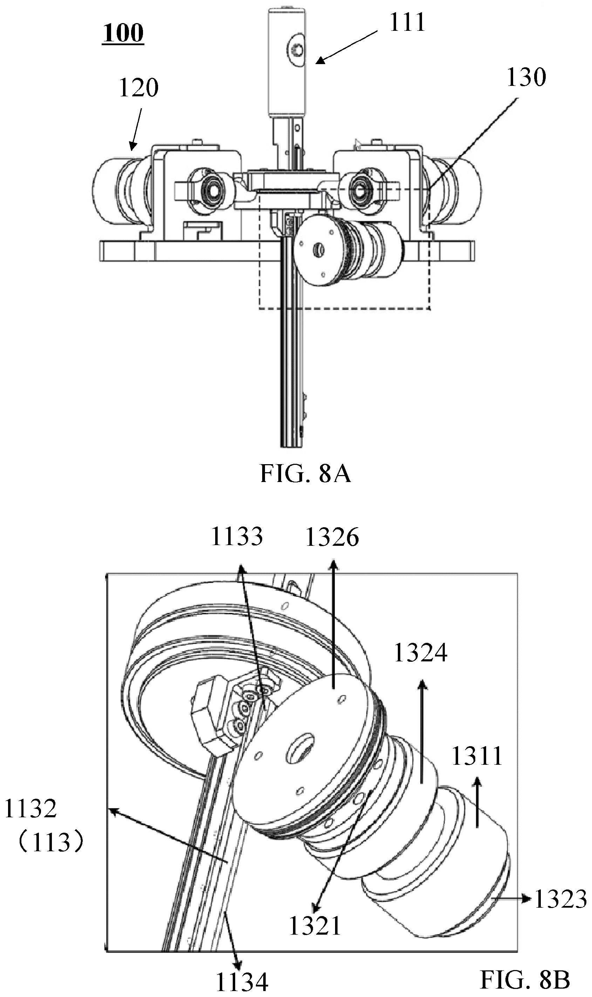

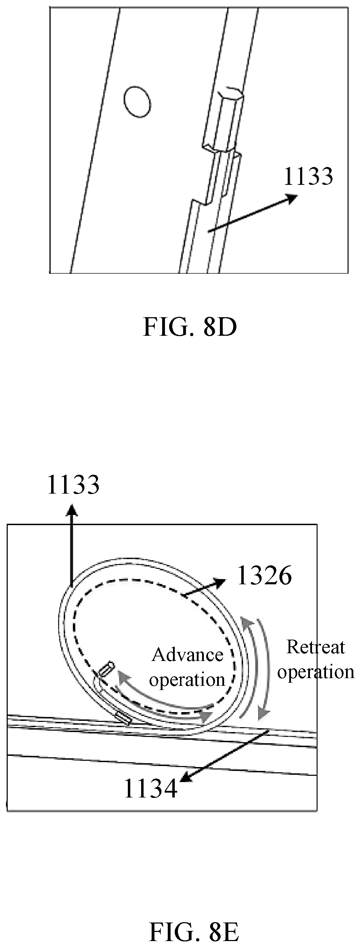

In some embodiments, as shown in FIGs. 8A-8E, the force output unit 131 includes a force feedback motor 1311, and the force transmission unit 132 includes a pulley 1326 connected to an output end of the force feedback motor 1311. The resistance transmission member 113 includes a mounting seat 1132, a first connection rope 1133, and a second connection rope 1134. The mounting seat 1132 is provided with two mounting portions, and the two mounting portions are spaced apart along a direction parallel to the axial direction of the rod structure 111. One end of the first connection rope 1133 is connected to one of the mounting portions. The first connection rope 1133 is wound around the pulley 1326, and another end of the first connection rope 1133 is fixed on the pulley 1326. One end of the second connection rope 1134 is connected to another of the mounting portions. The second connection rope 1134 is wound around the pulley 1326, and another end of the second connection rope 1134 is fixed on the pulley 1326. The first connection rope 1133 and the second connection rope 1134 are wound on the pulley 1326 in opposite directions.

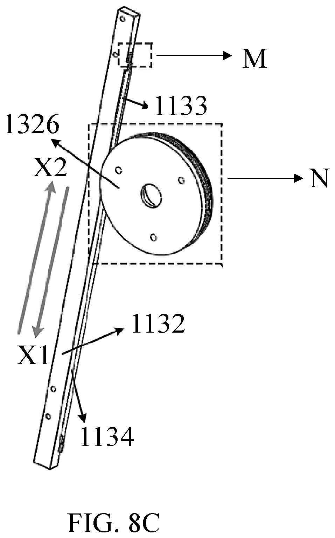

The mounting seat 1132 is a base of the resistance transmission member 113 for mounting connection ropes (e.g., the first connection rope 1133 and the second connection rope 1134). As shown in FIG. 8D, one end of the first connection rope 1133 and the second connection rope 1134 may be snap-connected to the mounting seat 1132. In other embodiments, one end of the first connection rope 1133 and the second connection rope 1134 may be bolted or adhesively bonded to the mounting seat 1132. In some embodiments, the mounting seat 1132 may be a strip-shaped structure, wherein an extension direction of the strip-shaped structure is parallel to the axial direction of the rod structure 111. In this case, the two mounting portions are respectively disposed at the two ends of the strip-shaped structure. The pulley 1326 is configured to wind the connection ropes (including the first connection rope 1133 and the second connection rope 1134). In some embodiments, the first connection rope 1133 and the second connection rope 1134 may be steel wire ropes. Illustratively, as shown in FIG. 8C, when a puncture insertion operation is performed (the mounting seat 1132 moves towards the X1 direction in FIG. 8C), the first connection rope 1133 is wound into the pulley 1326, and the second connection rope 1134 is unwound from the pulley 1326. When a puncture retraction operation is performed (the mounting seat 1132 moves towards the X2 direction in FIG. 8C), the first connection rope 1133 is unwound from the pulley 1326, and the second connection rope 1134 is wound into the pulley 1326.

When the rod structure 111 moves along its own axial direction, the mounting seat 1132 moves relative to the pulley 1326. The first connection rope 1133 and the second connection rope 1134 are accordingly wound into or unwound from the pulley 1326, thereby driving the pulley 1326 to rotate. After the force feedback motor 1311 outputs a torque, the torque can hinder the rotation of the pulley 1326, thereby hindering the first connection rope 1133 and the second connection rope 1134 from being accordingly wound into or unwound from the pulley 1326. This hinders the movement of the mounting seat 1132 relative to the pulley 1326, thereby causing the operator to feel a motion resistance. The greater the torque generated by the force feedback motor 1311, the greater the torque required for the pulley 1326 to rotate, and the more significant the motion resistance felt by the operator when holding the rod structure 111. The smaller the torque generated by the force feedback motor 1311, the smaller the torque required for the pulley 1326 to rotate, and the weaker the motion resistance felt by the operator.

In some embodiments, the coupling 1321 may also be fixedly connected to the output shaft of the force feedback motor 1311 and the pulley 1326, respectively, thereby transmitting the output torque of the force feedback motor 1311 to the pulley 1326. In this embodiment, the force feedback motor 1311 can generate a torque based on puncture force feedback information. The generated torque can be transmitted to the pulley 1326 through the coupling 1321. Then, the torque can be transmitted to the resistance transmission member 113 through the connection ropes (including the first connection rope 1133 and the second connection rope 1134). Finally, the torque can be transmitted to the rod structure 111 through the resistance transmission member 113, which is connected to the rod structure 111. This enables the operator to feel a motion resistance along the axial direction of the rod structure 111 when holding the rod structure 111.

The structure in which the pulley 1326 cooperates with the connection ropes (including the first connection rope 1133 and the second connection rope 1134) has no transmission backlash compared to the gear-rack structure. This enables more accurate transmission of the motion resistance generated by the force feedback motor, allowing the operator to more accurately feel a motion resistance equivalent to the resistance experienced by a puncture needle during a puncture operation, thereby enhancing the control and perception of the puncture process.

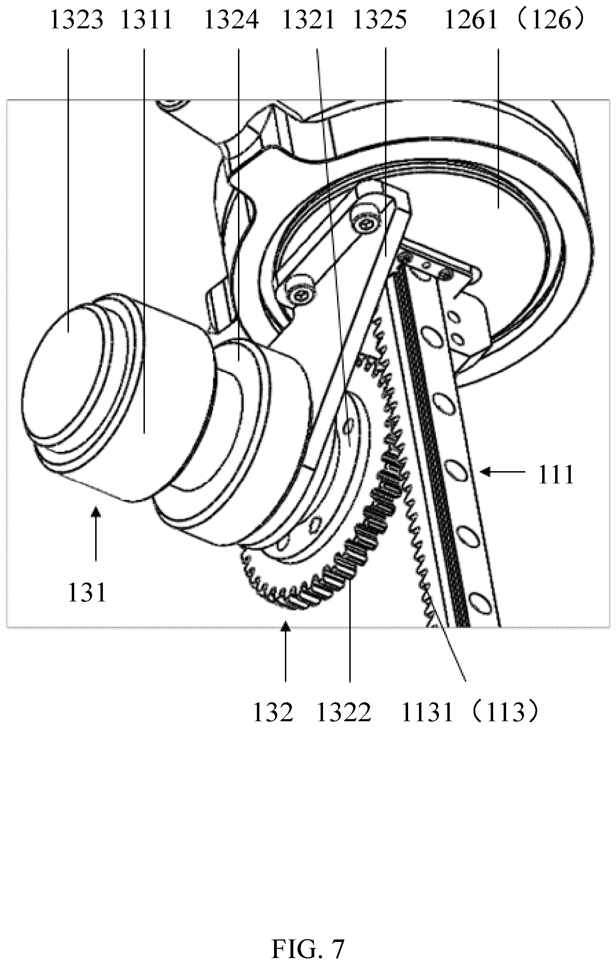

In some embodiments, as shown in conjunction with FIG. 1B, FIG. 6, FIG. 7, and FIG. 8B, the resistance transmission member 113 is fixedly connected to the rod structure 111, the force feedback component 130 further includes a first brake 1324, the first brake 1324 is fixedly connected to the attitude adjustment execution component 120, and the first brake 1324 is drivingly connected to the force transmission unit 132, the first brake 1324 is configured to restrict movement of the rod structure 111 along its own axial direction by controlling the force transmission unit 132 to be fixed relative to the resistance transmission member 113. Illustratively, the first brake 1324 may include a friction brake, a magnetic powder brake, a hysteresis brake, a water eddy current brake, or the like. Merely by way of example, the first brake 1324 may be a hysteresis brake, the hysteresis brake is connected to the output shaft of the force feedback motor 1311 and the coupler 1321, the hysteresis brake may generate a braking torque, the braking torque may be transmitted to the gear 1322 (or the pulley 1326) through the coupler 1321, thereby hindering rotation of the gear 1322 (or the pulley 1326), and thereby causing the gear 1322 (or the pulley 1326) to be fixed relative to the resistance transmission member 113 (i.e., the rack 1131 or the mounting seat 1132). Since the resistance transmission member 113 is fixedly connected to the rod structure 111, and the first brake 1324 is fixedly connected to the attitude adjustment execution component 120, when the gear 1322 (or the pulley 1326) is fixed relative to the rack 1131 (or the mounting seat 1132), the rod structure 111 is fixed relative to the attitude adjustment execution component 120. In some embodiments, as shown in FIG. 7, the force feedback component 130 further includes a force feedback mounting seat 1325, the first brake 1324 is fixedly connected to the attitude adjustment execution component 120 through the force feedback mounting seat 1325 (for example, the passive degree-of-freedom mounting seat 1261 of the attitude adjustment execution component 120, more details of the passive degree-of-freedom mounting seat 1261 may be referred to in descriptions of other embodiments of the present disclosure).

In some embodiments, the braking torque generated by the hysteresis brake is positively correlated with a current of the hysteresis brake, when the current in the hysteresis brake is larger, the generated braking torque is larger, and when the current in the hysteresis brake is smaller, the generated braking torque is smaller.

When it is desired to lock the puncture degree of freedom of the puncture needle, i.e., to prevent the puncture needle from advancing or retreating, the current of the hysteresis brake may be adjusted to a maximum value, thereby preventing rotation of the gear 1322 (or the pulley 1326) through the generated braking torque, and thereby preventing movement of the resistance transmission member 113 (i.e., the rack 1131 or the mounting seat 1132) and the rod structure 111 relative to the gear 1322 (or the pulley 1326). When unlocking the puncture degree of freedom of the puncture needle, i.e., allowing the puncture needle to advance or retreat, the current of the hysteresis brake may be adjusted to a minimum value, at this time, an influence of the generated braking torque on rotation of the gear 1322 (or the pulley 1326) may be small or negligible, therefore, the resistance transmission member 113 and the rod structure 111 can move relative to the gear 1322 (or the pulley 1326).

In some embodiments, as shown in conjunction with FIG. 4 and FIG. 7, the force feedback component 130 further includes a first angle sensor 1323, the first angle sensor 1323 is configured to detect a rotation angle of an output shaft of the force feedback motor 1311. Since the output shaft of the force feedback motor 1311 is connected to the gear 1322 (or the pulley 1326) through the coupler 1321, and the rod structure 111 needs to move along its own axial direction, which causes the resistance transmission member 113 (i.e., the rack 1131 or the mounting seat 1132) to move relative to the gear 1322 (or the pulley 1326), therefore, the first angle sensor 1323 can detect a rotation angle of the gear 1322 (or the pulley 1326) by detecting the rotation angle of the output shaft of the force feedback motor 1311, and further determine a distance moved by the resistance transmission member 113, and finally determine a position of the rod structure 111 along its own axial direction. In some embodiments, the first angle sensor 1323 may be an incremental encoder.

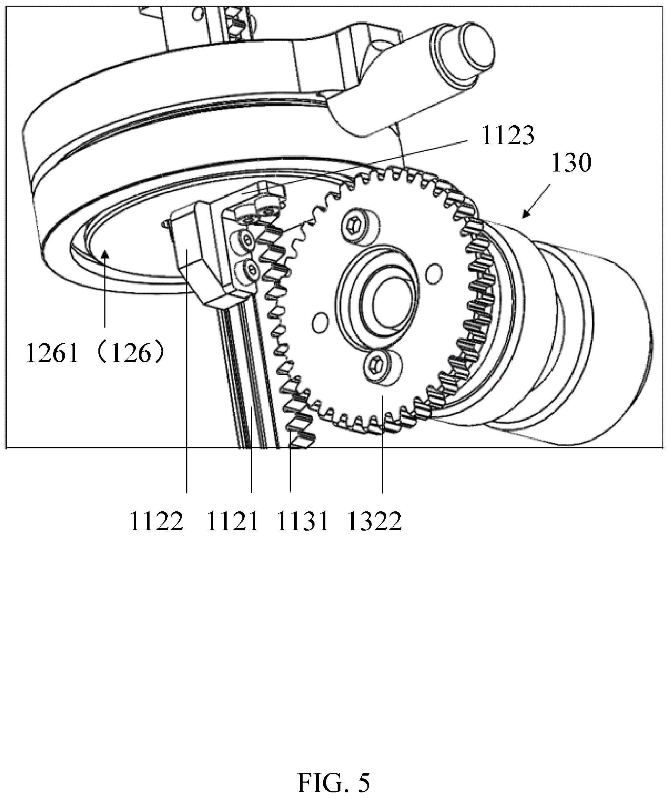

In some embodiments, as shown in FIG. 4 and FIG. 5, the puncture needle execution component 110 further includes a first position detection module 112, the first position detection module 112 is configured to obtain a position of the rod structure 111 along its own axial direction.

Illustratively, the first position detection module 112 may include a laser rangefinder, a displacement encoder, a linear scale, an inductive displacement sensor, or the like. In some embodiments, the first position detection module 112 may be arranged at any feasible position of the puncture needle execution component 110 and/or the attitude adjustment execution component 120. For example, on the rod structure 111 of the puncture needle execution component 110 or on the passive degree-of-freedom connection component 126 of the attitude adjustment execution component 120 (specific descriptions of which may be found below), or the like.

Through the first position detection module 112, the position of the rod structure 111 along its own axial direction can be accurately detected, which can further help an operator to flexibly control a puncture depth of the puncture needle, thereby realizing advance-retreat control of the puncture needle under master-slave teleoperation, and improving puncture safety.

The first angle sensor 1323 and the first position detection module 112 in the foregoing embodiments can both be used to obtain the position of the rod structure 111 along its own axial direction. In some embodiments, after setting the first position detection module 112, the first angle sensor 1323 may not need to be set. In other embodiments, after setting the first angle sensor 1323, the first position detection module 112 may also not be set. In still other embodiments, both the first position detection module 112 and the first angle sensor 1323 are set. Position data of the rod structure 111 along its own axial direction determined based on the first angle sensor 1323 and position data of the rod structure 111 along its own axial direction determined based on the first position detection module 112 in the foregoing embodiments may be subjected to data processing operations such as weighting and comparison, to further determine the position of the rod structure 111 along its own axial direction.

In some embodiments, as shown in conjunction with FIG. 4 and FIG. 5, the first position detection module 112 includes a first linear scale 1121 and a first linear scale reading member 1122, the first linear scale 1121 is disposed on the rod structure 111 along the axial direction of the rod structure 111, and the first linear scale reading member 1122 is disposed on the attitude adjustment execution component 120. In some embodiments, the first position detection module 112 is a linear scale. The first linear scale 1121 is a ruler grating, the first linear scale reading member 1122 includes a light source, a lens, an indicator grating, or the like. When a light source is used to illuminate the ruler grating in parallel, Moire fringes may appear within the first linear scale reading member 1122, when the first linear scale 1121 and the first linear scale reading member 1122 move relative to each other, the first linear scale reading member 1122 can read a grating scale by detecting a quantity of the Moire fringes and convert it into an electrical signal, and then position information can be determined through determination. Merely by way of example, the first linear scale 1121 may be fixedly connected to the rack 1131 (or the mounting seat 1132) of the puncture needle execution component 110, the rack 1131 or the mounting seat 1132 may be connected to the force feedback component 130 (specific descriptions of which may be found in related embodiments of the force feedback component 130 above), a linear scale reader mounting seat 1123 is disposed at the bottom of the attitude adjustment execution component 120, and the first linear scale reading member 1122 is connected to the attitude adjustment execution component 120 through the linear scale reader mounting seat 1123. When the operator controls the rod structure 111 to perform a puncture needle advance-retreat simulation action of a main operating end (i.e., the main hand control device 100), the rack 1131 (or the mounting seat 1132) can drive the first linear scale 1121 to move, the first linear scale reading member 1122 can read position information of the first linear scale 1121, thereby determining the position of the rod structure 111 along its own axial direction. It can be understood that, through cooperation of the first linear scale 1121 and the first linear scale reading member 1122, the position of the rod structure 111 along its own axial direction can be determined more accurately.

In some embodiments, the rod structure 111 is capable of rotating about its own axial direction. Illustratively, as shown in FIG. 1B to FIG. 3, the rod structure 111 is capable of rotating along an M2 direction shown in FIG. 3. With such a setting, the rod structure 111 can not only move along the axial direction of the rod structure 111 for an operator to perform an advance action or a retreat action, but also can rotate about the axial direction of the rod structure 111 during puncturing or attitude adjustment. During puncturing, control of linear motion and rotary motion of the puncture needle can be realized; during attitude adjustment, the operator can more conveniently swing the rod structure 111 relative to the attitude adjustment execution component 120, thereby realizing control of an attitude of the puncture needle.

In some cases, since a shape of the rod structure 111 is closer to an actual shape of the puncture needle, it enables that when the operator holds the rod structure 111 to move along its axial direction or rotate about its axial direction, linear motion or rotary motion generated when holding the puncture needle during a puncture operation can be better simulated, thereby improving puncture accuracy and puncture efficiency.

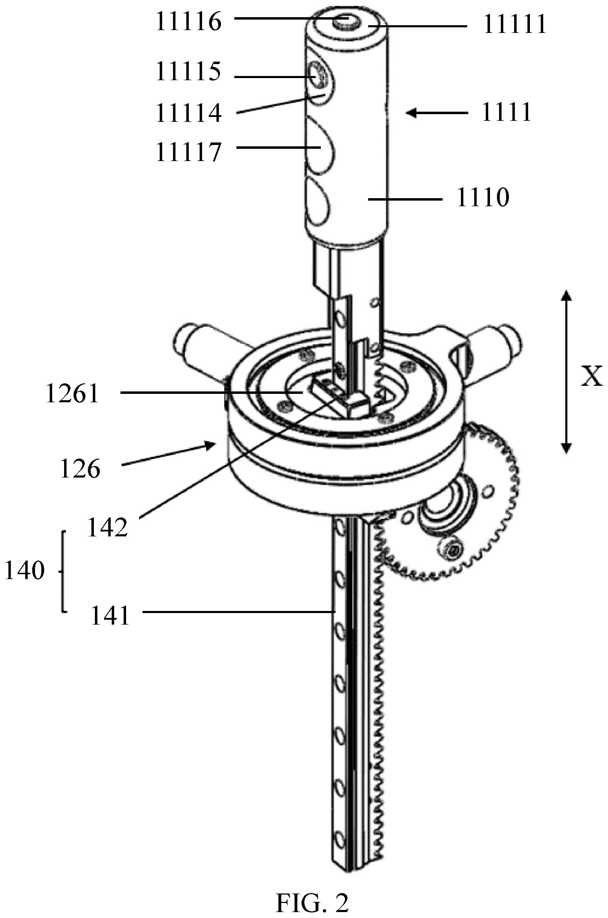

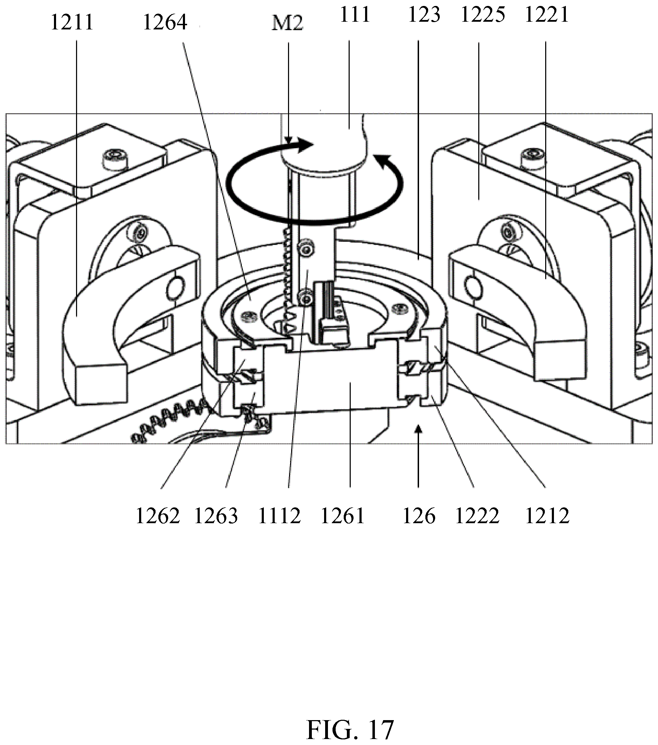

In some embodiments, the attitude adjustment execution component 120 includes a passive degree-of-freedom connection component 126, the rod structure 111 is connected to the attitude adjustment execution component 120 through the passive degree-of-freedom connection component 126, the rod structure 111 is capable of sliding along its own axial direction relative to the passive degree-of-freedom connection component 126 and the attitude adjustment execution component 120, and the rod structure 111 is capable of rotating about its own axial direction relative to the attitude adjustment execution component 120 through the passive degree-of-freedom connection component 126, thereby enabling the rod structure 111 to rotate about its own axial direction relative to the attitude adjustment execution component 120. More details about the passive degree-of-freedom connection component 126 may be referred to in the descriptions below regarding FIG. 2 and FIG. 17 and their embodiments in the present disclosure.

In some embodiments, rotation of the rod structure 111 about its own axial direction is configured not to affect movement of the puncture needle. That is to say, even if the operator controls the rod structure 111 to rotate about its own axial direction, it will not control the puncture needle to rotate about its own axial direction. During attitude adjustment of the rod structure 111 by the operator, there may be a rotation demand for the rod structure 111 relative to the attitude adjustment execution component 120 at a certain angle, so that the rod structure 111 can adapt to a gripping posture of the operator during attitude adjustment by the operator, thereby improving human-machine interaction comfort, with such a setting, while satisfying the human-machine interaction comfort, it can also avoid unexpected damage to a patient caused by rotation of the puncture needle during attitude adjustment.

In some embodiments, rotation of the rod structure 111 about its own axial direction is configured to be capable of controlling a puncture needle of a puncture device to correspondingly rotate about an axis of the puncture needle. That is to say, when the rod structure 111 rotates about its own axial direction, it will control the puncture needle to correspondingly rotate about its own axial direction. With such a setting, the operator can control the rod structure 111 not only to move along its own axial direction but also to rotate about its own axial direction during puncturing, so that the puncture needle arranged on the puncture device can advance while rotating, thereby realizing an active skin penetration operation under a master-slave mode, and further avoiding bending or even breaking of the puncture needle during puncturing, also avoiding a phenomenon of unexpected damage to a patient, and also improving puncture efficiency.

In some embodiments, during attitude adjustment performed by the main hand control device 100, rotation of the rod structure 111 about its own axial direction may be configured not to affect movement of the puncture needle. In some embodiments, during puncturing performed by the main hand control device 100, rotation of the rod structure 111 about its own axial direction may be configured to affect movement of the puncture needle. In some embodiments, the main hand control device 100 may include a rotation switching control member, the rotation switching control member may control whether rotation of the rod structure 111 about its own axial direction is capable of controlling a puncture needle of a puncture device to rotate about an axial direction of the puncture needle. The rotation switching control member may be a selection switching button, a rotation switching knob, or the like. Merely by way of example, when the rotation switching button is not pressed, rotation of the rod structure 111 about its own axial direction is not capable of controlling a puncture needle of a puncture device to rotate about an axial direction of the puncture needle; when the rotation switching button is pressed, rotation of the rod structure 111 about its own axial direction is capable of controlling a puncture needle of a puncture device to rotate about an axial direction of the puncture needle.

In some embodiments, the main hand control device 100 includes a rotation control member, the rotation control member is used to control the puncture needle of the puncture device 200 to rotate about its own axial direction. When it is necessary for the puncture needle to rotate about its own axial direction, so that the puncture needle can advance while rotating, control can be performed by triggering the rotation control member, thereby realizing an active skin penetration operation under a master-slave mode. The rotation control member may be a rotation control button, a rotation control knob, or the like. In some embodiments, when the rotation control member controls the puncture needle to rotate about its own axial direction, a drive structure for controlling a puncture degree of freedom on the puncture device (such as an advance-retreat drive motor and a rotary drive motor described below) is started. The advance-retreat drive motor is powered on, and is given a certain control current, the control current of the advance-retreat drive motor is proportional to a 'force between the puncture needle and a human body', so that the operator can feel a feedback force during an actual puncturing process on the main hand control device 100, thereby realizing a force feedback process; a drive structure for controlling an attitude adjustment degree of freedom on the puncture device (such as a sixth brake and a seventh brake described below) is locked, for example, the sixth brake and the seventh brake maintain a low level, and attitude adjustment actions cannot be performed. At this time, the puncture needle rotates about its own axial direction, realizing an active skin penetration function, the puncture needle may advance during puncturing or may not advance during puncturing when rotating about its own axial direction. If puncturing and advancing is desired, the puncture degree of freedom brake (such as the fifth brake described below) is at a high level, and the advance-retreat drive motor described below is capable of driving the puncture needle to move along its own axial direction to perform puncturing; if puncturing and advancing is not desired, the puncture degree of freedom brake (such as the fifth brake described below) is at a low level, and the puncture needle cannot move along its own axial direction, but can only rotate about its own axial direction. It should be noted that the level setting of the drive structure for controlling the attitude adjustment degree of freedom and the puncture degree of freedom brake described above is merely by way of example. For example, it may be that when the sixth brake and the seventh brake maintain a high level, attitude adjustment operations cannot be performed. For example, it may be that when the fifth brake maintains a low level, the advance-retreat drive motor described below is capable of driving the puncture needle to move along its own axial direction to perform puncturing.

An operator can control when the puncture needle rotates through the rotation control member, thereby realizing precise control of the puncturing operation. This helps to improve the accuracy and success rate of the puncturing operation.

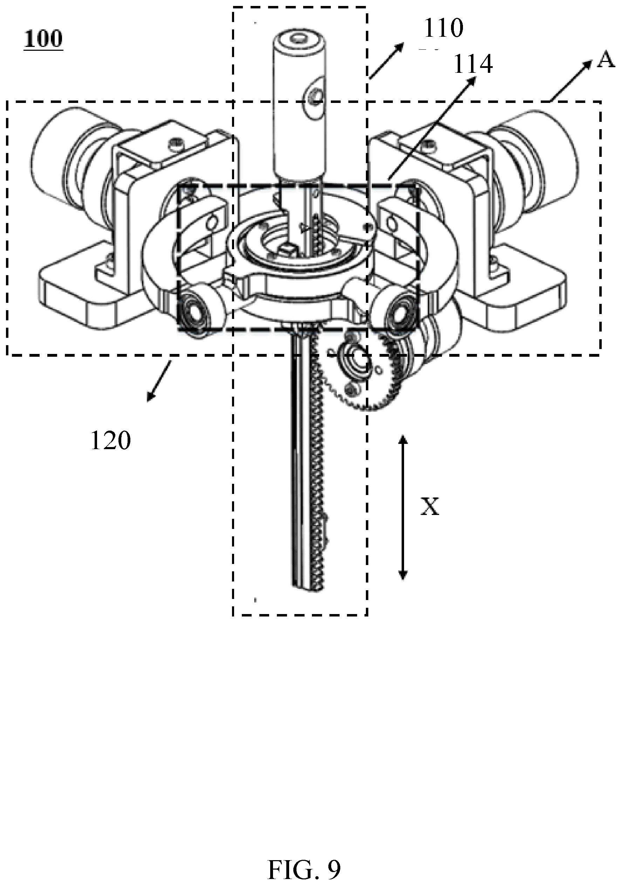

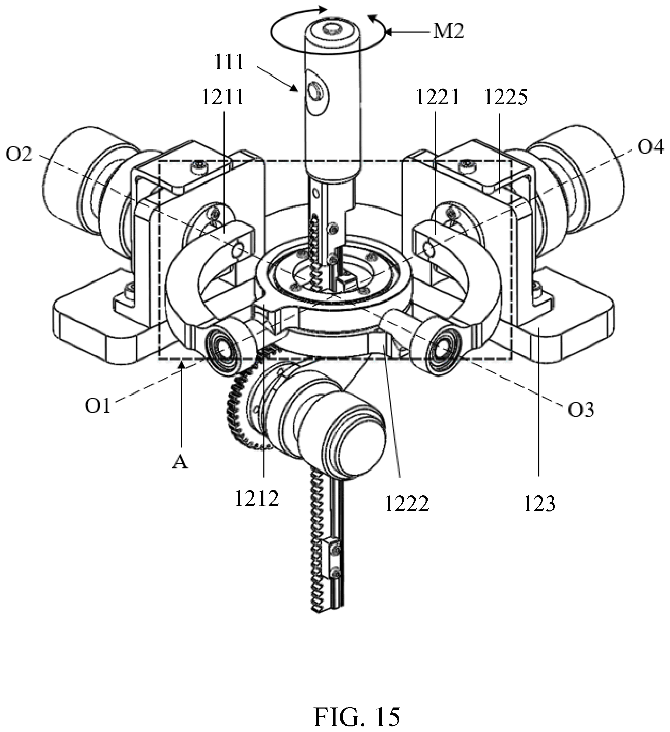

In some embodiments, as shown in FIG. 9, the puncture needle execution component 110 includes an angle detection component 114, and the angle detection component 114 is configured to obtain a rotation angle of the rod structure 111 rotating about its own axial direction. An exemplary angle detection component 114 may include a rotary potentiometer, a Hall sensor, a rotary transformer, or the like. In some embodiments, the angle detection component 114 may be disposed on a structural member arranged coaxially with the puncture needle execution component 110. For example, on the rod structure 111 of the puncture needle execution component 110 or on the first rotating ring 1212 of the attitude adjustment execution component 120, or the like. For a specific description of the first rotating ring 1212, reference may be made to FIGs. 15-18 and related description thereof.

It can be understood that, by providing the angle detection component 114, a rotation angle of the rod structure 111 rotating about its own axial direction can be obtained in real time.

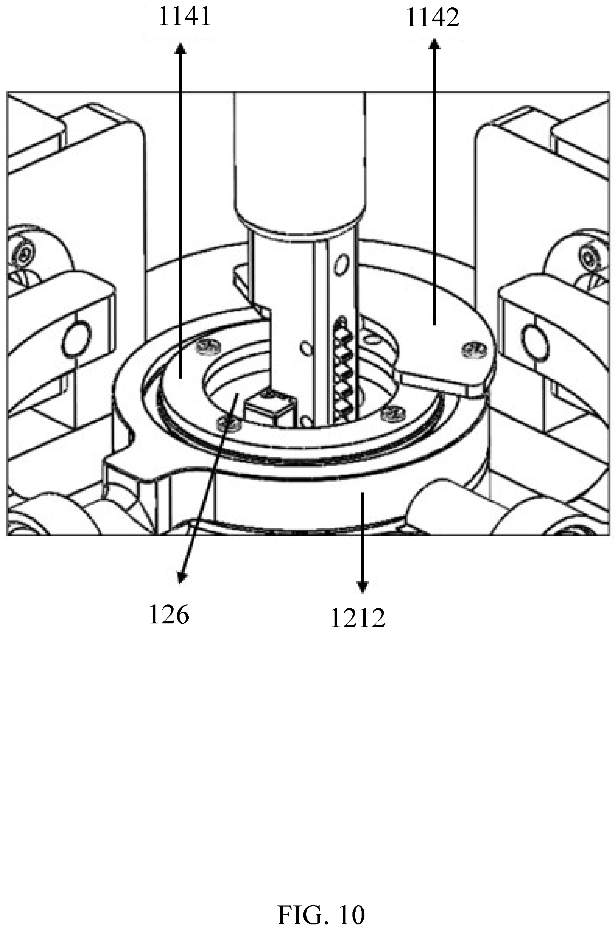

In some embodiments, in conjunction with FIGs. 9-10, the angle detection component 114 includes an encoder 1141 and an encoder reading member 1142, the encoder 1141 is disposed on the rod structure 111 and rotates synchronously with the rod structure 111, and the encoder reading member 1142 is disposed on the attitude adjustment execution component 120.

In some embodiments, the angle detection component 114 is a magnetic ring encoder. The encoder 1141 is a magnetic ring, which is typically made of a magnetic material and has a certain magnetic field strength. The encoder reading member 1142 is typically composed of a Hall element or a magnetoresistive sensor, for measuring a change in a magnetic field. When the encoder 1141 and the encoder reading member 1142 move relative to each other, the encoder reading member 1142 can sense a change in the magnetic field and convert it into an electrical signal, and a rotation angle information can be determined based on the change in the magnetic field. In some embodiments, the encoder 1141 is disposed on the passive degree-of-freedom connection component 126 (such as the passive degree-of-freedom mounting seat 1261 shown in FIG. 5), the encoder reading member 1142 is disposed on the attitude adjustment execution component 120 (such as the first rotating ring 1212 shown in FIGs. 4-6), and the rod structure 111 drives the passive degree-of-freedom connection component 126 to rotate synchronously to drive the encoder 1141 to rotate synchronously. Merely by way of example, the encoder 1141 may be fixedly connected to the passive degree-of-freedom mounting seat 1261 (such as by screw connection, or the like), and the encoder reading member 1142 may be fixedly connected to the first rotating ring 1212. When the operator controls the rod structure 111 to rotate about its own axial direction, by movement of the encoder 1141 relative to the encoder reading member 1142, the encoder reading member 1142 can read rotation information of the encoder 1141, thereby determining a rotation angle of the rod structure 111 rotating about its own axial direction. In some embodiments, the encoder reading member 1142 may be disposed on the passive degree-of-freedom connection component 126, and the encoder 1141 may be disposed on the attitude adjustment execution component 120. By the rod structure 111 driving the passive degree-of-freedom connection component 126 to rotate synchronously, the encoder reading member 1142 moves relative to the encoder 1141, and the encoder reading member 1142 can read rotation information of the encoder 1141, thereby determining a rotation angle of the rod structure 111 rotating about its own axial direction. In some embodiments, the attitude adjustment execution component 120 may include an encoder connecting member (not shown in the figures), and the encoder connecting member is rotatably connected to the passive degree-of-freedom connection component 126 and fixedly connected to the rod structure 111. The encoder reading member 1142 may be disposed on the attitude adjustment execution component 120. By the rod structure 111 driving the encoder connecting member to rotate synchronously, the encoder reading member 1142 moves relative to the encoder 1141, and the encoder reading member 1142 can read rotation information of the encoder 1141, thereby determining a rotation angle of the rod structure 111 rotating about its own axial direction.

It can be understood that, since a magnetic ring encoder has advantages such as high accuracy, good repeatability, and strong reliability, the angle detection component 114, by adopting the magnetic ring encoder, can more accurately determine a rotation angle of the rod structure 111 rotating about its own axial direction. It should be noted that the angle detection component 114 may also adopt other angle measuring instruments or devices such as an optical encoder, or the like, as long as they can satisfy actual detection requirements.

In some embodiments, the rod structure 111 is disposed on the attitude adjustment execution component 120, and the rod structure 111 has a puncture degree of freedom and an attitude adjustment degree of freedom relative to the attitude adjustment execution component 120. The puncture degree of freedom is a degree of freedom allowing a position change of the rod structure 111 along the axial direction of the rod structure 111. That is to say, the degree of freedom of the rod structure 111 moving relative to the attitude adjustment execution component 120 along its own axial direction is the puncture degree of freedom. The attitude adjustment degree of freedom is a degree of freedom allowing a change in the axial direction of the rod structure 111. That is to say, the degree of freedom of the axial direction of the rod structure 111 changing relative to the attitude adjustment execution component 120 is the attitude adjustment degree of freedom. It should be noted that the attitude adjustment degree of freedom may include two swing degrees of freedom allowing the rod structure 111 to swing about two different directions (such as two mutually orthogonal directions) (corresponding to the first degree of freedom and the second degree of freedom described below). In some embodiments, to ensure the safety of a puncturing operation, when the puncture degree of freedom is allowed, the attitude adjustment degree of freedom is locked; and when the attitude adjustment degree of freedom is allowed, the puncture degree of freedom is locked. In some embodiments, the rod structure 111 may also have a passive degree of freedom relative to the attitude adjustment execution component 120. The passive degree of freedom is a degree of freedom allowing the rod structure 111 to rotate about its own axial direction.

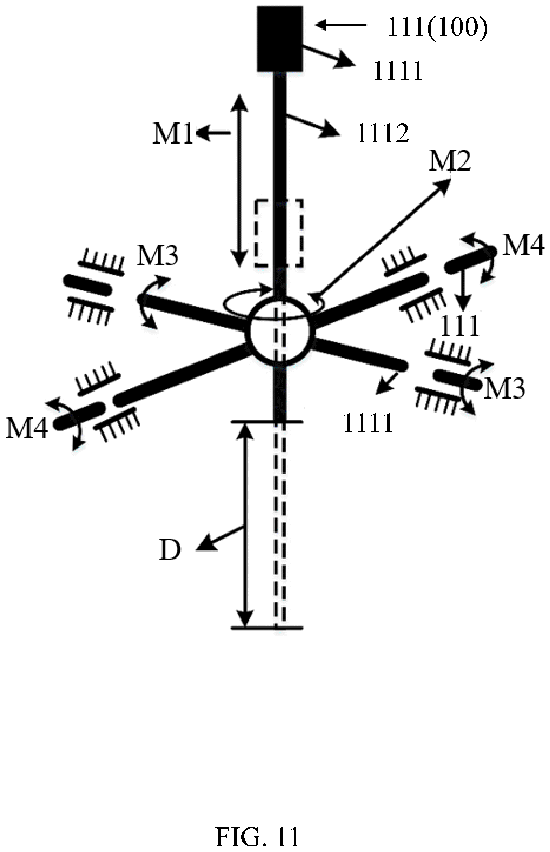

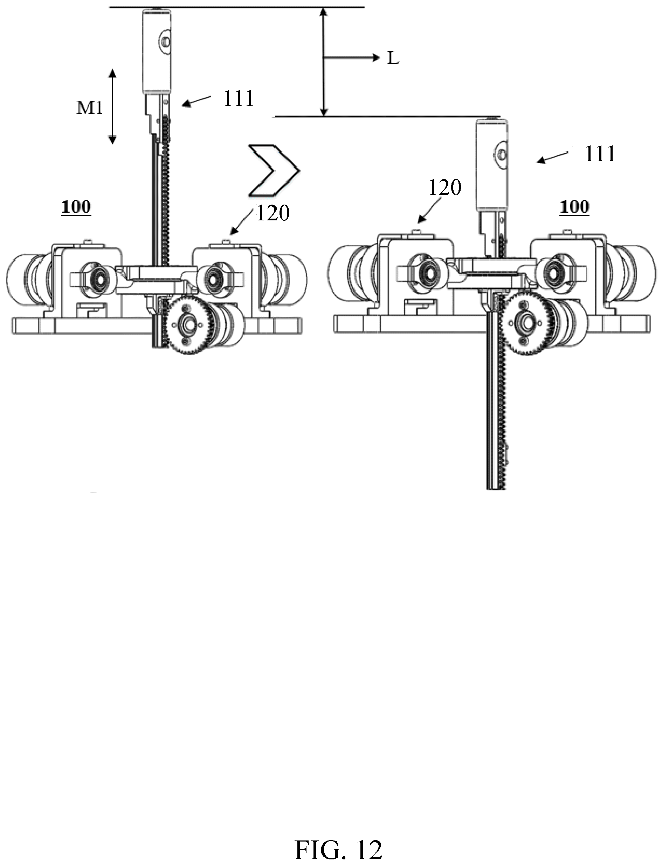

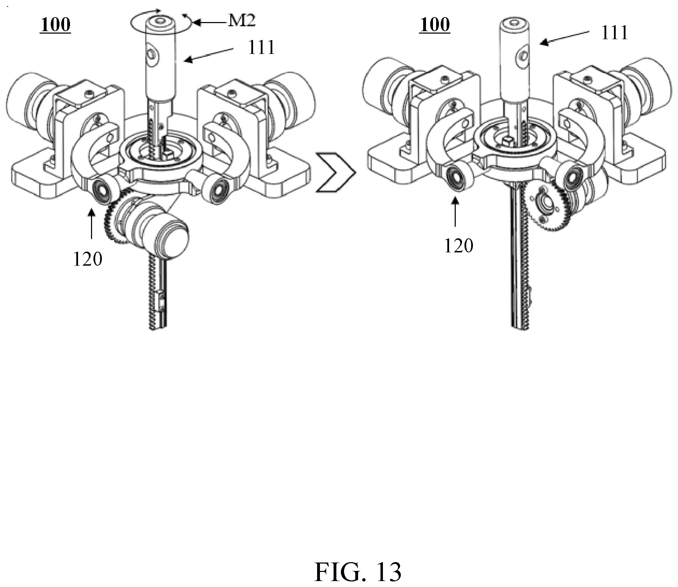

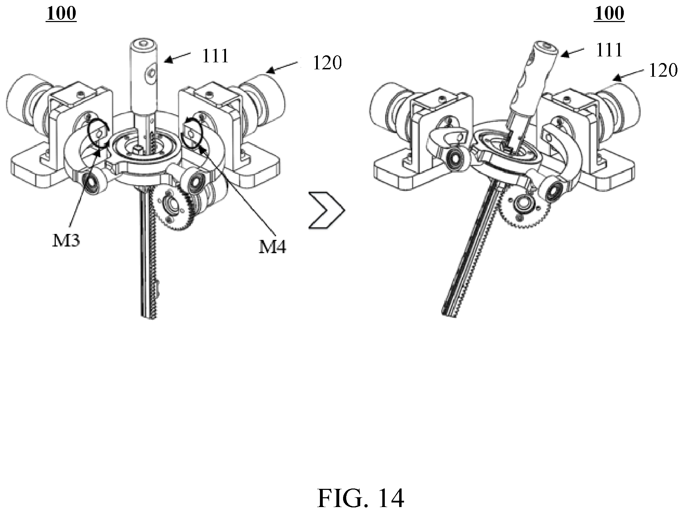

To more clearly illustrate the working principle of the main hand control device 100, FIG. 11 illustrates a schematic diagram of the working principle of the main hand control device 100. In conjunction with FIGs. 1B and 11, the main hand control device 100 provided by embodiments of the present disclosure may be a master-slave attitude incremental mapping four-degree-of-freedom force feedback main operating device. The four degrees of freedom include two rotational degrees of freedom of the attitude adjustment degree of freedom (i.e., the first degree of freedom and the second degree of freedom described below), the puncture degree of freedom, and the passive degree of freedom. A movement of the operator on the main hand control device 100 will be mapped to the puncture needle at a robot end, thereby controlling the puncture needle. By way of example, the rod structure 111 may move relative to the attitude adjustment execution component 120 along its own axial direction (e.g., move along an arrow X direction in FIG. 1B), that is, has a puncture degree of freedom (as shown by an arrow M1 in FIG. 11), and a distance of movement of the rod structure along the puncture degree of freedom is L. For example, in the embodiment shown in FIG. 12, a sequence from left to right is that the rod structure 111 performs puncturing relative to the attitude adjustment execution component 120 along the puncture degree of freedom, and a puncture depth is L. The rod structure 111 can rotate about its own axis through a passive degree-of-freedom mounting seat (e.g., the passive degree-of-freedom mounting seat 1261 in FIG. 17), that is, has the passive degree of freedom (as shown by an arrow M2 in FIG. 11). For example, in the embodiment shown in FIG. 13, a sequence from left to right is that the rod structure 111 rotates relative to the attitude adjustment execution component 120 along a rotational degree of freedom. By way of example, the rod structure 111 can swing relative to the attitude adjustment execution component 120 in a plane where a first rotation axis (e.g., O1 shown in FIG. 15) and a fourth rotation axis (e.g., O4 shown in FIG. 15) are located, that is, has the first degree of freedom (as shown by an arrow M3 in FIG. 11). The rod structure 111 can swing relative to the attitude adjustment execution component 120 in a plane where a second rotation axis (e.g., O2 shown in FIG. 15) and a third rotation axis (e.g., O3 shown in FIG. 15) are located, that is, has the second degree of freedom (as shown by an arrow M4 in FIG. 11). For example, in the embodiment shown in FIG. 14, a sequence from left to right is that the rod structure 111 swings relative to the attitude adjustment execution component 120 along the first degree of freedom and the second degree of freedom.

In some embodiments, the attitude adjustment execution component 120 is configured to apply an attitude adjustment resistance under the attitude adjustment degree of freedom to the rod structure 111 based on attitude adjustment force feedback information during attitude adjustment of the puncture needle.

The attitude adjustment force feedback information refers to resistance information received by the puncture needle disposed on the puncture device corresponding to an attitude adjustment operation when the rod structure 111 performs the attitude adjustment operation. For example, during the attitude adjustment of the puncture needle, the resistance received by the puncture needle from human tissue. In some embodiments, the resistance received by the puncture needle may be detected and obtained by a force sensor (such as the second force sensor described below) disposed on the puncture device, and then fed back to the attitude adjustment execution component 120 through a signal transmission component (such as the first signal transmission component described above and the second signal transmission component described below), and the attitude adjustment execution component 120 outputs an attitude adjustment resistance to the rod structure 111 that is equivalent to the resistance received during the attitude adjustment process of the puncture needle. Based on the description of FIG. 11 above, the attitude adjustment degree of freedom of the rod structure 111 may include two swing degrees of freedom, therefore, the attitude adjustment resistance output by the attitude adjustment execution component 120 to the rod structure 111 may include resistance torques respectively output for the two swing degrees of freedom.

With such a setting, when the operator performs an attitude adjustment operation, the operator can feel the resistance received during the attitude adjustment of the puncture needle through the attitude adjustment resistance fed back by the attitude adjustment execution component 120, to truly simulate a situation of holding the needle for attitude adjustment.

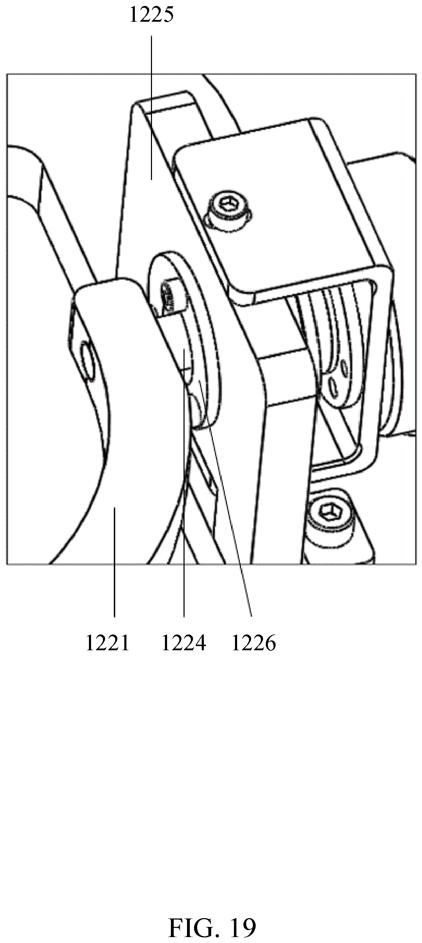

In some embodiments, referring to FIGs. 15-20, the attitude adjustment execution component 120 includes a first connecting rod 1211, a first rotating ring 1212, a second connecting rod 1221, a second rotating ring 1222, a second angle sensor 1213, a third angle sensor 1223, and a base 123. The first rotating ring 1212 and the second rotating ring 1222 are coaxial and arranged surrounding the rod structure 111. One end of the first connecting rod 1211 is rotatably connected to the first rotating ring 1212, another end of the first connecting rod 1211 is rotatably connected to the base 123, and the second angle sensor 1213 is further disposed at the another end of the first connecting rod 1211. One end of the second connecting rod 1221 is rotatably connected to the second rotating ring 1222, another end of the second connecting rod 1221 is rotatably connected to the base 123, and the third angle sensor 1223 is further disposed at the another end of the second connecting rod 1221. The second angle sensor 1213 is configured to detect an angle of rotation of the another end of the first connecting rod 1211 relative to the base 123. The third angle sensor 1223 is configured to detect an angle of rotation of the another end of the second connecting rod 1221 relative to the base 123.

The base 123 is configured to support other components of the attitude adjustment execution component 120. During attitude adjustment, the base 123 always remains stationary. For convenience of description, an axis of rotation of the first connecting rod 1211 relative to the first rotating ring 1212 may be referred to as a first rotation axis (as shown as O1 in FIG. 15), and an axis of rotation of the first connecting rod 1211 relative to the base 123 may be referred to as a second rotation axis (as shown as O2 in FIG. 15). An axis of rotation of the second connecting rod 1221 relative to the second rotating ring 1222 may be referred to as a third rotation axis (as shown as O3 in FIG. 15), and an axis of rotation of the second connecting rod 1221 relative to the base 123 may be referred to as a fourth rotation axis (as shown as O4 in FIG. 15). In some embodiments, the first rotation axis O1, the second rotation axis O2, the third rotation axis O3, and the fourth rotation axis O4 may be located in the same plane. In some embodiments, the first rotation axis O1, the second rotation axis O2, the third rotation axis O3, and the fourth rotation axis O4 may not be located in the same plane. Merely by way of example, since the base 123 always remains stationary, the axis of rotation of the first connecting rod 1211 relative to the base 123 (i.e., the second rotation axis O2) and the axis of rotation of the second connecting rod 1221 relative to the base 123 (i.e., the fourth rotation axis O4) always remain unchanged, while the axis of rotation of the first connecting rod 1211 relative to the first rotating ring 1212 (i.e., the first rotation axis O1) and the axis of rotation of the second connecting rod 1221 relative to the second rotating ring 1222 (i.e., the third rotation axis O3) may change with the movement of the first rotating ring 1212 and the second rotating ring 1222, respectively, in this case, the first rotation axis O1, the second rotation axis O2, the third rotation axis O3, and the fourth rotation axis O4 may not be located in the same plane. For example, the second rotation axis O2 and the fourth rotation axis O4 are located in the same plane, and the first rotation axis O1 and the third rotation axis O3 are located in the same plane. For example, the first rotation axis O1 and the fourth rotation axis O4 are located in the same plane, and the second rotation axis O2 and the third rotation axis O3 are located in the same plane.

In the present embodiment, since the first rotating ring 1212 and the second rotating ring 1222 are coaxially arranged surrounding the rod structure 111, the rod structure 111 always maintains a coaxial relationship with the first rotating ring 1212 and the second rotating ring 1222, that is, the axial direction of the rod structure 111 coincides with the central axis of the first rotating ring 1212 and the central axis of the second rotating ring 1222. Therefore, when the rod structure 111 swings relative to the base 123, it can drive the first rotating ring 1212 and the second rotating ring 1222 to swing relative to the base 123. Since the first connecting rod 1211 is disposed between the first rotating ring 1212 and the base 123, the swing of the first rotating ring 1212 relative to the base 123 will drive the first connecting rod 1211 to rotate about the second rotation axis O2. Similarly, since the second connecting rod 1221 is disposed between the second rotating ring 1222 and the base 123, the swing of the second rotating ring 1222 relative to the base 123 will drive the second connecting rod 1221 to rotate about the fourth rotation axis O4. The second angle sensor 1213 and the third angle sensor 1223 can detect the rotation angle of the first connecting rod 1211 rotating about the second rotation axis O2 and the rotation angle of the second connecting rod 1221 rotating about the fourth rotation axis O4, respectively, thereby determining the attitude of the rod structure 111 based on the rotation angles, so as to adjust the attitude of the puncture needle.

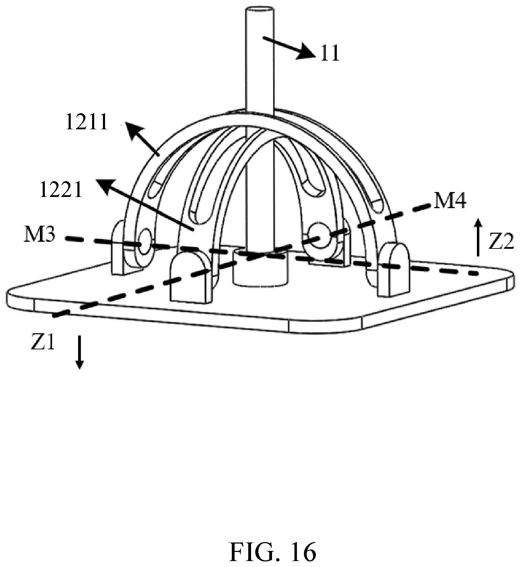

It should be noted that the two ends of the first connecting rod 1211 are rotatably connected to the first rotating ring 1212 and the base 123, respectively, and the two ends of the second connecting rod 1221 are rotatably connected to the second rotating ring 1222 and the base 123, respectively. That is, the first connecting rod 1211 and the second connecting rod 1221 are simultaneously rotatably connected to the base 123, which means the first connecting rod 1211 and the second connecting rod 1221 form a parallel structure. When the first rotating ring 1212 rotates about the first rotation axis O1, it will drive the first connecting rod 1211 to rotate about the second rotation axis O2, which in turn will drive the parallel second connecting rod 1221 to rotate about the fourth rotation axis O4, and thereby the rotation angle of the second connecting rod 1221 is detected by the third angle sensor 1223. When the second rotating ring 1222 rotates about the third rotation axis O3, it will drive the second connecting rod 1221 to rotate about the fourth rotation axis O4, which in turn will drive the parallel first connecting rod 1211 to rotate about the second rotation axis O2, and thereby the rotation angle of the first connecting rod 1211 is detected by the second angle sensor 1213. Therefore, the movement process of the rod structure 111 relative to the attitude adjustment execution component 120 can be simplified to the form shown in FIG. 16. FIG. 16 is merely a schematic diagram of a working principle of the attitude adjustment execution component 120, and the above embodiments may be implemented in various feasible manners and are not limited thereto. In FIG. 16, the first rotation axis O1, the second rotation axis O2, the third rotation axis O3, and the fourth rotation axis O4 are located in the same plane, at this time, an angle between the first rotation axis O1 and the fourth rotation axis O4 is 180°, and an angle between the second rotation axis O2 and the third rotation axis O3 is 180°. The rod structure 111 can rotate relative to the attitude adjustment execution component 120 in a plane formed by the first rotation axis O1 and the fourth rotation axis O4 (such as a vertical plane), that is, it has a third degree of freedom (as shown by arrow M3). The rod structure 111 can rotate relative to the attitude adjustment execution component 120 in a plane formed by the second rotation axis O2 and the third rotation axis O3 (such as a vertical plane), that is, it has a fourth degree of freedom (as shown by arrow M4).

It should be noted that since the first rotation axis O1 and the third rotation axis O3 may change with the movement of the first rotating ring 1212 and the second rotating ring 1222, respectively, therefore, the angle between the first rotation axis O1 and the fourth rotation axis O4 or the angle between the second rotation axis O2 and the third rotation axis O3 is not always 180°, and may also be 150°, 175°, or the like. When the angle between the first rotation axis O1 and the fourth rotation axis O4 and the angle between the second rotation axis O2 and the third rotation axis O3 are both 180°, that is, the first rotation axis O1 and the fourth rotation axis O4 are collinear and thus form a first rotation axis line Z1, and the second rotation axis O2 and the third rotation axis O3 are collinear and thus form a second rotation axis line Z2, at this time, a plane formed by the first rotation axis O1 and the fourth rotation axis O4 (i.e., a plane where the first rotation axis line Z1 is located) may be parallel to a horizontal plane, or may not be parallel to the horizontal plane. The plane formed by the second rotation axis O2 and the third rotation axis O3 (i.e., the plane where the second rotation axis line Z2 is located) may also be parallel to the horizontal plane or not parallel to the horizontal plane. When the angle between the first rotation axis O1 and the fourth rotation axis O4 and the angle between the second rotation axis O2 and the third rotation axis O3 are not 180°, the fourth rotation axis O4 forms the first rotation axis line Z1, and the second rotation axis O2 forms the second rotation axis line Z2. In some embodiments, the first rotation axis line Z1 and the second rotation axis line Z2 always intersect and are perpendicular to each other. Based on the above, it can be seen that when the rod structure 111 swings, it will cause the parallel first connecting rod 1211 and/or the second connecting rod 1221 to rotate, and further the rotation angles of the first connecting rod 1211 and the second connecting rod 1221 are detected by the second angle sensor 1213 and the third angle sensor 1223, thereby finally determining the attitude of the rod structure 111.

In some embodiments, the angle between the first rotation axis O1 and the third rotation axis O3 is greater than 10 degrees. In some embodiments, the angle between the first rotation axis O1 and the third rotation axis O3 is greater than 45 degrees. In some embodiments, the angle between the first rotation axis O1 and the third rotation axis O3 is greater than 60 degrees. In some embodiments, as shown in FIG. 15, the angle between the first rotation axis O1 and the third rotation axis O3 is 90 degrees, so as to enable the attitude adjustment execution component 120 to obtain a larger operation space. Similarly, in some embodiments, an angle between the second rotation axis O2 and the fourth rotation axis O4 is greater than 10 degrees. In some embodiments, as shown in FIG. 15, the angle between the second rotation axis O2 and the fourth rotation axis O4 is 90 degrees.

In some embodiments, the first connecting rod 1211 is shaped to match the first rotating ring 1212, and the second connecting rod 1221 is shaped to match the second rotating ring 1222. Merely by way of example, as shown in FIG. 15, the first rotating ring 1212 and the second rotating ring 1222 are circular rings, the first connecting rod 1211 and the second connecting rod 1221 are arc-shaped connecting rods, and the curvature of the arc-shaped connecting rods is the same as the curvature of the circular rings, so that the first connecting rod 1211 will not collide with the first rotating ring 1212 during rotation relative to the first rotating ring 1212, and the second connecting rod 1221 will not collide with the second rotating ring 1222 during rotation relative to the second rotating ring 1222, and at the same time, the structure may be more compact. It should be noted that the first connecting rod 1211 and the second connecting rod 1221 may also be designed into other arbitrary feasible shapes (such as right-angle shapes, or the like), as long as the corresponding connection function can be achieved.

In some embodiments, the attitude adjustment execution component 120 further includes a second connecting shaft 1224, a second support seat 1225, and a second shaft end cover 1226. The second support seat 1225 is disposed on the base 123, one end of the second connecting shaft 1224 is connected to the another end of the second connecting rod 1221, another end of the second connecting shaft 1224 is connected to the second support seat 1225 through a second bearing (not shown in the figures), the second shaft end cover 1226 fixes the second bearing on the second support seat 1225, and the second connecting rod 1221 can rotate relative to the second bearing and the second support seat 1225, thereby achieving rotation relative to the base 123. In some embodiments, the attitude adjustment execution component 120 further includes a first connecting shaft, a first support seat, and a first shaft end cover, and the specific arrangement manner of the first connecting shaft, the first support seat, and the first shaft end cover is the same as or similar to that of the second connecting shaft 1224, the second support seat 1225, and the second shaft end cover 1226.

In some embodiments, the attitude adjustment execution component 120 includes a passive degree-of-freedom connection component 126, the passive degree-of-freedom connection component 126 is rotatably connected to the first rotating ring 1212 and the second rotating ring 1222, and the passive degree-of-freedom connection component 126 is slidably connected to the rod structure 111. Merely by way of example, as shown in FIG. 17, the passive degree-of-freedom connection component 126 includes a passive degree-of-freedom mounting seat 1261, a first passive degree-of-freedom bearing 1262, and a second passive degree-of-freedom bearing 1263, and the first rotating ring 1212 is sleeved on an outer race of the first passive degree-of-freedom bearing 1262 and is relatively fixed to the outer race of the first passive degree-of-freedom bearing 1262. The second rotating ring 1222 is sleeved on an outer race of the second passive degree-of-freedom bearing 1263 and is relatively fixed to the outer race of the second passive degree-of-freedom bearing 1263. Inner races of the first passive degree-of-freedom bearing 1262 and the second passive degree-of-freedom bearing 1263 are both sleeved on the passive degree-of-freedom mounting seat 1261, and the inner races of the first passive degree-of-freedom bearing 1262 and the second passive degree-of-freedom bearing 1263 are relatively fixed to the passive degree-of-freedom mounting seat 1261. The rod structure 111 passes through the passive degree-of-freedom mounting seat 1261, and the passive degree-of-freedom mounting seat 1261 is slidably connected to the rod structure 111, so that the rod structure 111 can rotate relative to the first rotating ring 1212 and the second rotating ring 1222 along its own axial direction (as shown by arrow M2 in FIG. 17). In some embodiments, the rod structure 111 and the passive degree-of-freedom mounting seat 1261 cannot rotate relative to each other, while the rod structure 111 can realize rotation about the axial direction of the rod structure 111 relative to the first rotating ring 1212 and the second rotating ring 1222 through the first passive degree-of-freedom bearing 1262 and the second passive degree-of-freedom bearing 1263, so that the rod structure 111 can rotate about its own axial direction relative to the attitude adjustment execution component 120. In some embodiments, the first passive degree-of-freedom bearing 1262 and the second passive degree-of-freedom bearing 1263 are rolling bearings.

In other embodiments, the rod structure 111 may rotate relative to the passive degree-of-freedom mounting seat 1261, and the rod structure 111 can realize rotation about the axial direction of the rod structure 111 relative to the first rotating ring 1212 and the second rotating ring 1222 through the first passive degree-of-freedom bearing 1262 and the second passive degree-of-freedom bearing 1263, so that the rod structure 111 can also rotate about its own axial direction relative to the attitude adjustment execution component 120.

It should be pointed out that the passive degree-of-freedom mounting seat 1261 forms a rotating pair with the first rotating ring 1212 through the first passive degree-of-freedom bearing 1262, and also forms a rotating pair with the second rotating ring 1222 through the second passive degree-of-freedom bearing 1263. Since both the first connecting rod 1211 and the second connecting rod 1221 are connected to the base 123, when the first rotating ring 1212 rotates relative to the passive degree-of-freedom mounting seat 1261, it will drive the second rotating ring 1222 to rotate relative to the passive degree-of-freedom mounting seat 1261. Similarly, when the second rotating ring 1222 rotates relative to the passive degree-of-freedom mounting seat 1261, it will also drive the first rotating ring 1212 to rotate relative to the passive degree-of-freedom mounting seat 1261, so that the angle between the first rotation axis O1 and the second rotation axis O2 remains unchanged.

In some embodiments, as shown in FIG. 17, the attitude adjustment execution component 120 further includes a bearing locking seat 1264, the bearing locking seat 1264 is disposed on a top of the passive degree-of-freedom mounting seat 1261, and fixedly connects the inner races of the first passive degree-of-freedom bearing 1262 and the second passive degree-of-freedom bearing 1263 to the passive degree-of-freedom mounting seat 1261 (for example, through screw connection), to prevent the first passive degree-of-freedom bearing 1262 and the second passive degree-of-freedom bearing 1263 from detaching from the passive degree-of-freedom mounting seat 1261.

In some embodiments, the passive degree-of-freedom connection component 126 may not be essential, and rotation of the rod structure 111 about its own axial direction relative to the attitude adjustment execution component 120 may be achieved in other manners. Merely by way of example, the sliding portion 1112 and the operation portion 1111 of the rod structure 111 are rotatably connected (for example, via a bearing), wherein the sliding portion 1112 is slidably connected to the first rotating ring 1212 and the second rotating ring 1222, the first rotating ring 1212 and the second rotating ring 1222 may move relative to the sliding portion 1112 along an axial direction of the sliding portion 1112, and the operation portion 1111 may rotate relative to the sliding portion 1112 along the axial direction of the sliding portion 1112. Furthermore, the first rotating ring 1212 and the second rotating ring 1222 are also rotatably connected to each other, so that the first rotating ring 1212 and the second rotating ring 1222 can perform relative rotation along the axial direction of the rod structure 111. When an operator holds the operation portion 1111, the operator may also hold it in a more comfortable posture to satisfy a rotation requirement of the rod structure 111 at a certain angle relative to the attitude adjustment execution component 120, so that the rod structure 111 can adapt to the grip posture of the operator during the attitude adjustment of the operator. For a detailed description of the sliding portion 1112 and the operation portion 1111, please refer to the following text.

In some embodiments, the attitude adjustment execution component 120 may provide an attitude adjustment resistance that hinders deflection of the rod structure 111 (i.e., deflection relative to the base 123) to simulate the attitude adjustment resistance experienced during actual operation of the puncture needle. In some embodiments, the magnitude of the attitude adjustment resistance provided by the attitude adjustment execution component 120 is fixed. In some actual application scenarios, when performing attitude adjustment on the rod structure 111 (and the puncture needle at the distal end) during a needle insertion operation, when an operator applies a deflection force to the rod structure 111 for adjusting an attitude of the rod structure 111, as the rod structure 111 continuously moves along its own axial direction along a first direction (i.e., moving downwards along arrow X in FIG. 1B), the force arm of the deflection force applied by the operator on the rod structure 111 (the length of the force arm may be equal to a distance along the axial direction of the rod structure 111 between a force application point of the operator on the rod structure 111 and a contact point between the rod structure 111 and the attitude adjustment execution component 120) gradually decreases. Since the torque (i.e., the attitude adjustment resistance applied by the attitude adjustment execution component 120 to the rod structure 111 that hinders deflection of the rod structure 111) remains unchanged, the motion resistance perceived by the operator will gradually increase. When the rod structure 111 moves a certain distance along the first direction, the motion resistance perceived by the operator is too large, at this time, a large force is required to enable the rod structure 111 to continue moving along the first direction, which results in the axial movement of the rod structure 111 being too difficult, and is not conducive to precise adjustment of the puncture depth of the puncture needle. Similarly, during a needle retraction operation, as the rod structure 111 continuously moves along its own axial direction along the second direction (i.e., moving upwards along arrow X in FIG. 1B), the force arm of an acting force applied by the operator on the rod structure 111 gradually increases, and the motion resistance perceived by the operator will gradually decrease. When the rod structure 111 moves a certain distance along the second direction, the motion resistance perceived by the operator is too small, at this time, only a small force is required to enable the rod structure 111 to continue moving along the second direction, which results in the axial movement of the rod structure 111 being too flexible. Based on the above content, it can be known that although the attitude adjustment execution component 120 provides a fixed magnitude of attitude adjustment resistance, it may also simulate the attitude adjustment resistance of the puncture needle to a certain extent, however, when the rod structure 111 moves a certain distance along the first direction, it may lead to the axial movement of the rod structure 111 being too difficult, which is not conducive to precise adjustment of the puncture depth of the puncture needle. When the rod structure 111 moves a certain distance along the second direction, it may lead to the axial movement of the rod structure 111 being too flexible, which is also not conducive to precise adjustment of the puncture depth of the puncture needle.

In some embodiments, the attitude adjustment execution component 120 includes at least one motor. The motor can implement homing (for example, enabling the first connecting rod 1211 to return to a zero position), force feedback (for example, outputting attitude adjustment resistance), and outputting torque to overcome damping, or the like, as one or a plurality of functions.

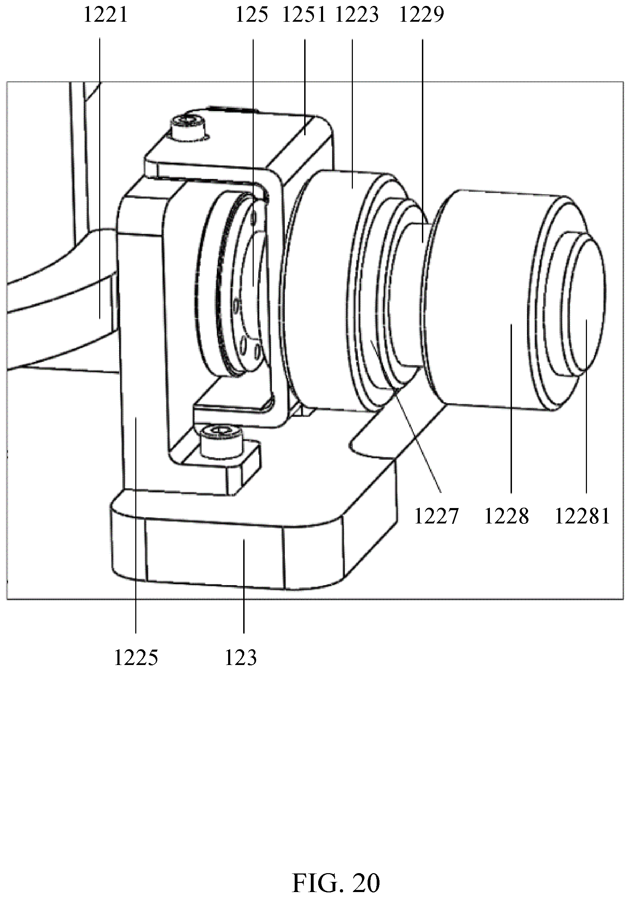

In some embodiments, as shown in FIG. 20, the attitude adjustment execution component 120 includes a first motor (not shown in the figure) and a second motor 1228. The first motor is configured to apply a torque to the first connecting rod 1211. By applying torques of different magnitudes and/or different directions to the first connecting rod 1211 at different times, the first motor can implement functions such as enabling the first connecting rod 1211 to return to the zero position and applying attitude adjustment resistance to the first connecting rod 1211, or the like. The second motor 1228 is configured to apply a torque to the second connecting rod 1221. By applying torques of different magnitudes and/or different directions to the second connecting rod 1221 at different times, the second motor 1228 can implement functions such as enabling the second connecting rod 1221 to return to the zero position and applying attitude adjustment resistance to the second connecting rod 1221, or the like. In other embodiments, only one motor may be provided, the motor applies torque to the first connecting rod 1211 and the second connecting rod 1221 respectively through a transmission structure (for example, a clutch).

In some embodiments, the first motor is configured to apply an attitude adjustment resistance torque to the first connecting rod 1211 based on attitude adjustment force feedback information during attitude adjustment of the puncture needle. The second motor 1228 is configured to apply an attitude adjustment resistance torque to the second connecting rod 1221 based on attitude adjustment force feedback information during attitude adjustment of the puncture needle.

Through the collaborative work of the first motor applying attitude adjustment resistance to the first connecting rod 1211 and the second motor 1228 applying attitude adjustment resistance to the second connecting rod 1221, the attitude adjustment execution component 120 can accurately output attitude adjustment resistance based on attitude adjustment force feedback information during attitude adjustment, to ensure that an operator can accurately perceive the resistance experienced during attitude adjustment of the puncture needle when performing an attitude adjustment operation.

In some embodiments, the attitude adjustment execution component 120 further includes a first homing component and a second homing component, the first homing component is configured to enable the first connecting rod 1211 to return to the zero position, and the second homing component is configured to enable the second connecting rod 1221 to return to the zero position. The first homing component includes the above-mentioned first motor, and the second homing component includes the above-mentioned second motor. The zero position refers to a standard position of the first connecting rod 1211 and the second connecting rod 1221. In some embodiments, the zero position may be set according to usage requirements. For example, when an angle between a second rotation axis (for example, shown as O2 in FIG. 15) and the plane of the first rotating ring 1212 is a first preset angle, and an angle between a fourth rotation axis and the plane of the second rotating ring 1222 is a second preset angle, the position of the first connecting rod 1211 and the second connecting rod 1221 may be defined as the zero position. For convenience of description, in embodiments of the present disclosure, the position of the first connecting rod 1211 and the second connecting rod 1221 may be defined as the zero position when a first rotation axis (for example, shown as O1 in FIG. 15) is coaxial with the fourth rotation axis (meaning the angle between the fourth rotation axis and the plane of the second rotating ring 1222 is 0), and the second rotation axis is coaxial with a third rotation axis (for example, shown as O3 in FIG. 15) (meaning the angle between the second rotation axis and the plane of the first rotating ring 1212 is 0), as shown in FIG. 15.

In some actual application scenarios, an operator may perform a homing operation before a puncture surgery, to ensure that the attitude adjustment execution component 120 is in the zero position. The operator may also perform a homing operation after the puncture surgery, to ensure that the attitude adjustment execution component 120 returns to the zero position, for use in a next puncture surgery.

In some embodiments, the attitude adjustment execution component 120 includes a first reducer (not shown in the figure) and a second reducer 1229. The first motor is connected to the first damper through the first reducer, and the second motor 1228 is connected to the second damper 125 through the second reducer 1229. The first motor is configured to output a torque to overcome an output damping of the first damper, the second motor 1228 is configured to output a torque to overcome an output damping of the second damper 125, the first reducer is configured to increase an output torque of the first damper, and the second reducer 1229 is configured to increase an output torque of the second damper 125. In some embodiments, the output shaft of the first motor is connected to the input shaft of the first reducer, and the output shaft of the first reducer is connected to the first damper. The output shaft of the second motor 1228 is connected to the input shaft of the second reducer 1229, and the output shaft of the second reducer 1229 is connected to the second damper 125. The first reducer increases the torque of the output shaft of the first motor by reducing the rotation speed of the output shaft of the first motor. The second reducer 1229 increases the torque of the output shaft of the second motor 1228 by reducing the rotation speed of the output shaft of the second motor 1228.

In some embodiments, the first homing component includes the first motor (not shown in the figure) and the first reducer (not shown in the figure), and the second homing component includes the second motor 1228 and the second reducer 1229. In the present embodiment, when homing of the first connecting rod 1211 and the second connecting rod 1221 is required, the first motor and the second motor 1228 may be driven to work. The output torque of the first motor is amplified by the first reducer and then transmitted to the first damper, completely overcoming the output damping of the first damper, thereby enabling the first connecting rod 1211 to rotate (i.e., rotate about the second rotation axis) relative to the base 123 and return to the zero position. At the same time, the rotation of the first connecting rod 1211 relative to the base 123 will also drive the second connecting rod 1221 to rotate (i.e., rotate about the third rotation axis) relative to the second rotating ring 1222 and return to the zero position. Similarly, the output torque of the second motor 1228 is amplified by the second reducer 1229 and then transmitted to the second damper 125, completely overcoming the output damping of the second damper 125, thereby enabling the second connecting rod 1221 to rotate (i.e., rotate about the fourth rotation axis) relative to the base 123 and return to the zero position, which further drives the first connecting rod 1211 to rotate (i.e., rotate about the first rotation axis) relative to the first rotating ring 1212 and return to the zero position.

In some embodiments, the first homing component further includes a first homing angle sensor (not shown in the figure), and the second homing component further includes a second homing angle sensor 12281. The first homing angle sensor is configured to detect the rotation speed of the output shaft of the first motor, so as to implement control of the output torque of the first motor during the homing process. The second homing angle sensor 12281 is configured to detect the rotation speed of the output shaft of the second motor 1228, so as to implement control of the output torque of the output shaft of the second motor 1228 during the homing process.

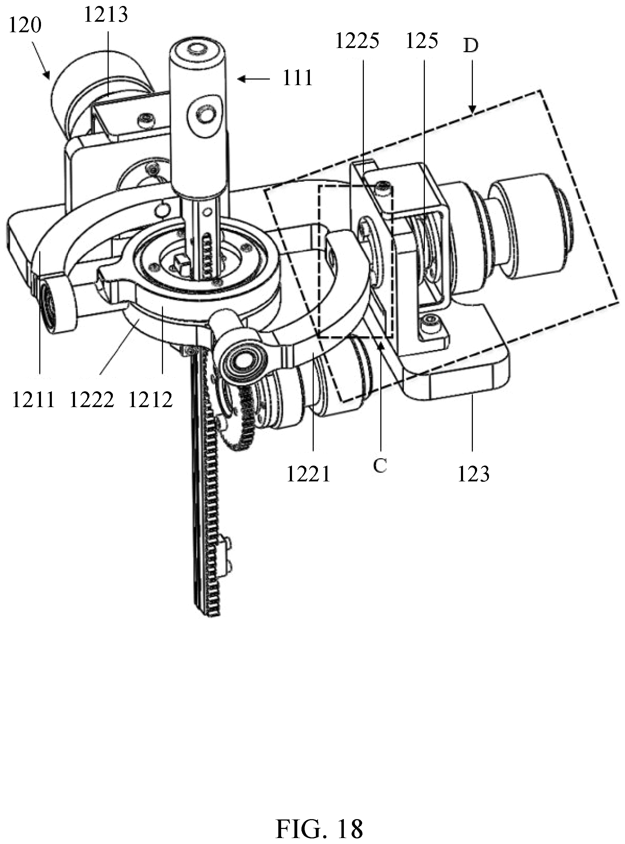

In some embodiments, in conjunction with FIGs. 18-20, the attitude adjustment execution component 120 further includes a first damper (not shown in the figure) and a second damper 125. The first damper is configured to provide a motion resistance hindering rotation of the first connecting rod (1211) relative to the base (123) based on a position of the rod structure (111) along its own axial direction. The second damper (125) is configured to provide a motion resistance hindering rotation of the second connecting rod (1221) relative to the base (123) based on the position of the rod structure (111) along its own axial direction. Merely by way of example, as shown in FIG. 18, taking the second damper (125) as an example, the second damper (125) may include a rotating end and a fixed end. The rotating end is fixedly connected to a second connecting shaft 1224. The fixed end is fixed on a second support seat 1225 via a second damper mounting seat 1251. A pressure exists between the rotating end and the fixed end, which generates a friction force hindering rotation of the rotating end relative to the fixed end, thereby generating a damping hindering rotation of the second connecting rod (1221) relative to the base (123). In some embodiments, the friction force between the rotating end and the fixed end is positively correlated with a current of the second damper (125). The second damper (125) may obtain the position of the rod structure (111) along its own axial direction from the first position detection module (112) or the first angle sensor (1323), and then adjust the current based on the position of the rod structure (111), and finally change the output damping. For example, the greater a stroke along a first direction, which indicates the deeper the puncture depth of the puncture needle, then the current of the second damper (125) may be reduced to reduce the output damping, so as to ensure that the motion resistance perceived by an operator at the rod structure (111) during the attitude adjustment process basically remains constant. For example, the greater the stroke along a second direction, which indicates the shallower the puncture depth of the puncture needle, then the current of the second damper (125) may be increased to increase the output damping, so as to ensure that the motion resistance perceived by the operator at the rod structure (111) during the attitude adjustment process basically remains constant. In some embodiments, the structure and working principle of the first damper may be the same as or similar to those of the second damper (125), details of which will not be described again herein.

It should be noted that the structure of a first homing component and a second homing component shown in FIG. 20 is merely for illustrative purposes, and is not intended to limit the specific structure of the first homing component and the second homing component. In some embodiments, the specific structures of the first homing component and the second homing component may be adjusted according to actual situations. Taking the second homing component as an example, to enable the second connecting rod (1221) to return to a zero position, it is necessary to overcome the output damping of the second damper (125). If the second damper (125) is an adaptively adjustable damper, when the puncturing of the puncture needle is completed, the current of the second damper (125) may be adjusted to a minimum value (for example, 0). At this time, the output damping of the second damper (125) is relatively small or may be negligible. At this time, the torque output by the second motor (1228) can fully overcome the output damping of the second damper (125), so as to enable the second connecting rod (1221) to return to the zero position. If the second damper (125) is a fixed output damper, since the output damping of the second damper (125) is constant (not adjustable), the output damping of the second damper (125) remains unchanged at all times. The torque output by the second motor (1228) may not be able to fully overcome the output damping of the second damper (125), and thus may not be able to enable the second connecting rod (1221) to completely return to the zero position. At this time, it is necessary to increase the torque output by the second motor (1228) through the second reducer (1229).

As described above, in some actual application scenarios, the attitude adjustment of the puncture needle and the advance and retreat operations of the puncture needle cannot be performed simultaneously, so as to avoid causing damage to human tissue. Therefore, in some embodiments, in conjunction with FIGs. 18-20, the attitude adjustment execution component (120) further includes the second brake (not shown in the figure) and the third brake (1227). An output shaft of the second brake is drivingly connected to the first connecting rod (1211) through the first damper, the second brake is configured to restrict rotation of the first connecting rod (1211) relative to the base (123), an output shaft of the third brake (1227) is drivingly connected to the second connecting rod (1221) through the second damper (125), and the third brake (1227) is configured to restrict rotation of the second connecting rod (1221) relative to the base (123). In some embodiments, the second brake is configured to restrict the first connecting rod (1211) from rotating about a third rotation axis (for example, as indicated by O3 in FIG. 15), thereby restricting rotation of the first connecting rod (1211) relative to the base (123). The third brake (1227) is configured to restrict the second connecting rod (1221) from rotating about a fourth rotation axis (for example, as indicated by O4 in FIG. 15), thereby restricting rotation of the second connecting rod (1221) relative to the base (123). Taking the third brake (1227) as an example, the third brake (1227) may be connected to the second damper (125) to restrict the second connecting rod (1221) from rotating about the fourth rotation axis by restricting relative rotation between the fixed end and the rotating end of the second damper (125), thereby restricting rotation of the second connecting rod (1221) relative to the base (123). In some embodiments, the structure and working principle of the second brake and the third brake (1227) may be the same as or similar to the structure and working principle of the first brake (1324) in the foregoing embodiments, details of which will not be described again herein.

It can be understood that by providing the second brake and the third brake (1227) to respectively restrict the rotation of the first connecting rod (1211) and the second connecting rod (1221), it can be effectively ensured that the attitude of the puncture needle will not swing when the puncture needle performs a puncture operation.

In some embodiments, when it is necessary to control the rod structure (111) to perform an advance or retreat operation, it is necessary to restrict attitude adjustment of the rod structure (111) by the attitude adjustment execution component (120). At this time, the current of the second brake and the third brake (1227) may be adjusted to a maximum value, thereby respectively preventing the first connecting rod (1211) and the second connecting rod (1221) from rotating relative to the base (123) by the generated braking torque, and further preventing attitude adjustment of the rod structure (111) by the attitude adjustment execution component (120). When it is necessary to adjust the attitude of the rod structure (111) through the attitude adjustment execution component (120), the current of the second brake and the third brake (1227) may be adjusted to a minimum value. At this time, the generated braking torque may have a small or negligible effect on the rotation of the first connecting rod (1211) and the second connecting rod (1221) relative to the base (123). Therefore, the rack (1131) and the rod structure (111) can move relative to the gear (1322).

In some embodiments, the second brake and the third brake (1227) are communicatively connected to the first brake (1324). When the braking torque output by the second brake and the third brake (1227) is a maximum value (that is, restricting attitude adjustment of the rod structure (111) by the attitude adjustment execution component (120)), the braking torque output by the first brake (1324) is a minimum value (that is, allowing advance or retreat operations on the rod structure (111)). When the braking torque output by the second brake and the third brake (1227) is a minimum value (that is, allowing attitude adjustment of the rod structure (111) by the attitude adjustment execution component (120)), the braking torque output by the first brake (1324) is a maximum value (that is, restricting advance or retreat operations on the rod structure (111)).

In some embodiments, the main hand control device (100) includes at least one of a mode selection member, a degree-of-freedom selection member, and a quick switching member.

The mode selection member is a control member for selecting a working mode of the main hand control device (100). In some embodiments, the mode selection member is used to select the main hand control device (100) to be in a puncture mode or an attitude adjustment mode. In the puncture mode, the rod structure (111) has a puncture degree of freedom relative to the attitude adjustment execution component (120), and in the attitude adjustment mode, the rod structure (111) has an attitude adjustment degree of freedom relative to the attitude adjustment execution component (120). Merely by way of example, the mode selection member is a mode switching button. The mode switching button is used to switch between different operation modes. When the mode switching button is pressed, the main hand control device (100) is in the puncture mode. When the mode switching button is not pressed, the main hand control device (100) is in the attitude adjustment mode. When the puncture mode is selected by the mode selection member, the drive structure (such as the advance-retreat drive motor described below) on the puncture device that controls the puncture degree of freedom is started. The advance-retreat drive motor is powered on and supplied with a certain control current. The control current of the advance-retreat drive motor is positively proportional to an acting force between the puncture needle and the human body. As a result, an operator can perceive a feedback force during an actual puncture process on the main hand control device (100), thereby realizing a force feedback process. The drive structure (such as the sixth brake and the seventh brake described below) on the puncture device that controls the attitude adjustment degree of freedom is locked. For example, the sixth brake and the seventh brake maintain a low level, so that an attitude adjustment action cannot be performed. When the attitude adjustment mode is selected by the mode selection member, contrary to the control of the puncture mode described above, the drive structure controlling the attitude adjustment degree of freedom is started, and the drive structure controlling the puncture degree of freedom is locked, so that a puncture action cannot be performed.

The degree-of-freedom selection member is a control member for selecting the attitude adjustment degree of freedom. The attitude adjustment degree of freedom includes at least one of a first degree of freedom and a second degree of freedom described above. The degree-of-freedom selection member controls an attitude adjustment degree of freedom of the puncture needle (211) relative to the puncture device (200) to be at least one of the first degree of freedom and the second degree of freedom. Merely by way of example, the degree-of-freedom selection member may be a degree-of-freedom selection knob. When the degree-of-freedom selection knob rotates to a first position, the rod structure (111) is only allowed to perform attitude adjustment under the first degree of freedom. When the degree-of-freedom selection knob rotates to a second position, the rod structure (111) is only allowed to perform attitude adjustment under the second degree of freedom. When the degree-of-freedom selection knob rotates to a third position, the rod structure (111) is allowed to perform attitude adjustment under the first degree of freedom and the second degree of freedom. For details about the first degree of freedom and the second degree of freedom, refer to relevant descriptions in FIGs. 12-15.

In some embodiments, the setting of the first degree of freedom and the second degree of freedom (such as the setting of a specific direction allowed by the degrees of freedom) may be determined based on medical images of a patient. Merely by way of example, for a Computed Tomography (CT) image, it includes a plurality of tomographic images. At this time, the first degree of freedom may be a degree of freedom that only allows the rod structure (111) to control the puncture needle to move in an intra-layer region of any tomographic image for attitude adjustment, and the second degree of freedom may be a degree of freedom that only allows the rod structure (111) to control the puncture needle to move between different layers corresponding to the respective tomographic images for attitude adjustment. When the degree-of-freedom selection member only allows the first degree of freedom, it may be in an intra-layer attitude adjustment mode. An intra-layer degree-of-freedom brake (such as the sixth brake described below) is supplied with a high level, an inter-layer degree-of-freedom brake (such as the seventh brake described above) maintains a low level, and a puncture degree-of-freedom brake (such as the fifth brake described below) is at a low level, so that advance and puncture cannot be performed. When the degree-of-freedom selection member only allows the second degree of freedom, it may be in an inter-layer attitude adjustment mode. The inter-layer degree-of-freedom brake (such as the seventh brake described below) is supplied with a high level, the intra-layer degree-of-freedom brake (such as the sixth brake described below) maintains a low level, and the puncture degree-of-freedom brake (such as the fifth brake described below) is at a low level, so that advance and puncture cannot be performed. When the degree-of-freedom selection member allows the first degree of freedom and the second degree of freedom, it may be in a free mode. The intra-layer and inter-layer degree-of-freedom brakes (such as the sixth brake and the seventh brake described below) are both supplied with a high level, and the puncture degree-of-freedom brake (such as the fifth brake described below) is at a low level, so that advance and puncture cannot be performed.

The quick switching member is a control member for performing quick switching of a mode of the main hand control device. The quick switching member is used to control the main hand control device 100 to switch from the attitude adjustment mode to the puncture mode, and/or to control the main hand control device 100 to switch back from the puncture mode to the attitude adjustment mode. For example, the quick switching member may be a quick switching button.

In some embodiments, the main hand control device 100 comprises a release control member. The release control member is used to control a force feedback component 130 and the attitude adjustment execution component 120 to release a force applied to the rod structure 111. In some embodiments, when a puncture operation is completed, the release control member is triggered, and at this time, the brakes of the three degrees of freedom of the main hand control device 100 (including the puncture degree of freedom, the first degree of freedom, and the second degree of freedom of the attitude adjustment degree of freedom), such as the first brake, the second brake, and the third brake described above, are released. In some embodiments, When the release control member is triggered, the motors of the three degrees of freedom (including the puncture degree of freedom, the first degree of freedom, and the second degree of freedom of the attitude adjustment degree of freedom), such as the force feedback motor, the first motor, and the second motor, may also be controlled to drive the rod structure to return to a zero position (e.g., the rod structure 111 is perpendicular to a horizontal plane and a bottom end of the rod structure 111 is connected to the attitude adjustment execution component 120). After returning to the zero position, the brakes of the three degrees of freedom (such as the first brake, the second brake, and the third brake described above) are locked, and the rod structure 111 cannot move.

In some embodiments, in conjunction with FIGs. 2-4, the rod structure 111 comprises an operation portion 1111 and a sliding portion 1112. The operation portion 1111 is configured to receive a user operation. The sliding portion 1112 is configured to move relative to the attitude adjustment execution component 120 along its axial direction. The operation portion 1111 is located at an upper end of the rod structure 111. A user may be an operator. The operator may control the operation portion 1111 to perform corresponding operations, so as to control the rod structure 111 to perform corresponding actions, and ultimately achieve the purpose of controlling the puncture needle. For example, the operation portion 1111 is connected to the sliding portion 1112, and the sliding portion 1112 is slidably connected to the attitude adjustment execution component 120 or slidably connected thereto through a connecting member (such as a passive degree-of-freedom connection component 126 or the like described below). The operator may control the sliding portion 1112 to move along the axial direction of the rod structure 111 relative to the attitude adjustment execution component 120 through the operation portion 1111, so as to control the puncture needle to advance or retreat. For example, the operator may control the sliding portion 1112 to swing through the operation portion 1111, thereby adjusting the attitude of the puncture needle.

In some embodiments, the operation portion 1111 may comprise an interactive handle 1110, and the operator may control the rod structure 111 by holding the interactive handle 1110. In some embodiments, the interactive handle 1110 may be a structure such as a prism, a cylinder, or the like. In some embodiments, the interactive handle 1110 is a cylindrical structure. Since the attitude when holding the interactive handle 1110 is relatively similar to the attitude when holding the puncture needle, the operator can therefore feel clinical puncture sensation as much as possible, thereby improving a success rate of a puncture operation.

In some embodiments, the operation portion 1111 comprises a plurality of operation regions 11111 and at least one enable button. The enable button refers to a button that controls activation or deactivation of master-slave end movement enablement of a control system. For example, when one or a plurality of enable buttons are triggered, the master-slave end movement enablement is activated, and an action applied to the main hand control device 100 may be mapped to the robot, so as to control the puncture needle to perform the same action as the rod structure 111 through the robot.

In some embodiments, the rod structure 111 further comprises a circuit board (not shown) and a signal transmission mechanism (not shown). The circuit board is connected to the signal transmission mechanism, and a lower end of the enable button is electrically connected to the circuit board. When the enable button is pressed, the circuit board processes a trigger signal of the enable button, and transmits the trigger signal to a signal transmission component through the signal transmission mechanism (for example, an antenna), so as to realize the master-slave movement enablement control. In some embodiments, the enable button is a silicone button.

In some embodiments, a plurality of control regions 11111 comprise a first control region 11112 and at least one second control region 11114, and the first control region 11112 and the second control region 11114 are arranged opposite to each other. The arrangement opposite to each other refers to the first control region 11112 and the second control region 11114 being symmetrically arranged at two distant positions on the operation portion 1111. The control region refers to a region of the rod structure 111 that is controlled by fingers of the operator. Merely by way of example, in conjunction with FIGs. 2-3, the first control region 11112 and the second control region 11114 may be symmetrically arranged on two sides of a peripheral wall of the interactive handle 1110 along the axial direction of the rod structure 111, and the operator may place different fingers on the first control region 11112 and the second control region 11114 respectively to hold and control the rod structure 111.

In some cases, since the first control region 11112 and the second control region 11114 are arranged opposite to each other, the operator can therefore hold the operation portion 1111 more firmly, and it is convenient for the operator to apply the force to the rod structure 111.

In some embodiments, the at least one enable button comprises a first enable button 11113 arranged in the first control region 11112. In some embodiments, the number of the first enable buttons 11113 is one. Merely by way of example, in conjunction with FIGs. 2 and 3, the first control region 11112 may include one first enable button 11113, a thumb of the operator may be placed at the first enable button 11113, and at least one of an index finger, a middle finger, and a ring finger of the operator may be placed in the second control region 11114. When the operator presses the first enable button 11113, the master-slave end movement enablement may be activated. In some embodiments, there may be a plurality of the first enable buttons 11113. Merely by way of example, the first control region 11112 may include two first enable buttons 11113, wherein the two first enable buttons 11113 are distributed along the axial direction of the rod structure 111, and a thumb of the operator may be placed on any one of the two first enable buttons 11113. When the operator presses any one of the first enable buttons 11113, the master-slave end movement enablement may be activated.

In some cases, since there are a plurality of the first enable buttons 11113, the operator can therefore select a more suitable holding attitude for holding, which can effectively improve an operation experience of the operator.

In some embodiments, the number of the first control regions 11112 may be one, for example, in the embodiment shown in FIG. 3, the number of the first control regions 11112 is one, and one first enable button 11113 is arranged in the first control region 11112. In some embodiments, there may be a plurality of the first control regions 11112. For example, the number of the first control regions 11112 may be two, and specific positions of the two first control regions 11112 may be arranged according to actual needs.

In some embodiments, the at least one enable button comprises at least one second enable button 11115 arranged in the second control region 11114. Merely by way of example, referring to FIGs. 2-3, the first control region 11112 may include a first enable button 11113, and the second control region 11114 may include a second enable button 11115. The thumb of an operator may be placed on the first enable button 11113, and any one of the index finger, the middle finger, and the ring finger of the operator may be placed on the second enable button 11115. When the operator simultaneously presses the first enable button 11113 and the second enable button 11115, master-slave movement enablement may be enabled, thereby effectively preventing misoperations caused by accidental touches on the enable buttons. Conversely, when the operator does not simultaneously press the first enable button 11113 and the second enable button 11115, the master-slave movement enablement is disabled. In another example, the second control region 11114 may include two second enable buttons 11115. The thumb of the operator may be placed on the first enable button 11113, and any two of the index finger, the middle finger, and the ring finger may be respectively placed on the two second enable buttons 11115. When the operator simultaneously presses the first enable button 11113 and the two second enable buttons 11115, the master-slave movement enablement may be enabled, thereby effectively preventing misoperations caused by accidental touches on the enable buttons. Conversely, when the operator does not simultaneously press the first enable button 11113 and the two second enable buttons 11115, the master-slave movement enablement is disabled.

In some embodiments, the first control region 11112 and/or the second control region 11114 may include a finger placement region 11117. The finger placement region 11117 can help the operator better grip the rod structure 111, improve operation efficiency, and facilitate applying force to the rod structure 111. In some embodiments, the finger placement region 11117 may protrude from a surface of the rod structure 111. In some embodiments, the finger placement region 11117 may be recessed relative to a surface of the rod structure 111. In some embodiments, the finger placement region 11117 may be provided with an anti-slip material, For example, rubber, silicone, or the like, to facilitate the operator's grip. Merely by way of example, as shown in FIG. 2, the second control region 11114 may include a second enable button 11115 and two finger placement regions 11117. The index finger of the operator may be placed on the second enable button 11115, and any two of the middle finger, the ring finger, and the little finger may be placed on the finger placement regions 11117.

In some embodiments, the at least one enable button further includes a third enable button 11116 disposed on a top of the operation portion 1111. Merely by way of example, referring to FIGs. 2-3, the first control region 11112 may include a first enable button 11113, and the second control region 11114 may include a second enable button 11115 and two finger placement regions 11117. The thumb of the operator may be placed on the first enable button 11113, the index finger may be placed on the third enable button 11116, the middle finger may be placed on the second enable button 11115, and the ring finger and the little finger may be placed on the finger placement regions 11117. When the operator simultaneously presses any two of the first enable button 11113, the second enable button 11115, and the third enable button 11116, master-slave movement enablement may be controlled to be enabled.

In some cases, due to the first enable button 11113, the second enable button 11115, and the third enable button 11116 being simultaneously disposed on the rod structure 111, the operator only needs to press any two of them to enable the master-slave movement enablement. This may provide the operator with a plurality of selectable operation postures, so that the operator can perform a puncture operation in a more comfortable gripping posture, making the posture of the operator when gripping the rod structure 111 more flexible and applicable to a wider range of scenarios.

It should be noted that the arrangement forms of the operation regions 11111 and the enable buttons shown in FIGs. 2 and 3 are merely for illustrative purposes, and are not intended to limit the number and arrangement positions of the operation regions 11111 and the enable buttons. In practical application scenarios, the number and arrangement positions of the operation regions 11111 and the enable buttons may be adjusted according to the gripping habits and gripping postures of the operator.

In some embodiments, referring to FIGs. 3 and 6, the sliding portion 1112 is provided with a first limit structure 1113 and a second limit structure 1114 respectively at an end of the sliding portion 1112 close to the operation portion 1111 and an end of the sliding portion 1112 away from the operation portion 1111. The first limit structure 1113 and the second limit structure 1114 are configured to limit a stroke range of movement of the rod structure 111 along its own axial direction.

In some cases, the first limit structure 1113 and the second limit structure 1114 can define extreme positions of movement of the rod structure 111 along its own axial direction, preventing the rod structure 111 from over-stroke operation, ensuring accuracy of a motion trajectory of a puncture needle, and preventing over-stroke puncturing of the puncture needle, so as to avoid accidental occurrences. In some embodiments, a distance between the first limit structure 1113 and the second limit structure 1114 is a maximum stroke of movement of the rod structure 111 along its own axial direction. Merely by way of example, when assembly of the main hand control device 100 is completed, the first limit structure 1113 and the second limit structure 1114 are respectively located above and below the attitude adjustment execution component 120. During movement of the rod structure 111 along its own axial direction, the first limit structure 1113 and the second limit structure 1114 may respectively abut against the attitude adjustment execution component 120 to restrict the rod structure 111 from further movement.

In some embodiments, the first limit structure 1113 and the second limit structure 1114 may be limit blocks. For example, referring to FIGs. 1B and 3, the first limit structure 1113 (e.g., a first limit block) is located above the attitude adjustment execution component 120 (e.g., the passive degree-of-freedom connection component 126 of the attitude adjustment execution component 120), and the second limit structure 1114 (e.g., a second limit block) is located below the attitude adjustment execution component 120. During a needle advance operation of the rod structure 111 (i.e., when the first limit structure 1113 moves toward the passive degree-of-freedom connection component 126), when the rod structure 111 drives the first limit structure 1113 to abut against the passive degree-of-freedom connection component 126, it indicates that the rod structure 111 moves to an extreme needle advance position. At this point, the rod structure 111 cannot continue to advance the needle. During a needle retraction operation of the rod structure 111 (i.e., when the first limit structure 1113 moves away from the passive degree-of-freedom connection component 126), when the rod structure 111, by retraction, drives the second limit structure 1114 to abut against the passive degree-of-freedom connection component 126, it indicates that the rod structure 111 moves to an extreme needle retraction position. At this point, the rod structure 111 cannot continue to advance the needle.

In some embodiments, the first limit structure 1113 may include a first photoelectric sensor, and the second limit structure 1114 may include a second photoelectric sensor. An inner side wall of the first rotating ring 1212 is provided with a first photoelectric induction sheet, and an inner side wall of the second rotating ring 1222 is provided with a second photoelectric induction sheet. The photoelectric sensors can detect the first photoelectric induction sheet and generate a first sensing signal, and detect the second photoelectric induction sheet and generate a second sensing signal. The first sensing signal is different from the second sensing signal. During the movement of the rod structure 111 along its own axial direction, when the first photoelectric sensor generates the first sensing signal, it indicates that the first photoelectric induction sheet moves to the passive degree-of-freedom connection component 126, that is, the rod structure 111 reaches the extreme needle advance position. When the second photoelectric sensor generates the second sensing signal, it indicates that the second photoelectric induction sheet moves to the passive degree-of-freedom connection component 126, that is, the rod structure 111 reaches the extreme needle retraction position. In some embodiments, the first photoelectric sensor and the second photoelectric sensor are electrically connected to a signal transmission component. The sensing signals generated by the first photoelectric sensor and the second photoelectric sensor may be fed back to the signal transmission component. When the rod structure 111 moves to any extreme position, the signal transmission component can issue a warning to the operator. For example, the signal transmission component issues a voice warning via a microphone disposed on the main hand control device 100. In another example, the signal transmission component controls a warning light disposed on the main hand control device 100 to be enabled to issue a warning to the operator. In some embodiments, the signal transmission component may control the force feedback component 130 to adjust a motion resistance applied to the rod structure 111 to a maximum value, to prevent the rod structure 111 from further movement.

In some practical application scenarios, performing a puncture operation by a remotecontrolled image-guided puncture robot cannot provide feedback on a magnitude of resistance encountered by a puncture needle during the puncture process, and thus cannot effectively simulate an actual puncture process. A lack of force perception by an operator may increase surgical risks and uncertainties, simultaneously increase surgical time, reduce surgical efficiency, and affect a success rate of the puncture operation.

In some cases, by providing the force feedback component 130, an axial motion resistance may be applied to the rod structure 111, so that the operator can perceive the motion resistance when gripping the rod structure 111 to perform linear movement, thereby simulating an actual puncture process and improving the success rate of the puncture operation.

It should be noted that the structures of the force transmission unit 132 and the resistance transmission member 113 shown in FIGs. 5 and 6 are merely for illustrative purposes, and are not intended to limit the specific structures of the force transmission unit 132 and the resistance transmission member 113. In some embodiments, the specific structures of the force transmission unit 132 and the resistance transmission member 113 may be related to a type of motion resistance. In some embodiments, the motion resistance may be friction resistance, transmission force, or the like. Merely by way of example, the motion resistance may be friction resistance. For example, the force output unit 131 may include a linear motion motor. The force transmission unit 132 may include a friction transmission plate, the friction transmission plate including a friction force transmission surface. The resistance transmission member 113 may include a friction receiving plate, the friction receiving plate including a friction force receiving surface. The friction receiving plate is disposed on the rod structure 111, the friction transmission plate is connected to an output shaft of the linear motion motor, and the friction force transmission surface of the friction transmission plate abuts against the friction force receiving surface of the friction receiving plate. Forward rotation and reverse rotation of the linear motion motor can control the pressure between the friction transmission plate and the friction receiving plate, thereby increasing or decreasing the friction resistance between the friction transmission plate and the friction receiving plate. In another example, when the force transmission unit 132 is a friction transmission plate, the resistance transmission member 113 may not be provided, that is, friction resistance may be directly received via a surface of the rod structure 111. In another example, the motion resistance may be transmission force. For example, the force output unit 131 may be the force feedback motor 1311 in the foregoing embodiments, the force transmission unit 132 may include a coupler 1321 and the gear 1322 in the foregoing embodiments, and the resistance transmission member 113 may be the rack 1131 in the foregoing embodiments. In another example, when the force output unit 131 is the force feedback motor 1311, the force transmission unit 132 may include a worm wheel and the coupler 1321, the worm wheel is connected to the coupler 1321, and the resistance transmission member 113 may be a worm adapted to the worm wheel, the worm is disposed on the rod structure 111. In yet another example, the force transmission unit 132 may include a transmission wheel and the coupler 1321, and the resistance transmission member 113 may include a conveyor belt adapted to the transmission wheel, the conveyor belt is disposed on the rod structure 111.

In some practical application scenarios, attitude adjustment of a puncture needle and needle advance/retraction operations of the puncture needle cannot be performed simultaneously, to avoid causing damage to human tissue. That is, when the operator adjusts the attitude of the rod structure 111 through the attitude adjustment execution component 120, the rod structure 111 cannot be controlled to move along its own axial direction. Conversely, when the operator controls the rod structure 111 to move along its own axial direction, the attitude of the rod structure 111 cannot be adjusted through the attitude adjustment execution component 120.

In some embodiments, the main hand control device 100 further comprises a linear guiding component 140. The linear guiding component 140 is configured to restrict the rod structure 111 to always perform linear motion along its own axial direction, so as to simulate the process of the operator holding a needle and puncturing during the puncture operation. Relevant descriptions of the linear guiding component 140 may refer to FIG. 2 and embodiments thereof.

In some embodiments, as shown in FIG. 2, the linear guiding component 140 includes a linear rail 141 and a slider 142 capable of sliding along the linear rail 141. The linear rail 141 is disposed on the rod structure 111. An arrangement direction of the linear rail 141 is parallel to the axial direction of the rod structure 111. Merely by way of example, the slider 142 may be connected to the passive degree-of-freedom connection component 126 (For example, a passive degree-of-freedom mounting seat 1261). The linear rail 141 may be connected to the rack 1131 on the rod structure 111, such that the rod structure 111 may move relative to the slider.

In some embodiments, the linear rail 141 and the rack 1131 may be fixedly connected. Exemplary fixed connection manners may include welding, riveting, adhesion, or the like. In some embodiments, the linear rail 141 and the rack 1131 may be detachably connected. Exemplary detachable connection manners may include magnetic connection, snap connection, screw connection, or the like. Similarly, in some embodiments, the slider 142 and the passive degree-of-freedom mounting seat 1261 may be fixedly connected. In other embodiments, the slider 142 and the passive degree-of-freedom mounting seat 1261 may be detachably connected.

In some embodiments, in addition to a form of the slider 142 and the linear rail 141, the linear guiding component 140 may further include a magnetic attraction component, a worm gear component, or the like, details of which are not repeated here.

In some cases, the main hand control device 100 of the present disclosure improves puncture and attitude adjustment approaches by adopting the rod structure 111 and the linear guiding component 140 to simulate clinical puncture actions, which not only better conforms to a clinical puncture process, but also better meets human-machine interaction requirements under master-slave teleoperation.

The beneficial effects of the main hand control device for a robot provided by the present disclosure may include, but are not limited to: (1) The main hand control device can simulate linear motion generated when the operator holds a needle for puncture during the puncture operation, so that the operator can perceive the feeling of clinical puncture as much as possible, and improve the success rate of the puncture operation. (2) Based on the puncture needle execution component, the main hand control device can truly simulate a puncture process of the puncture device and can realize controlling the puncture device to advance the needle while rotating, which is beneficial for improving the success rate of the puncture operation. Based on the attitude adjustment execution component, the main hand control device allows the rod structure to swing relative to the attitude adjustment execution component, so that the rod structure can adapt to the operator's grip posture, and improves human-machine interaction comfort. (3) The puncture needle execution component of a master-slave control device can obtain a puncture execution signal (such as a rotation angle of the rod structure relative to the attitude adjustment execution component and a position of the rod structure along its own axial direction) by disposing an angle detection component and a first position detection module. This enables the puncture needle drive module to perform a corresponding puncture operation based on the puncture execution signal, and to realize master-slave puncture motion control. (4) The attitude adjustment execution component of the main hand control device adopts a parallel configuration, and the attitude adjustment execution component is provided with a damper for adaptive attitude adjustment, which can correspondingly change a damping value based on the position of the rod structure along its own axial direction, to ensure constant damping during an attitude adjustment process. Furthermore, the attitude adjustment execution component is further provided with a homing component and is provided with a homing angle sensor, which can detect a rotation speed of an output shaft of a homing motor in real time, and can ensure homing accuracy to a certain extent. (5) The main hand control device combines expert trial and clinical feedback requirements, and improves the puncture and attitude adjustment approaches by adopting an operation needle handle and the linear guiding component to simulate clinical puncture actions, which not only better conforms to the clinical puncture process, but also better meets human-machine interaction requirements of a master end under master-slave teleoperation. (6) After disposing the force feedback component, when the operator holds the rod structure to move along the axial direction of the rod structure, a motion resistance may be applied to the rod structure through the force feedback component, so that the operator perceives the motion resistance received by the puncture needle during actual puncturing, thereby simulating an actual puncture process and improving the success rate of the puncture operation. (7) Since there is a plurality of enable buttons, the operator can therefore select a more suitable grip posture for holding, which can effectively improve the operator's operating experience.

Some embodiments of the present disclosure provide a puncture device 200. In some embodiments, in conjunction with FIGs. 21A-23, the puncture device 200 includes a puncture needle drive module 210 and an attitude control module 220. The puncture needle drive module 210 includes an advance-retreat drive mechanism 213. The advance-retreat drive mechanism 213 is connected to a puncture needle 211. In some embodiments, the attitude control module 220 is provided with the advance-retreat drive mechanism 213. The advance-retreat drive mechanism 213 is configured to drive the puncture needle 211 to move relative to the attitude control module 220 along an axial direction of the puncture needle 211.

The puncture needle drive module 210 is a main structure of the puncture device 200 for driving the puncture needle 211 to perform an advance needle operation or a retreat needle operation. In some embodiments, the puncture needle drive module 210 can perform corresponding movements based on real-time movement conditions of the puncture needle execution component 110 (such as movement of the rod structure 111 along its own axial direction and rotation about its own axial direction), so as to perform the advance needle operation or the retreat needle operation.

The attitude control module 220 is a main structure of the puncture device 200 for controlling an attitude of the puncture needle 211. In some embodiments, the attitude control module 220 can control the attitude adjustment drive module 223 to perform corresponding rotations based on rotation angle information of the rod structure 111 relative to the attitude adjustment execution component 120 (such as a rotation angle of a first connecting rod 1211 relative to a base 123, a rotation angle of a second connecting rod 1221 relative to the base 123, or the like). This aims to control an attitude of the puncture needle 211, enabling the puncture needle 211 to aim at a target puncture target point, and ensuring accuracy of the puncture operation.

In some embodiments, the puncture device 200 further comprises a second signal transmission component (not shown in the figure). The second signal transmission component communicates with/is connected to the puncture needle drive module 210 and the attitude control module 220. The second signal transmission component can receive various signals fed back by the puncture needle drive module 210 and the attitude control module 220 (such as rotation angle information, puncture force feedback information, or the like), and outputs a corresponding control signal based on the received signals, to meet use requirements of different scenarios (such as an attitude adjustment scenario, a puncture scenario, or the like).

In some embodiments, the second signal transmission component may communicate with/be connected to a processor of a robot, for establishing signal transmission between the puncture device 200 and the robot, and realizing information interaction between the puncture device 200 and the robot. In some embodiments, the second signal transmission component may include, but is not limited to, Ethernet, serial ports, wireless communication, CAN bus, EtherCAT bus, or the like. For example, the second signal transmission component can realize information interaction through Ethernet. It should be noted that the second signal transmission component and a first signal transmission component may also be integrated into a single signal transmission component. Communication with/connection to the processor of the robot by the main hand control device 100 and the puncture device 200 can be realized based on a single signal transmission component.

In some embodiments, the puncture needle drive module 210 includes a rotary drive mechanism 212. The rotary drive mechanism 212 is for connecting to the puncture needle 211, and the rotary drive mechanism 212 is in transmission connection with the advance-retreat drive mechanism 213. The rotary drive mechanism 212 is disposed on the attitude control module 220. The rotary drive mechanism 212 is configured to drive the puncture needle 211 to rotate relative to the attitude control module 220 about an axial direction of the puncture needle 211.

The rotary drive mechanism 212 is a main structure for driving the puncture needle 211 to rotate about its own axial direction.

In some embodiments, in conjunction with FIGs. 21A-23, the rotary drive mechanism 212 includes a rotary drive motor 2121, a fourth angle sensor 2122, and a fourth brake 2123. The rotary drive motor 2121 is configured to drive the puncture needle 211 to rotate about its own axial direction. The fourth angle sensor 2122 is configured to detect a rotation angle of an output shaft of the rotary drive motor 2121, so as to realize rotation angle detection of the puncture needle 211 and its closed-loop control. The fourth brake 2123 is disposed between the puncture needle 211 and the rotary drive motor 2121. The fourth brake 2123 is configured to restrict the puncture needle 211 from rotating about its own axial direction, so as to ensure that the rotation angle of the puncture needle 211 is always consistent with the rotation angle of the rod structure 111 rotating about its own axial direction.

In some embodiments, the rotary drive mechanism 212 is fixedly connected to the puncture needle 211. The fourth angle sensor 2122 is disposed at one end of the rotary drive motor 2121, and the fourth brake 2123 is disposed at another end of the rotary drive motor 2121. In some embodiments, the fourth angle sensor 2122 can detect a rotation angle of an output shaft of the rotary drive motor 2121 in real time and transmit the rotation angle to the processor of the robot based on the second signal transmission component. The processor may control the rotary drive motor 2121 to work based on a rotation angle of an interaction handle 1110 rotating about the axial direction of the rod structure 111 and the rotation angle of the output shaft of the rotary drive motor 2121. This ensures that the rotation angle of the output shaft of the rotary drive motor 2121 (that is, the rotation angle of the puncture needle 211 rotating about its own axial direction) always remains consistent with the rotation angle of the interaction handle 1110 rotating about the axial direction of the rod structure 111, thereby realizing rotation control of the puncture needle 211 disposed on the puncture device 200 (slave end) by the main hand control device 100 (master end). Merely by way of example, when the operator controls the interaction handle 1110 to rotate about the axial direction of the rod structure 111, the angle detection component 114 obtains a rotation angle of the interaction handle 1110 rotating about the axial direction of the rod structure 111, and transmits the rotation angle to the processor of the robot through a first signal transmission component. The processor controls the rotary drive motor 2121 to start working, so as to drive the puncture needle 211 to rotate about its own axial direction. Based on real-time feedback of the fourth angle sensor 2122, this makes the rotation angle of the output shaft of the rotary drive motor 2121 (that is, the rotation angle of the puncture needle 211 rotating about its own axial direction) consistent with the rotation angle of the interaction handle 1110 rotating about the axial direction of the rod structure 111. When the interaction handle 1110 stops rotating, the processor controls the rotary drive motor 2121 to stop working and simultaneously controls the fourth brake 2123 to be engaged, to realize restriction of the puncture needle 211 from rotating about its own axial direction, so as to further ensure consistency of the rotation angle.

In some embodiments of the present disclosure, the rotary drive mechanism 212, by adopting the rotary drive motor 2121, the fourth angle sensor 2122, and the fourth brake 2123, can realize rotation control of the puncture needle 211 disposed on the puncture device 200 (slave end) by the main hand control device 100 (master end). Furthermore, by disposing the fourth angle sensor 2122 and the fourth brake 2123, consistency of the rotation angle can be effectively ensured, and control accuracy can be improved.

The advance-retreat drive mechanism 213 is a main structure for driving the puncture needle 211 to perform an advance operation or a retreat operation. In some embodiments, as shown in FIGs. 21A-23, the advance-retreat drive mechanism 213 includes an advance-retreat drive motor 2131, a fifth angle sensor 2132, and a fifth brake 2133. The advance-retreat drive motor 2131 is configured to drive the puncture needle 211 to move along its own axial direction. The fifth angle sensor 2132 is configured to detect a rotation angle of an output shaft of the advance-retreat drive motor 2131, so as to achieve detection of the rotation angle of the output shaft of the advance-retreat drive motor 2131 and its closed-loop control. The fifth brake 2133 is disposed between the puncture needle 211 and the advance-retreat drive motor 2131. The fifth brake 2133 is configured to restrict the puncture needle 211 from moving along its own axial direction, so as to ensure that the rotation angle of the output shaft of the advance-retreat drive motor 2131 is always consistent with the rotation angle of the output shaft of the force feedback motor 1311.

In some embodiments, the advance-retreat drive mechanism 213 is in transmission connection with the puncture needle 211. The fifth angle sensor 2132 is disposed at one end of the advance-retreat drive motor 2131. The fifth brake 2133 is disposed at another end of the advance-retreat drive motor 2131. In some embodiments, the fifth angle sensor 2132 can detect, in real time, the rotation angle of the output shaft of the advance-retreat drive motor 2131 and transmits the rotation angle through a second signal transmission component to a processor of the robot. The processor can control the advance-retreat drive motor 2131 to operate based on the rotation angle of the output shaft of the force feedback motor 1311 and the rotation angle of the output shaft of the advance-retreat drive motor 2131, so that the rotation angle of the output shaft of the force feedback motor 1311 is always consistent with the rotation angle of the output shaft of the advance-retreat drive motor 2131. This thereby realizes speed control of the advance-retreat drive motor 2131, and further controls a puncture speed of the puncture needle 211. Merely by way of example, when an operator controls the rod structure 111 to move along its own axial direction, the first angle sensor 1323 acquires the rotation angle of the output shaft of the force feedback motor 1311 and transmits the rotation angle through a first signal transmission component to the processor of the robot. The processor controls the advance-retreat drive motor 2131 to operate, so as to drive the puncture needle 211 to move along its own axial direction. Based on real-time feedback of the fifth angle sensor 2132, the processor makes the rotation angle of the output shaft of the advance-retreat drive motor 2131 consistent with the rotation angle of the output shaft of the force feedback motor 1311. When the rod structure 111 stops moving along its own axial direction, the processor can achieve restriction of the puncture needle 211 from moving along its own axial direction by controlling the advance-retreat drive motor 2131 to stop operating and simultaneously controlling the fifth brake 2133 to be engaged, so as to further ensure consistency of the rotation angle.

In some embodiments of the present disclosure, by adopting the advance-retreat drive motor 2131, the fifth angle sensor 2132, and the fifth brake 2133, the advance-retreat drive mechanism 213 may realize control of a puncture speed of the puncture needle 211, which is arranged on the puncture device 200 (slave end), by the main hand control device 100 (master end). Furthermore, by providing the fifth angle sensor 2132 and the fifth brake 2133, consistency between the rotation angle of the output shaft of the advance-retreat drive motor 2131 and the rotation angle of the output shaft of the force feedback motor 1311 can be effectively ensured, thereby improving control accuracy.

In some embodiments, as shown in FIGs. 21A-22, the advance-retreat drive mechanism 213 further includes a linear movement component. The linear movement component includes a linear rail 2134, a slider 2135, and a mounting seat 2136. The linear rail 2134 is arranged parallel to the puncture needle 211. The slider 2135 is in transmission connection with the advance-retreat drive motor 2131, and the slider 2135 is slidably connected to the linear rail 2134. The mounting seat 2136 is fixedly connected to the slider 2135, and the puncture needle 211 is mounted on the mounting seat 2136. The mounting seat 2136 is fixedly connected to the rotary drive mechanism 212. The advance-retreat drive mechanism 213 drives the mounting seat 2136 to move along the linear rail 2134, thereby causing the puncture needle 211 to move along its own axial direction.

In some embodiments, the advance-retreat drive mechanism 213 may further include a transmission mechanism 2137. The slider 2135 is in transmission connection with the advance-retreat drive motor 2131 through the transmission mechanism 2137. The transmission mechanism 2137 refers to a component capable of realizing power transmission. In some embodiments, the transmission mechanism 2137 may include a ball nut 21371 and a ball screw 21372. The ball nut 21371 is fixedly connected to the mounting seat 2136, and the ball screw 21372 is in transmission connection with the advance-retreat drive motor 2131. In some embodiments, the advance-retreat drive mechanism 213 further includes a second coupler 2138. The ball screw 21372 is fixedly connected to the output shaft of the advance-retreat drive motor 2131 through the second coupler 2138. Rotating of the advance-retreat drive motor 2131 can drive the ball screw 21372 to rotate. The ball nut 21371 converts the rotation of the ball screw 21372 into linear movement and drives the mounting seat 2136 to move along the linear rail 2134, thereby causing the puncture needle 211 to move along its own axial direction. It should be noted that, in addition to a form of the ball nut 21371 and the ball screw 21372, the transmission mechanism 2137 may further include a gear-rack structure, a worm gear structure, or the like.

In some embodiments of the present disclosure, by providing the linear movement component, stability when the puncture needle 211 performs a puncture operation can be effectively ensured, so as to improve puncture accuracy and avoid accidental injury to a patient.

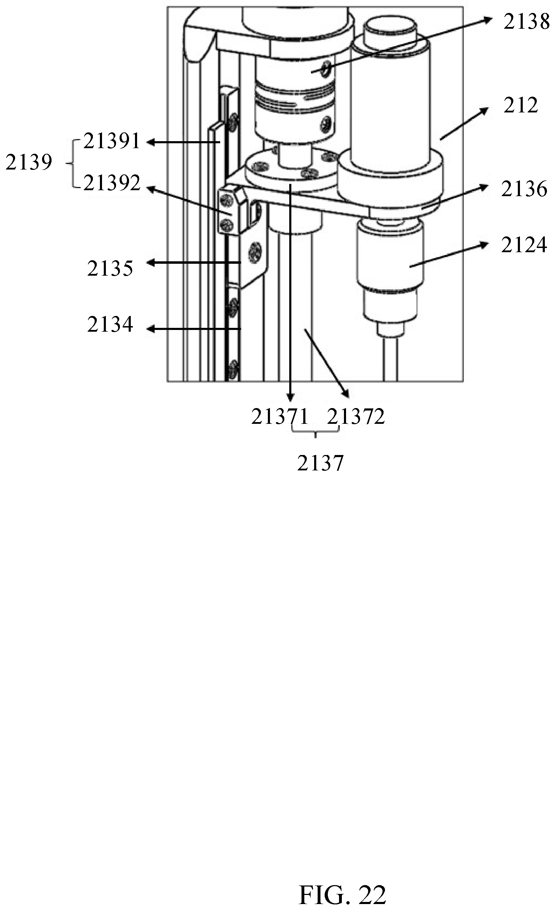

In some embodiments, as shown in FIG. 22, the advance-retreat drive mechanism 213 further includes the second position detection module 2139. The second position detection module 2139 is configured to acquire a position of the puncture needle 211 in its own axial direction. The second position detection module 2139 includes a second linear scale 21391 and a second linear scale reading member 21392. The second linear scale 21391 is disposed on the linear rail 2134 along an axial direction of the linear rail 2134, and the second linear scale reading member 21392 is disposed on the slider 2135.

In some embodiments, the second position detection module 2139 may be a laser rangefinder, a displacement encoder, a linear scale, an inductive displacement sensor, or the like. In some embodiments, the second position detection module 2139 is a linear scale. The second linear scale 21391 is a scale grating, and the second linear scale reading member 21392 includes a light source, a lens, an indicator grating, or the like. When a light source illuminates the scale grating in parallel, Moire fringes may appear in the second linear scale reading member 21392. When the second linear scale 21391 and the second linear scale reading member 21392 move relative to each other, the second linear scale reading member 21392 can read a grating scale by detecting the number of the Moire fringes and convert the grating scale into an electrical signal. Position information can be determined by obtaining through calculation. In some embodiments, when the slider 2135 moves along the linear rail 2134, the second linear scale reading member 21392 can read position information of the second linear scale 21391, thereby determining a position of the puncture needle 211 in its own axial direction. It can be understood that the second linear scale reading member 21392 is capable of real-time acquiring the position of the puncture needle 211 in its own axial direction and transmits the position through the second signal transmission component to the processor of the robot. The processor can control the advance-retreat drive motor 2131 to operate based on the position of the rod structure 111 in its own axial direction and the position of the puncture needle 211 in its own axial direction, so as to make a linear movement distance of the rod structure 111 consistent with a linear movement distance of the puncture needle 211 or satisfy a mapping ratio, thereby controlling a puncture depth of the puncture needle 211. Merely by way of example, when the operator controls the rod structure 111 to move along its own axial direction, the first position detection module 112 acquires the position of the rod structure 111 in its own axial direction and transmits the position through the first signal transmission component to the processor of the robot. The processor controls the advance-retreat drive motor 2131 to operate, so as to drive the puncture needle 211 to move along its own axial direction. Based on real-time feedback of the second position detection module 2139, the processor makes the linear movement distance of the rod structure 111 consistent with the linear movement distance of the puncture needle 211 or satisfy a mapping ratio. When the rod structure 111 stops moving, the processor controls the advance-retreat drive motor 2131 to stop operating.

In some embodiments of the present disclosure, the main hand control device 100 and the puncture device 200 both adopt a linear scale detection system to respectively detect the linear movement distance of the rod structure 111 and the linear movement distance of the puncture needle 211, which may enable detection results not to be limited by transmission stiffness of the device, thereby effectively ensuring detection accuracy and improving control accuracy.

In some embodiments, as shown in FIGs. 21A-22, the advance-retreat drive mechanism 213 further includes a mounting base 2140. The linear rail 2134 and the second linear scale 21391 are both fixedly connected to the mounting base 2140. The mounting base 2140 is provided with a slewing bearing 21401. The slewing bearing 21401 is configured to support the ball screw 21372.

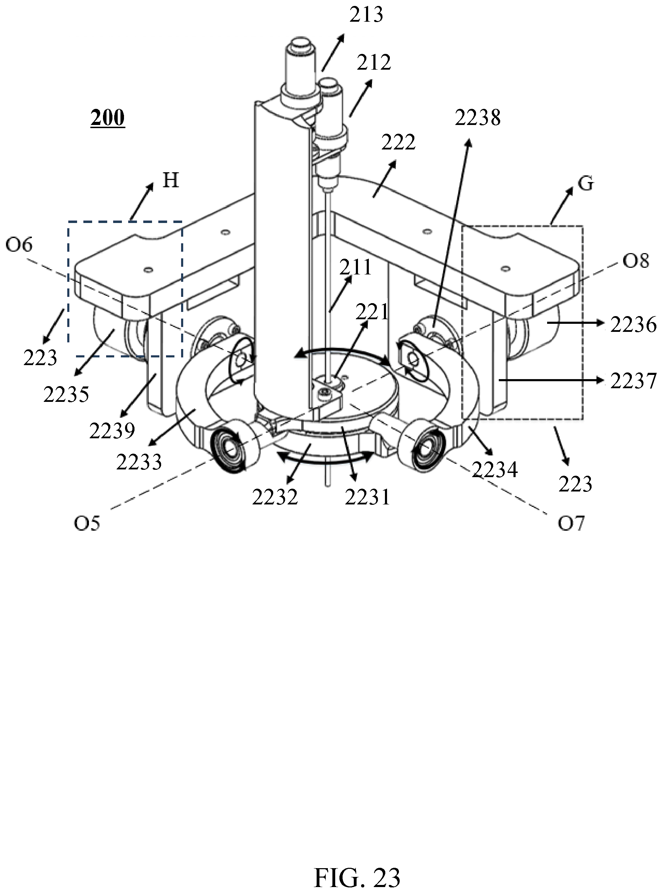

In some embodiments, the mounting base 2140 is fixedly connected to the first passive ring 2231 of the attitude control module 220. Exemplary fixed connection manners include threaded connection, welding, or the like. For specific details regarding the first passive ring 2231, reference may be made to FIG. 23 and related description in the subsequent text.

It can be understood that, since the mounting base 2140 is provided with the slewing bearing 21401 and the ball screw 21372 passes through the slewing bearing 21401, the advance-retreat drive motor 2131 can easily drive the ball screw 21372 to rotate. Moreover, adopting the slewing bearing 21401 can also ensure stability during rotation of the ball screw 21372 to a certain extent, so as to ensure stability when the puncture needle 211 performs a puncture operation.

In some embodiments, since the puncture needle 211 may be subjected to resistance from human tissues (such as skin, or the like) when performing an advance or retreat operation, in order to enable the main hand control device 100 (such as the force feedback component 130) to accurately provide simulated force feedback during puncture of the puncture needle 211 to an operator in master-slave teleoperation, and further simulate a linear movement generated when a doctor holds a needle for puncturing during a puncture surgery, so as to make an operation process safer and more efficient, and improve puncture accuracy, the puncture needle drive module 210 further includes a force sensor.

In some embodiments, as shown in FIGs. 21A-22, the rotary drive mechanism 212 further includes the first force sensor 2124. The first force sensor 2124 is disposed between the puncture needle 211 and the puncture needle drive module 210. The first force sensor 2124 is configured to acquire puncture force feedback information during puncture of the puncture needle 211. The puncture force feedback information is used to determine a puncture motion resistance output by the main hand control device for controlling the puncture device.

In some embodiments, as shown in FIGs. 21A-22, the first force sensor 2124 is disposed between the puncture needle 211 and the puncture needle drive module 210 (such as the fourth brake 2123). Exemplary, the first force sensor includes but is not limited to a variable type force sensor, a capacitive force sensor, an electrode bending type force sensor, or the like. In some embodiments, the first force sensor 2124 can real-time acquire puncture force feedback information during puncture of the puncture needle 211 and transmits the puncture force feedback information through the second signal transmission component to the processor of the robot. The processor controls the force output unit 131 of the force feedback component 130 based on the puncture force feedback information. The force output unit 131 then outputs a puncture motion resistance, which is equivalent to resistance experienced by the puncture needle during a puncture process, to the rod structure 111 through the resistance transmission member 113. In this manner, when an operator operates the main hand control device 100 to perform a puncture operation, the operator can feel resistance received by the puncture needle 211 through the puncture motion resistance fed back by the force feedback component 130, thereby truly simulating a situation of holding a needle for puncturing, so as to control the puncture device 200 more accurately, and further improve puncture accuracy. For further description regarding puncture force feedback, reference may be made to FIGs. 6-7 and related description.

In some embodiments, as shown in FIGs. 21A-23, the attitude control module 220 includes an attitude adjustment base 222 and an attitude adjustment drive module 223. The attitude adjustment drive module 223 is arranged on the attitude adjustment base 222. The puncture needle drive module 210 is mounted to the attitude adjustment base 222 through the attitude adjustment drive module 223. The attitude adjustment drive module 223 drives the puncture needle 211 to adjust an attitude of the puncture needle 211 relative to the attitude adjustment base 222.

The attitude adjustment base 222 is configured to be a structure for mounting the attitude adjustment drive module 223. During attitude adjustment, the attitude adjustment base 222 always remains stationary. In some embodiments, the attitude adjustment base 222 is fixedly connected to the attitude adjustment drive module 223. The puncture needle 211 can be mounted to the attitude adjustment base 222 through a guiding structure 221 disposed on the first passive ring 2231.

In some embodiments, as shown in FIGs. 21A-24, the attitude adjustment drive module 223 includes the first passive ring 2231, a second passive ring 2232, a first coupling rod 2233, a second coupling rod 2234, a first attitude adjustment drive mechanism 2235, and a second attitude adjustment drive mechanism 2236. The first passive ring 2231 and the second passive ring 2232 are coaxial and rotatably connected to the attitude adjustment base 222. The puncture needle 211 passes through the first passive ring 2231 and the second passive ring 2232 along an axial direction of the first passive ring 2231. The first passive ring 2231 is rotatably connected to one end of the first coupling rod 2233, the other end of the first coupling rod 2233 is rotatably connected to the attitude adjustment base 222, and the first attitude adjustment drive mechanism 2235 is further arranged at the other end of the first coupling rod 2233. The second passive ring 2232 is rotatably connected to one end of the second coupling rod 2234, the other end of the second coupling rod 2234 is rotatably connected to the attitude adjustment base 222, and the second attitude adjustment drive mechanism 2236 is further arranged at the other end of the second coupling rod 2234.

In some embodiments, for convenience of description, an axis of rotation of the first coupling rod 2233 relative to the first passive ring 2231 may be referred to as a fifth rotation axis (as shown as O5 in FIG. 23), and an axis of rotation of the first coupling rod 2233 relative to the attitude adjustment base 222 may be referred to as a sixth rotation axis (as shown as O6 in FIG. 23). An axis of rotation of the second coupling rod 2234 relative to the second passive ring 2232 may be referred to as a seventh rotation axis (as shown as O7 in FIG. 23), and an axis of rotation of the second coupling rod 2234 relative to the attitude adjustment base 222 may be referred to as an eighth rotation axis (as shown as O8 in FIG. 23). In some embodiments, the fifth rotation axis O5, the sixth rotation axis O6, the seventh rotation axis O7, and the eighth rotation axis O8 may be in the same plane. In some embodiments, the fifth rotation axis O5, the sixth rotation axis O6, the seventh rotation axis O7, and the eighth rotation axis O8 may not be in the same plane. Merely by way of example, since the attitude adjustment base 222 always remains stationary, the sixth rotation axis O6 and the eighth rotation axis O8 always remain unchanged. The fifth rotation axis O5 and the seventh rotation axis O7 respectively change with movement of the first passive ring 2231 and the second passive ring 2232. In this case, the fifth rotation axis O5, the sixth rotation axis O6, the seventh rotation axis O7, and the eighth rotation axis O8 may not be in the same plane. For example, the sixth rotation axis O6 and the eighth rotation axis O8 are in the same plane, and the fifth rotation axis O5 and the seventh rotation axis O7 are in the same plane. For example, the fifth rotation axis O5 and the eighth rotation axis O8 are in the same plane, and the sixth rotation axis O6 and the seventh rotation axis O7 are in the same plane.

In the present embodiment, since the first passive ring 2231 and the second passive ring 2232 are arranged coaxially, and the puncture needle 211 passes through the first passive ring 2231 and the second passive ring 2232 along an axial direction of the first passive ring 2231, the puncture needle 211 always maintains a coaxial relationship with the first passive ring 2231 and the second passive ring 2232, that is, an axial direction of the puncture needle 211 coincides with a central axis of the first passive ring 2231 and a central axis of the second passive ring 2232. Therefore, when the first passive ring 2231 and the second passive ring 2232 swing relative to the attitude adjustment base 222, the first passive ring 2231 and the second passive ring 2232 can drive the puncture needle 211 to swing relative to the attitude adjustment base 222. Since the first coupling rod 2233 is disposed between the first passive ring 2231 and the attitude adjustment base 222, and the first attitude adjustment drive mechanism 2235 is further disposed at the other end of the first coupling rod 2233, when the first attitude adjustment drive mechanism 2235 rotates relative to the attitude adjustment base 222, the first attitude adjustment drive mechanism 2235 drives the first coupling rod 2233 to rotate about the sixth rotation axis, which further drives the first coupling rod 2233 to rotate about the fifth rotation axis, thereby driving the first passive ring 2231 to swing relative to the attitude adjustment base 222. Similarly, since the second coupling rod 2234 is disposed between the second passive ring 2232 and the attitude adjustment base 222, and the second attitude adjustment drive mechanism 2236 is further disposed at the other end of the second coupling rod 2234, when the second attitude adjustment drive mechanism 2236 rotates relative to the attitude adjustment base 222, the second attitude adjustment drive mechanism 2236 drives the second coupling rod 2234 to rotate about the eighth rotation axis, which further drives the second coupling rod 2234 to rotate about the eighth rotation axis O8, thereby driving the second passive ring 2232 to swing relative to the attitude adjustment base 222.

It should be noted that, since two ends of the first coupling rod 2233 are respectively rotatably connected to the first passive ring 2231 and the attitude adjustment base 222, and two ends of the second coupling rod 2234 are respectively rotatably connected to the second passive ring 2232 and the attitude adjustment base 222. That is, the first coupling rod 2233 and the second coupling rod 2234 are simultaneously rotatably connected to the attitude adjustment base 222, which means that the first coupling rod 2233 and the second coupling rod 2234 are a parallel structure. When the first coupling rod 2233 rotates about the sixth rotation axis O6, it drives the parallel second coupling rod 2234 to rotate about the seventh rotation axis O7. When the second coupling rod 2234 rotates about the eighth rotation axis O8, it drives the parallel first coupling rod 2233 to rotate about the fifth rotation axis O5. That is to say, the attitude adjustment drive module 223 of the puncture device 200 and the attitude adjustment execution component 120 of the main hand control device 100 both adopt a parallel configuration, and the configurations are substantially the same. Therefore, a movement process of the puncture needle 211 relative to the attitude control module 220 can also be simplified to a form shown in FIG. 16. The fifth rotation axis O5 (equivalent to a first rotation axis O1), the sixth rotation axis O6 (equivalent to a second rotation axis O2), the seventh rotation axis O7 (equivalent to a third rotation axis O3), and the eighth rotation axis O8 (equivalent to a fourth rotation axis O4) are in the same plane. In this case, an angle between the fifth rotation axis O5 and the eighth rotation axis O8 is 180°, and an angle between the sixth rotation axis O6 and the seventh rotation axis O7 is 180°. The puncture needle 211 can rotate relative to the attitude control module 220 in a plane formed by the fifth rotation axis O5 and the eighth rotation axis O8 (such as a vertical plane), that is, has a third degree of freedom (as shown by an arrow M3). The puncture needle 211 can rotate relative to the attitude control module 220 in a plane formed by the sixth rotation axis O6 and the seventh rotation axis O7 (such as a vertical plane), that is, has a fourth degree of freedom (as shown by an arrow M4).

It should be noted that, since the fifth rotation axis O5 and the seventh rotation axis O7 respectively change with movement of the first passive ring 2231 and the second passive ring 2232, an angle between the fifth rotation axis O5 and the eighth rotation axis O8 or an angle between the sixth rotation axis O6 and the seventh rotation axis O7 does not always remain 180°, and may also be 150°, 160°, or the like. When the angle between the fifth rotation axis O5 and the eighth rotation axis O8 and the angle between the sixth rotation axis O6 and the seventh rotation axis O7 are both 180°, that is, the fifth rotation axis O5 and the eighth rotation axis O8 are collinear and form a third rotation axis (equivalent to a first rotation axis), and the sixth rotation axis O6 and the seventh rotation axis O7 are collinear and form a fourth rotation axis (equivalent to a second rotation axis), in this case, a plane formed by the fifth rotation axis O5 and the eighth rotation axis O8 (that is, a plane where the third rotation axis is located) may be parallel to a horizontal plane, and may also be not parallel to the horizontal plane, and a plane formed by the sixth rotation axis O6 and the seventh rotation axis O7 (that is, a plane where the fourth rotation axis is located) may also be parallel to the horizontal plane or not parallel to the horizontal plane. When the angle between the fifth rotation axis O5 and the eighth rotation axis O8 and the angle between the sixth rotation axis O6 and the seventh rotation axis O7 are not 180°, the eighth rotation axis O8 forms the third rotation axis, and the sixth rotation axis O6 forms the fourth rotation axis. In some embodiments, the third rotation axis and the fourth rotation axis always intersect and are perpendicular. Based on the above content, it can be known that when the parallel first coupling rod 2233 and the second coupling rod 2234 rotate together, the puncture needle 211 will swing.

In some embodiments, the angle between the fifth rotation axis O5 and the seventh rotation axis O7 is greater than 10 degrees. In some embodiments, the angle between the fifth rotation axis O5 and the seventh rotation axis O7 is greater than 45 degrees. In some embodiments, the angle between the fifth rotation axis O5 and the seventh rotation axis O7 is greater than 60 degrees. In some embodiments, as shown in FIG. 23, the angle between the fifth rotation axis O5 and the seventh rotation axis O7 is 90 degrees, so that the attitude control module 220 obtains a larger operating space. Similarly, in some embodiments, the angle between the sixth rotation axis O6 and the eighth rotation axis O8 is greater than 10 degrees. In some embodiments, as shown in FIG. 23, the angle between the sixth rotation axis O6 and the eighth rotation axis O8 is 90 degrees.

In some embodiments, the shape of the first coupling rod 2233 is adapted to the shape of the first passive ring 2231, and the shape of the second coupling rod 2234 is adapted to the shape of the second passive ring 2232. Merely by way of example, as shown in FIG. 23, the first passive ring 2231 and the second passive ring 2232 are disc-shaped structures, the first coupling rod 2233 and the second coupling rod 2234 are arc-shaped connecting rods, and contours of the first coupling rod 2233 and the first passive ring 2231 are both circular rings. A curvature of the arc-shaped connecting rods is the same as a curvature of the circular rings, so that the first coupling rod 2233 will not always collide with the first passive ring 2231 during rotation relative to the first passive ring 2231, and the second coupling rod 2234 will not always collide with the second passive ring 2232 during rotation relative to the second passive ring 2232. At the same time, a structure can also be made more compact. It should be noted that, the first coupling rod 2233 and the second coupling rod 2234 may also be designed into any other feasible shapes (such as a right-angle type, or the like) as long as they can achieve corresponding connection functions.

In some embodiments, as shown in FIGs. 23-24, the attitude adjustment drive module 223 further includes a second attitude adjustment connecting shaft (not shown in the figures), a second attitude adjustment support seat 2237, and a second attitude adjustment end cap 2238. The second attitude adjustment support seat 2237 is disposed on the attitude adjustment base 222. One end of the second attitude adjustment connecting shaft is connected to another end of the second coupling rod 2234. Another end of the second attitude adjustment connecting shaft is connected to the second attitude adjustment support seat 2237 through a second attitude adjustment bearing (not shown in the figures). The second attitude adjustment end cap 2238 fixes the second attitude adjustment bearing on the second attitude adjustment support seat 2237. The second coupling rod 2234 is capable of rotating relative to the second attitude adjustment bearing and the second attitude adjustment support seat 2237, thereby realizing rotation relative to the attitude adjustment base 222. In some embodiments, the attitude adjustment drive module 223 further includes a first attitude adjustment connecting shaft, a first attitude adjustment support seat 2239, and a first attitude adjustment end cap. The first attitude adjustment connecting shaft, the first attitude adjustment support seat 2239, and the first attitude adjustment end cap are located in a dashed box H in FIG. 21A. A specific arrangement manner of the first attitude adjustment connecting shaft, the first attitude adjustment support seat 2239, and the first attitude adjustment end cap is respectively the same as or similar to the second attitude adjustment connecting shaft, the second attitude adjustment support seat 2237, and the second attitude adjustment end cap 2238.

As described above, in some actual application scenarios, the attitude adjustment of the puncture needle and advance and retreat operations of the puncture needle cannot be performed simultaneously, to avoid causing damage to human tissue. Therefore, in some embodiments, as shown in FIG. 23, the first attitude adjustment drive mechanism 2235 includes a sixth brake (not shown in the figures), a first reducer (not shown in the figures), a first attitude adjustment drive motor (not shown in the figures), and a sixth angle sensor (not shown in the figures). The sixth brake, the first reducer, the first attitude adjustment drive motor, and the sixth angle sensor are disposed at another end of the first coupling rod 2233. The sixth angle sensor is configured to detect a rotation angle of an output shaft of the first attitude adjustment drive motor.

In some embodiments, the second attitude adjustment drive mechanism 2236 includes a seventh brake 22361, a second reducer 22362, a second attitude adjustment drive motor 22363, and a seventh angle sensor 22364. The seventh brake 22361, the second reducer 22362, the second attitude adjustment drive motor 22363, and the seventh angle sensor 22364 are successively disposed at another end of the second coupling rod 2234. The seventh angle sensor 22364 is configured to detect a rotation angle of an output shaft of the second attitude adjustment drive motor 22363.

In some embodiments, when an operator controls the rod structure 111 of the main hand control device 100 to move relative to the attitude adjustment execution component 120 along its own axial direction, the attitude control module 220 of the puncture device 200 may correspondingly adjust the attitude of the puncture needle 211. Merely by way of example, when the operator controls the rod structure 111 of the main hand control device 100 to move relative to the attitude adjustment execution component 120 along its own axial direction, that is, when the operator controls the rod structure 111 to swing relative to the base 1241, the second angle sensor 1213 and the third angle sensor can respectively detect a rotation angle of the first connecting rod 1211 rotating about a second rotation axis O2 and a rotation angle of the second connecting rod 1221 rotating about a fourth rotation axis O4, and transmit the above-mentioned rotation angles to a processor of a robot through a first signal transmission component. Based on the above-mentioned rotation angles, the processor may respectively control the first attitude adjustment drive motor and the second attitude adjustment drive motor 22363 to start working. When the first attitude adjustment drive motor rotates and is decelerated by the first reducer, and drives the first coupling rod 2233 to rotate about a fifth rotation axis O5 and/or about a sixth rotation axis O6, it will drive the first passive ring 2231 to swing relative to the attitude adjustment base 222. When the second attitude adjustment drive motor 22363 rotates and is decelerated by the second reducer 22362, and drives the second coupling rod 2234 to rotate about a seventh rotation axis O7 and/or about an eighth rotation axis O8, it will drive the second passive ring 2232 to swing relative to the attitude adjustment base 222, thereby may cause the puncture needle 211 to swing relative to the attitude adjustment base 222, to adjust the attitude of the puncture needle 211. In some embodiments, the sixth angle sensor and the seventh angle sensor 22364 can respectively feedback in real time the rotation angle of an output shaft of the first attitude adjustment drive motor and the rotation angle of an output shaft of the second attitude adjustment drive motor 22363. The processor may control the first coupling rod 2233 and the second coupling rod 2234 to respectively follow the first connecting rod 1211 and the second connecting rod 1221 to rotate and turn corresponding angles based on the above-mentioned rotation angles, thereby realizing the adjustment of the attitude of the puncture needle 211 disposed on the puncture device 200 by the main hand control device 100. In some embodiments, the sixth angle sensor and the seventh angle sensor 22364 may be an absolute encoder. It can be understood that, by providing corresponding angle sensors, an attitude adjustment drive mechanism may realize rotation angle detection of connecting rods and its closed-loop control.

In some embodiments, the sixth brake and the seventh brake 22361 have similar functions to the fourth brake 2123 and the fifth brake 2133. When the operator controls the rod structure 111 to stop swinging, the processor controls the first attitude adjustment drive motor and the second attitude adjustment drive motor 22363 to stop working, and simultaneously controls the sixth brake and the seventh brake 22361 to be enabled, to respectively restrict the rotation of the output shafts of the first attitude adjustment drive motor and the second attitude adjustment drive motor 22363, thereby may further ensure the consistency of the corresponding rotation angles.

In some embodiments of the present disclosure, by providing brakes and angle sensors, the attitude adjustment drive mechanism is beneficial to ensure that the connecting rods of the puncture device 200 and the connecting rods of the main hand control device 100 rotate at corresponding angles, thereby improving puncture accuracy on the basis of realizing master-slave puncture attitude adjustment.

In some embodiments, the puncture device 200 further includes a second force sensor. In some embodiments, the second force sensor is disposed between the puncture needle 211 and the puncture needle drive module 210. In this case, the second force sensor and the first force sensor 2124 may be the same sensor. In some embodiments, the second force sensor is disposed between the puncture needle 211 and the attitude control module 220. The second force sensor is configured to acquire attitude adjustment force feedback information during attitude adjustment of the puncture needle 211. The attitude adjustment force feedback information is used to determine an attitude adjustment resistance output by the main hand control device 100.

Exemplarily, the second force sensor includes but is not limited to strain force sensors, capacitive force sensors, and electrode bending force sensors, or the like. In some embodiments, the second force sensor can acquire attitude adjustment force feedback information during attitude adjustment of the puncture needle 211 in real time, and transmit the attitude adjustment force feedback information to the processor of the robot through a second signal transmission component. The processor controls the first motor and the second motor 1228 of the attitude adjustment execution component of the main hand control device to output an attitude adjustment resistance equivalent to a resistance received during the attitude adjustment process of the puncture needle based on the attitude adjustment force feedback information. In this manner, when the operator operates the main hand control device 100 to perform a puncture operation, the operator can perceive the resistance received by the puncture needle 211 through the attitude adjustment resistance fed back by the attitude adjustment execution component 120, truly simulating the situation of holding the needle for attitude adjustment, to more precisely control the puncture device 200, thereby improving puncture accuracy. For more descriptions of attitude adjustment force feedback, reference may be made to FIG. 20 and its related descriptions.

In some embodiments, as shown in FIG. 21A and FIG. 23, the attitude control module 220 is provided with a guiding structure 221, and the puncture needle 211 passes through the guiding structure 221. The guiding structure 221 is configured to provide guiding for the puncture needle 211 along its own axial direction.

The guiding structure 221 is a structure capable of providing guiding for the puncture needle 211. In some embodiments, the guiding structure 221 may include a housing fixedly connected to the first passive ring 2231, and guiding balls (not shown in the figures) disposed in the housing. The housing is provided with an annular guiding groove (not shown in the figures). The number of the guiding balls is at least two. A plurality of the guiding balls are clamped in the annular guiding groove along a circumferential direction of the annular guiding groove, and are in contact with the puncture needle 211. When the puncture needle 211 moves along its own axial direction, the plurality of guiding balls can cluster the puncture needle 211 in the annular guiding groove to generate an axial movement, thereby providing guiding for the puncture needle 211 along its own axial direction. When the puncture needle 211 rotates about its own axial direction, the plurality of guiding balls can generate a rotary movement in the annular guiding groove, thereby providing guiding for the puncture needle 211 about its own axial direction. It should be noted that the guiding structure 221 may also be any other feasible structure, as long as it can provide guiding for the puncture needle 211 along its own axial direction. For example, the guiding structure 221 may also be a hole-shaped structure with an elastic inner wall, or the like.

In some embodiments of the present disclosure, by providing the guiding structure 221 to provide guiding for the puncture needle 211 along its own axial direction, it may enable the puncture needle 211 to be smoother when performing an advance operation or a retreat operation, which not only may improve puncture stability, but also is beneficial to improve puncture efficiency and puncture experience.

The beneficial effects provided by the puncture device in the present disclosure may include but are not limited to the following: (1) The puncture device, based on the puncture needle drive module and the attitude adjustment execution component, is capable of stably performing puncture operations and attitude adjustment operations controlled by the main hand control device. (2) The puncture needle can perform advance or retreat operations while rotating, thereby may realize active skin puncture in a master-slave manner, avoid causing accidental injury to patients, and improve puncture efficiency. (3) The rotary drive mechanism of the puncture device, by adopting the rotary drive motor, the fourth angle sensor, and the fourth brake, may realize rotation control of the puncture needle disposed on the puncture device by the main hand control device, and by providing the fourth angle sensor and the fourth brake, it can effectively ensure the consistency of the rotation angle and improve control accuracy. (4) By providing a first force sensor and/or a second force sensor on the puncture device, it can directly detect an interaction force between the puncture needle and human tissue during a puncture process and/or an adjustment process (puncture force feedback information and/or attitude adjustment force feedback information), thereby is beneficial for the main hand control device to simulate an actual puncture process/attitude adjustment process and improve a success rate of a puncture operation. (5) The attitude control module of the puncture device adopts a parallel configuration and has configuration consistency with the attitude adjustment execution component of the main hand control device, which makes motion transmission and motion control simple and easy to realize. (6) By providing a linear scale system (the second position detection module), the puncture device can detect and feedback the puncture depth of the puncture needle in real time, which is beneficial to improve master-slave puncture accuracy.

In some embodiments, the puncture device 200 further includes at least one of an instrument verification control member and a manual switching control.

The instrument verification control member is configured to control the puncture device after clamping the puncture needle to enter an attitude adjustment state or a puncture state. For example, the instrument verification control member may be a control button, a control lever, or the like. After the puncture needle is clamped by the puncture device, triggering the instrument verification control member, the next step of puncture operation or attitude adjustment operation may be performed. For example, when the instrument verification control member is triggered, the selection of a mode may be performed through the mode selection member described above, to confirm that the main hand control device enters a puncture mode or an attitude adjustment mode.

The manual switching control is configured to control the puncture needle to be manually released from the puncture device. That is to say, after the manual switching control is triggered, the puncture needle may be manually released from the puncture device. For example, the manual switching control may be a manual switching button, a manual switching lever, or the like. In certain emergency situations (such as power failure situations), by providing the manual switching control, it may enable the puncture needle to be manually released and removed, to avoid causing further damage to patients.

Due to the puncture device 200 generally being used in an aperture of a medical imaging device, the puncture device 200 may be exposed to a large dose of radiation. Therefore, mechanisms or components on the puncture device 200 (such as an angle sensor, a brake, or a force sensor, or the like) are all provided with a lead plate shielding for shielding radiation. Meanwhile, to prevent artifacts generated by a structure of the puncture device 200 itself from blocking a lesion region, a material of the puncture device 200 is often made of materials such as plastic or ceramic, or the like, that generate fewer artifacts. Moreover, when the puncture device 200 performs a puncture operation, the puncture needle drive module 210 will typically deflect at a certain angle relative to the attitude control module 220 along an axial direction thereof, to cause the puncture needle drive module 210 to deviate from the lesion region, thereby preventing artifacts from overlapping with a medical image of the lesion region.

Some embodiments of the present disclosure also provide a robot. The robot includes the main hand control device 100 as described in any one of the foregoing technical solutions and the puncture device 200 as described in any one of the foregoing technical solutions. The puncture needle drive module 210 of the puncture device 200 drives the puncture needle 211 to move in response to a puncture execution signal from the puncture needle execution component 110 of the main hand control device 100. The puncture execution signal refers to a control signal triggered by an operator controlling movement of the puncture needle execution component 110. The control signals are used to control puncture movement of the puncture needle 211.

In some embodiments, with reference to FIGs. 1A-20, the main hand control device 100 comprises a puncture needle execution component 110 and an attitude adjustment execution component 120. The puncture needle execution component 110 includes a rod structure 111. The attitude adjustment execution component 120 is configured to obtain an attitude of the rod structure 111. The rod structure 111 is capable of moving along its own axial direction.

The puncture needle execution component 110 is a main structure of the main hand control device 100 for controlling the puncture device 200 to perform an advance operation or a retreat operation. The advance operation refers to related operations of the puncture needle 211 disposed on the puncture device 200 for puncturing into a patient's body. The retreat operation refers to related operations of the puncture needle 211 disposed on the puncture device 200 for retreating from the patient's body. In some embodiments, the main hand control device 100 can communicate with/be connected to a processor of the robot (not shown in the figure). When the puncture needle execution component 110 moves, movement of the puncture needle execution component 110 can be fed back to the processor in real time, and then the processor can control the puncture device 200 to drive the puncture needle 211 to perform a puncture operation based on the movement of the puncture needle execution component 110.

The attitude adjustment execution component 120 is a main structure of the main hand control device 100 for adjusting an attitude of the puncture needle 211. Obtaining the attitude of the rod structure 111 refers to obtaining rotation angle information of the axial direction of the rod structure 111 relative to the attitude adjustment execution component 120. Merely by way of example, when it is necessary to adjust the attitude of the puncture needle 211, the operator can control the rod structure 111 to swing relative to the attitude adjustment execution component 120. The attitude adjustment execution component 120 can detect the rotation angle information of the rod structure 111 relative to the attitude adjustment execution component 120 and feed back the rotation angle information to the processor of the robot. The processor can adjust the attitude of the puncture needle 211 based on the rotation angle information of the rod structure 111 relative to the attitude adjustment execution component 120, to achieve the purpose of adjusting the attitude of the puncture needle 211, so that the puncture needle 211 can align with a target puncture target and ensure the accuracy of the puncture operation.

The rod structure 111 is a structure of the main hand control device 100 for the operator to hold and operate. In some embodiments, the rod structure 111 may be provided for the operator to move along the axial direction of the rod structure 111 when performing an advance operation or a retreat operation, to achieve control over a linear motion of the puncture needle 211, and for the operator to swing relative to the attitude adjustment execution component 120 when performing attitude adjustment, thereby achieving control over the attitude of the puncture needle 211. In some embodiments, the rod structure 111 can perform a linear motion. After the linear motion is fed back to the processor of the robot, the processor can control the puncture needle 211 to perform an advance operation based on a distance of the linear motion of the rod structure 111, so that the puncture needle 211 can smoothly puncture into the target puncture target. After a puncture surgery is completed, the main hand control device 100 performs reverse movement to the advance operation to enable the puncture needle 211 to exit the patient's body. The principle thereof is substantially the same as that of the advance operation. In some embodiments, the axial direction of the rod structure 111 can be represented by an arrow X in FIG. 1B. When the rod structure 111 moves downward along the direction of the arrow X, the puncture needle 211 performs an advance operation. When the rod structure 111 moves upward along the direction of the arrow X, the puncture needle 211 performs a retreat operation. In some cases, since the rod structure 111 is closer to the real structure of the puncture needle 211, it allows the operator, when holding the rod structure 111 and moving it along its axial direction, to better simulate linear motion generated during a puncture surgery when holding the puncture needle 211, thereby improving the puncture accuracy and the puncture efficiency. For further descriptions of the main hand control device 100 and the puncture needle execution component 110, reference may be made to FIGs. 1A-20 and related descriptions. For the puncture device 200 and the puncture needle drive module 210, reference may be made to FIGs. 21A-24 and related descriptions.

In some embodiments, with reference to FIGs. 21A-23, the puncture device 200 comprises a puncture needle drive module 210 and an attitude control module 220. The puncture needle drive module 210 is a main structure of the puncture device 200 for driving the puncture needle 211 to perform an advance operation or a retreat operation. In some embodiments, the puncture needle drive module 210 can perform corresponding movement based on real-time movement of the puncture needle execution component 110 (such as movement of the rod structure 111 along its own axial direction), to perform an advance operation or a retreat operation. In some embodiments, the puncture needle drive module 210 includes an advance-retreat drive mechanism 213. The advance-retreat drive mechanism 213 is configured to drive the puncture needle 211 to move relative to the attitude control module 220 along an axial direction of the puncture needle 211.

The advance-retreat drive mechanism 213 is a main structure for driving the puncture needle 211 to perform an advance operation or a retreat operation. In some embodiments, with reference to FIGs. 21A-23, the advance-retreat drive mechanism 213 includes an advance-retreat drive motor 2131, a fifth angle sensor 2132, and a fifth brake 2133. The advance-retreat drive motor 2131 is configured to drive the puncture needle 211 to move along its own axial direction. The fifth angle sensor 2132 is configured to detect a rotation angle of an output shaft of the advance-retreat drive motor 2131, to realize rotation angle detection of the output shaft of the advance-retreat drive motor 2131 and closed-loop control thereof. The fifth brake 2133 is arranged between the puncture needle 211 and the advance-retreat drive motor 2131. The fifth brake 2133 is configured to restrict the puncture needle 211 from moving along its own axial direction, to ensure that the rotation angle of the output shaft of the advance-retreat drive motor 2131 is always consistent with the rotation angle of the output shaft of the force feedback motor 1311.

In some embodiments, as shown in FIG. 17, the advance-retreat drive mechanism 213 further includes a second position detection module 2139. The second position detection module 2139 is configured to acquire a position of the puncture needle 211 along its own axial direction. The second position detection module 2139 includes a second linear scale 21391 and a second linear scale reading member 21392. The second linear scale 21391 is arranged on the linear rail 2134 along an axial direction of the linear rail 2134, and the second linear scale reading member 21392 is arranged on the slider 2135.

The attitude control module 220 is a main structure of the puncture device 200 for controlling an attitude of the puncture needle 211. In some embodiments, the attitude control module 220 can control an attitude adjustment drive module 223 to perform corresponding rotation based on the rotation angle information of the rod structure 111 relative to the attitude adjustment execution component 120 (such as a rotation angle of the first connecting rod 1211 relative to a base 1241, a rotation angle of the second connecting rod 1221 relative to a base 1241, or the like), to achieve the purpose of controlling the attitude of the puncture needle 211, so that the puncture needle 211 can align with the target puncture target and ensure the accuracy of the puncture operation. For more details about the puncture device 200, reference may be made to the foregoing corresponding descriptions.

In some embodiments, a robot further includes a processor (not shown), and the processor is configured to process data related to the main hand control device 100 and the puncture device 200. Merely by way of example, the processor can, in response to a puncture execution signal from the puncture needle execution component 110 of the main hand control device 100, control the puncture needle drive module 210 of the puncture device 200 to drive the puncture needle 211 to move. In some embodiments, the processor may be a single server or a server group. The server group may be centralized or distributed. In some embodiments, the processor may be local or remote. In some embodiments, the processor may be implemented on a cloud platform. Merely by way of example, the cloud platform may include private clouds, public clouds, hybrid clouds, community clouds, distributed clouds, internal clouds, multi-layer clouds, or the like, or any combination thereof.

In some embodiments, the puncture needle execution component 110 includes a first position detection module 112, and the first position detection module 112 is configured to acquire a position of the rod structure 111 in its own axial direction. The puncture execution signal includes the position of the rod structure 111 in its own axial direction. The advance-retreat drive motor 2131 is configured to drive the puncture needle 211 to move a corresponding distance along its own axial direction based on the position of the rod structure 111 in its own axial direction.

In some embodiments, the position of the rod structure 111 in its own axial direction acquired by the first position detection module 112 may reflect a distance of movement of the rod structure 111 along its own axial direction (that is, a distance of linear movement of the rod structure 111). In some embodiments, the corresponding distance of movement of the puncture needle 211 along its own axial direction may be understood as that a corresponding relationship exists between a distance of movement of the puncture needle 211 along its own axial direction (that is, a distance of linear movement of the puncture needle 211) and the distance of movement of the rod structure 111 along its own axial direction, including a mapping relationship or a proportional mapping relationship. For example, the distance of movement of the puncture needle 211 along its own axial direction and the distance of movement of the rod structure 111 along its own axial direction may be a 1:1 mapping, that is, the distance of movement of the puncture needle 211 along its own axial direction is consistent with the distance of movement of the rod structure 111 along its own axial direction. For example, a distance difference always exists between the distance of movement of the puncture needle 211 along its own axial direction and the distance of movement of the rod structure 111 along its own axial direction, such as a distance difference of 0.5 mm, 1 mm, or the like. For example, a distance ratio always exists between the distance of movement of the puncture needle 211 along its own axial direction and the distance of movement of the rod structure 111 along its own axial direction, such as a distance ratio of 1:1.2, 1:1.5, 1:2, 2:1, 1.5:1, or the like. In some embodiments, the distance of movement of the puncture needle 211 along its own axial direction and the distance of movement of the rod structure 111 along its own axial direction may be a 1:1 mapping, so that when an operator operates the rod structure 111 to output a preset distance, the operator can control the puncture needle 211 to move the preset distance, thereby enabling the operator to experience a clinical puncture sensation as much as possible, improving the operation experience of the operator, and improving the success rate of puncture operations. Regarding the first position detection module 112, reference may be made to the relevant description of FIGs. 4-5. Regarding the advance-retreat drive mechanism 213, reference may be made to the relevant description of FIGs. 21A-24.

In some embodiments, the rod structure 111 is capable of rotating about its own axial direction. In some embodiments, the puncture needle drive module 210 may include a rotary drive mechanism 212, the rotary drive mechanism 212 is configured to be connected to the puncture needle 211, and the rotary drive mechanism 212 is in transmission connection with the advance-retreat drive mechanism 213. In some embodiments, the rotary drive mechanism 212 and the advance-retreat drive mechanism 213 are arranged on the attitude control module 220.

The rotary drive mechanism 212 is a main structure configured to drive the puncture needle 211 to rotate about its own axial direction. In some embodiments, as shown in conjunction with FIGs. 21A-23, the rotary drive mechanism 212 includes a rotary drive motor 2121, a fourth angle sensor 2122, and a fourth brake 2123. The rotary drive motor 2121 is configured to drive the puncture needle 211 to rotate about its own axial direction. The fourth angle sensor 2122 is configured to detect a rotation angle of an output shaft of the rotary drive motor 2121, so as to realize rotation angle detection of the puncture needle 211 and its closed-loop control. The fourth brake 2123 is arranged between the puncture needle 211 and the rotary drive motor 2121, and the fourth brake 2123 is configured to restrict the puncture needle 211 from rotating about its own axial direction, to ensure that the rotation angle of the puncture needle 211 is always consistent with the rotation angle of the rod structure 111 rotating about its own axial direction.

In some embodiments, the puncture needle execution component 110 includes an angle detection component 114, and the angle detection component 114 is configured to obtain a rotation angle of the rod structure 111 rotating about its own axial direction. The puncture execution signal includes the rotation angle. The rotary drive mechanism 212 includes the rotary drive motor 2121, and the rotary drive motor 2121 is configured to drive the puncture needle 211 to rotate a corresponding angle about its own axial direction based on the rotation angle.

The corresponding angle of rotation of the puncture needle 211 about its own axial direction may be understood as that a corresponding relationship exists between a rotation angle of the puncture needle 211 rotating about its own axial direction and the rotation angle of the rod structure 111 rotating about its own axial direction, including a mapping relationship or a proportional mapping relationship. For example, the rotation angle of the puncture needle 211 rotating about its own axial direction and the rotation angle of the rod structure 111 rotating about its own axial direction may be a 1:1 mapping, that is, the rotation angle of the puncture needle 211 rotating about its own axial direction is consistent with the rotation angle of the rod structure 111 rotating about its own axial direction. For example, an angle difference always exists between the rotation angle of the puncture needle 211 rotating about its own axial direction and the rotation angle of the rod structure 111 rotating about its own axial direction, such as an angle difference of 5°, 10°, or the like. For example, an angle ratio always exists between the rotation angle of the puncture needle 211 rotating about its own axial direction and the rotation angle of the rod structure 111 rotating about its own axial direction, such as an angle ratio of 1:1.5, 1:2, 2:1, or the like. In some embodiments, the rotation angle of the puncture needle 211 rotating about its own axial direction and the rotation angle of the rod structure 111 rotating about its own axial direction may be a 1:1 mapping, so that when the operator operates the rod structure 111 to rotate a preset angle about its own axial direction, the operator can control the puncture needle 211 to rotate the preset angle about its own axial direction, thereby improving puncture accuracy and the success rate of puncture operations.

In some embodiments of the present disclosure, the puncture needle execution component 110 of the main hand control device 100 and the puncture needle drive module 210 of the puncture device 200 are both provided with corresponding detection components or instruments (such as an angle detection component and a position detection module), which are beneficial for realizing closed-loop control of puncture operations under a master-slave mode, to make the master-slave puncture motion control more precise, thereby improving puncture accuracy and the success rate of puncture operations.

Regarding the angle detection component, reference may be made to FIGs. 9-10 and the relevant description thereof. Regarding the position detection module, reference may be made to FIGs. 4-5 and FIG. 22 and the relevant description thereof.

In some embodiments, the puncture device 200 further includes a first force sensor 2124, and the first force sensor 2124 is arranged between the puncture needle drive module 210 and the puncture needle 211. The first force sensor 2124 is configured to acquire puncture force feedback information during puncture of the puncture needle 211. The main hand control device 100 further includes a force feedback component 130, and the force feedback component 130 is configured to apply a puncture motion resistance along the axial direction of the rod structure 111 to the rod structure 111. The puncture motion resistance is determined based on the puncture force feedback information.

Regarding the first force sensor 2124, reference may be made to the relevant description of FIGs. 21A-24. Regarding the force feedback component 130, reference may be made to the relevant description of FIGs. 6-7.

In some embodiments, the attitude control module (220) includes an attitude adjustment base (222) and an attitude adjustment drive module (223), wherein the attitude adjustment drive module (223) is arranged on the attitude adjustment base (222), and the puncture needle drive module (210) is mounted to the attitude adjustment base (222) through the attitude adjustment drive module (223). The attitude adjustment drive module (223) adjusts an attitude of the puncture needle (211) relative to the attitude adjustment base (222) in response to an attitude adjustment signal from the attitude adjustment execution component (120) of the main hand control device (100).

Reference may be made to FIGs. 21A-24 and related descriptions for the attitude adjustment base (222) and the attitude adjustment drive module (223).

In some embodiments, the attitude adjustment execution component (120) includes a first connecting rod (1211), a first rotating ring (1212), a second connecting rod (1221), a second rotating ring (1222), a second angle sensor (1213), a third angle sensor (1223), and a base (123). The first rotating ring (1212) and the second rotating ring (1222) are coaxial and arranged surrounding the rod structure (111). One end of the first connecting rod (1211) is rotatably connected to the first rotating ring (1212), another end of the first connecting rod (1211) is rotatably connected to the base (123), and the second angle sensor (1213) is further disposed at the another end of the first connecting rod (1211). One end of the second connecting rod (1221) is rotatably connected to the second rotating ring (1222), another end of the second connecting rod (1221) is rotatably connected to the base (123), and the third angle sensor (1223) is further disposed at the another end of the second connecting rod (1221). The second angle sensor (1213) is configured to detect an angle of rotation of the another end of the first connecting rod (1211) relative to the base (123), and the third angle sensor (1223) is configured to detect an angle of rotation of the another end of the second connecting rod (1221) relative to the base (123). The attitude adjustment signal includes the angle of the another end of the first connecting rod (1211) rotating relative to the base (123) and the angle of the another end of the second connecting rod (1221) rotating relative to the base (123).

In some embodiments, the attitude adjustment drive module (223) includes a first passive ring (2231), a second passive ring (2232), a first coupling rod (2233), a second coupling rod (2234), a first attitude adjustment drive mechanism (2235), and a second attitude adjustment drive mechanism (2236), wherein the first passive ring (2231) and the second passive ring (2232) are coaxial and rotatably connected to the attitude adjustment base (222). The puncture needle (211) passes through the first passive ring (2231) and the second passive ring (2232) along an axial direction of the first passive ring (2231). The first passive ring (2231) is rotatably connected to one end of the first coupling rod (2233), the other end of the first coupling rod (2233) is rotatably connected to the attitude adjustment base (222), and the first attitude adjustment drive mechanism (2235) is further arranged at the other end of the first coupling rod (2233). The second passive ring (2232) is rotatably connected to one end of the second coupling rod (2234), the other end of the second coupling rod (2234) is rotatably connected to the attitude adjustment base (222), and the second attitude adjustment drive mechanism (2236) is further arranged at the other end of the second coupling rod (2234).

In some embodiments, the attitude adjustment drive module (223) adjusting the attitude of the puncture needle (211) relative to the attitude adjustment base (222) in response to the attitude adjustment signal from the attitude adjustment execution component (120) of the main hand control device (100) includes: the first attitude adjustment drive mechanism (2235) driving the first coupling rod (2233) to rotate a corresponding angle relative to the attitude adjustment base (222) based on the angle of the another end of the first connecting rod (1211) rotating relative to the base (123); and the second attitude adjustment drive mechanism (2236) driving the second coupling rod (2234) to rotate a corresponding angle relative to the attitude adjustment base (222) based on the angle of the another end of the second connecting rod (1221) rotating relative to the base (123). For more details on how the attitude adjustment drive module (223) adjusts the puncture attitude of the puncture needle (211) based on the attitude adjustment signal, reference may be made to FIGs. 21A-24 and their related descriptions in the foregoing.

In some embodiments, the puncture device (200) further includes a second force sensor, wherein the second force sensor is arranged between the puncture needle (211) and the attitude adjustment drive module (223). The second force sensor is configured to acquire attitude adjustment force feedback information during attitude adjustment of the puncture needle (211). The attitude adjustment drive module (223) is configured to apply an attitude adjustment resistance torque to the rod structure (111). The attitude adjustment resistance torque is determined based on attitude adjustment force feedback information.

In some embodiments, the attitude adjustment execution component (120) includes a first motor and a second motor (1228). The first motor is configured to apply an attitude adjustment resistance to the first connecting rod (1211), and the second motor (1228) is configured to apply an attitude adjustment resistance to the second connecting rod (1221). The attitude adjustment resistance applied by the first motor to the first connecting rod (1211) and the attitude adjustment resistance applied by the second motor (1228) to the second connecting rod (1221) are both determined based on attitude adjustment force feedback information.

Reference may be made to FIG. 20 and related descriptions for the first motor and the second motor.

In some embodiments of the present disclosure, the attitude adjustment execution component (120) of the main hand control device (100) and the attitude control module (220) of the puncture device (200) adopt a similar structure and configuration (such as a parallel configuration), and have the same degrees of freedom, which is beneficial for realizing master-slave attitude adjustment motion control. Furthermore, both the attitude adjustment execution component (120) and the attitude control module (220) are provided with corresponding angle sensors, which is beneficial for realizing closed-loop control of attitude adjustment operations in a master-slave mode, so as to make the master-slave attitude adjustment motion control more precise, thereby improving puncture accuracy and the success rate of puncture surgery.

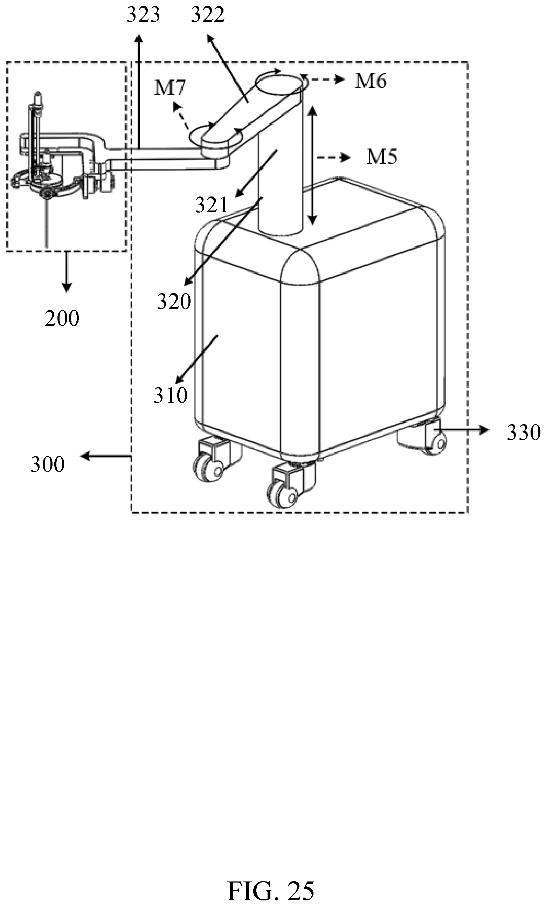

In some embodiments, as shown in FIG. 25, the robot further includes a mobile device (300). The mobile device (300) is connected to the puncture device (200), and the mobile device (300) drives the puncture device (200) to move in a plurality of degrees of freedom.

The mobile device (300) is a device configured to support the puncture device (200) and capable of adjusting an overall position of the puncture device (200). In some embodiments, the mobile device (300) may be a mobile cart. The mobile cart includes a cart body (310), a transmission connection component (320), and a plurality of casters (330). The plurality of casters (330) are respectively disposed at corners of a lower end face of the cart body (310), and the transmission connection component (320) is disposed on an upper end face of the cart body (310). The casters (330) are configured to drive the puncture device (200) to perform overall movement, so as to realize an adjustment of an overall position of the puncture device (200). The transmission connection component (320) is configured to connect to the puncture device (200) and provide movement in a plurality of degrees of freedom for the puncture device (200).

In some embodiments, as shown in FIG. 25, the transmission connection component (320) may include a telescopic portion (321), a first connection portion (322), and a second connection portion (323). The telescopic portion (321) is arranged along a vertical direction (parallel to an arrow X direction in the figure). The first connection portion (322) and the second connection portion (323) are both arranged along a horizontal direction (perpendicular to the arrow X direction in the figure). One end of the telescopic portion (321) is fixedly connected to the cart body (310), the other end of the telescopic portion (321) is rotatably connected to one end of the first connection portion (322), the other end of the first connection portion (322) is rotatably connected to one end of the second connection portion (323), and the other end of the second connection portion (323) is fixedly connected to the puncture device (200). In some embodiments, the telescopic portion 321 may include a cylinder, a piston, or the like. A transmission bearing is disposed between the first connection portion 322 and the telescopic portion 321, and between the first connection portion 322 and the second connection portion 323. In some embodiments, the transmission connection component 320 may also be a robotic arm, or any other feasible structure, or the like.

In some embodiments, the transmission connection component 320 is capable of achieving three degrees of freedom. Merely by way of example, the telescopic portion 321 may move along the arrow X direction in the figure, that is, having a fifth degree of freedom (as shown by arrow M5); the first connection portion 322 and the telescopic portion 321 are capable of generating a rotation therebetween, that is, having a sixth degree of freedom (as shown by arrow M6); and the first connection portion 322 and the second connection portion 323 are capable of generating a rotation therebetween, that is, having a seventh degree of freedom (as shown by arrow M7). It can be understood that, based on the fifth degree of freedom, movement of the puncture device 200 in a vertical direction can be achieved; and based on the sixth degree of freedom and the seventh degree of freedom, movement of the puncture device 200 in a horizontal direction can be achieved.

It should be noted that the mobile device 300 may be any other feasible structural form, as long as it is capable of achieving an overall position adjustment requirement of the puncture device 200. Merely by way of example, the mobile device 300 may also employ a bedside robotic arm, or robotic arms of other configurations, or the like.

In some embodiments, the mobile device 300 may also include a third signal transmission component (not shown in the figure), to realize automatic control of the mobile device 300 based on the third signal transmission component communicating with/connecting to the processor. In some embodiments, when an operator controls the main hand control device 100 to perform an attitude adjustment operation, the transmission connection component 320 of the mobile device 300 is capable of generating a follow-up movement, so that the needle tip of the puncture needle 211 remains stationary at a preset position. In some embodiments, for puncture surgery guided by medical images, when the operator controls the main hand control device 100 to perform the attitude adjustment operation, the transmission connection component 320 of the mobile device 300 is capable of generating a follow-up movement, so that the needle tip of the puncture needle 211 remains stationary at the preset position in the medical image.

Some embodiments of the present disclosure, by providing the mobile device, achieve adjustment of an overall position of the puncture device, which may enable the puncture device to be in an optimal position, so as to facilitate the operator to further adjust the puncture needle, thereby improving puncture accuracy.

In some embodiments, the robot further includes a processor (not shown), wherein the processor is configured to process data related to the main hand control device 100 and the puncture device 200. Merely by way of example, the processor is capable of controlling the puncture needle drive module 210 of the puncture device 200 to drive the puncture needle 211 to move in response to a puncture execution signal from the puncture needle execution component 110 of the main hand control device 100. In some embodiments, the processor may be a single server or a server group. The server group may be centralized or distributed. In some embodiments, the processor may be local or remote. In some embodiments, the processor may be implemented on a cloud platform. Merely by way of example, the cloud platform may include private clouds, public clouds, hybrid clouds, community clouds, distributed clouds, internal clouds, multi-layer clouds, or the like, or any combination thereof.

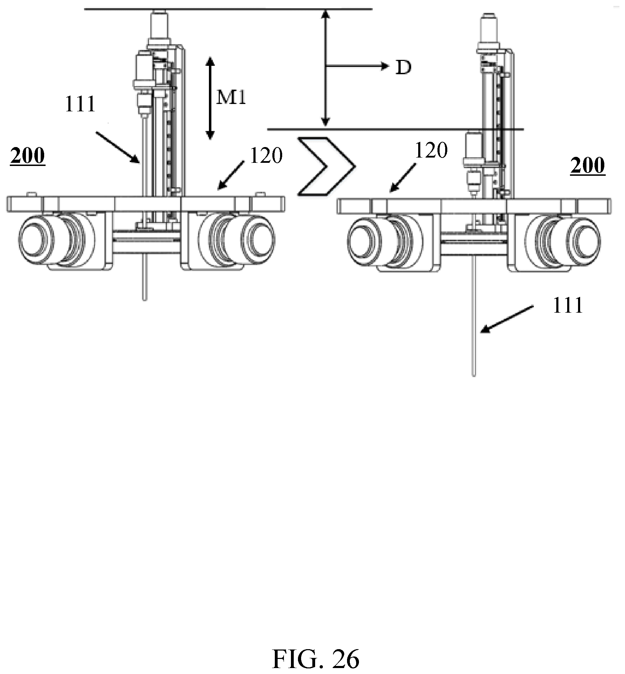

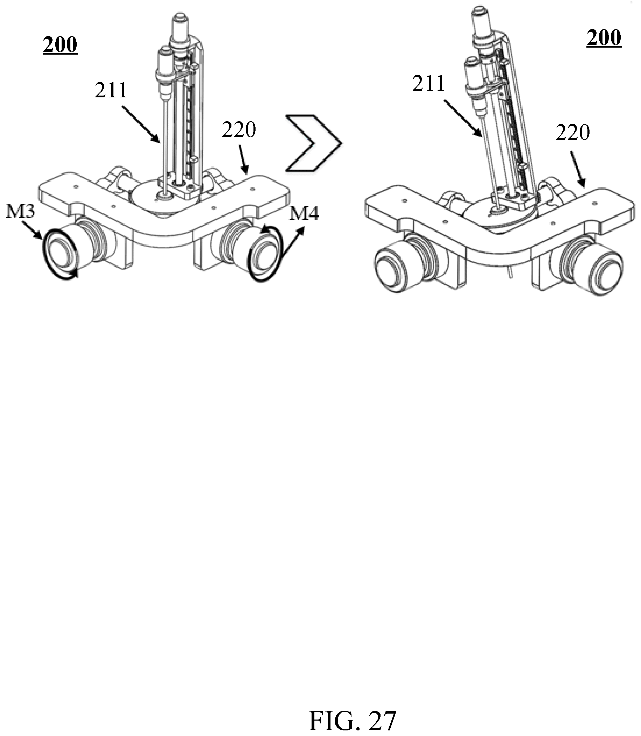

To more clearly illustrate a working principle of the robot, FIG. 11 is a schematic diagram illustrating a working principle of the main hand control device 100. As shown in conjunction with FIG. 1B and FIG. 11, the main hand control device 100 provided in some embodiments of the present disclosure is an attitude increment mapping-type four-degree-of-freedom force feedback master operating device. That is, movement of the operator on the main hand control device 100 will be mapped to the puncture needle 211 disposed on the puncture device 200, thereby controlling the puncture needle 211. The rod structure 111 is capable of moving relative to the attitude adjustment execution component 120 along its own axial direction (moving along the arrow X direction in FIG. 1B), that is, having a first degree of freedom (as shown by arrow M1). A movement distance of the rod structure 111 along the first degree of freedom is D, and the puncture needle drive module 210 drives the puncture needle 211 to move a corresponding distance D along its own axial direction. For example, in the embodiment shown in FIG. 12, the sequence from left to right is that the rod structure 111 performs puncture relative to the attitude adjustment execution component 120 along the first degree of freedom, wherein a puncture depth is D. In the embodiment shown in FIG. 26, the sequence from left to right is that the puncture needle 211 performs puncture relative to the attitude control module 220 along the first degree of freedom, wherein a puncture depth is D. The rod structure 111 is capable of rotating about its own axial direction through a passive degree-of-freedom mounting seat (such as the passive degree-of-freedom mounting seat 1261 in FIG. 5), that is, having a second degree of freedom (as shown by arrow M2 in FIG. 15). The rod structure 111 is capable of rotating relative to the attitude adjustment execution component 120 within a plane where a first rotation axis O1 and a fourth rotation axis O4 are located (e.g., a vertical plane), that is, having a third degree of freedom (as shown by arrow M3). The rod structure 111 is capable of swinging relative to the attitude adjustment execution component 120 within a plane where a second rotation axis O2 and a third rotation axis O3 are located (e.g., a vertical plane), that is, having a fourth degree of freedom (as shown by arrow M4). For example, in the embodiment shown in FIG. 14, the sequence from left to right is that the rod structure 111 performs swinging relative to the attitude adjustment execution component 120 along the fourth degree of freedom. In the embodiment shown in FIG. 27, the sequence from left to right is that the puncture needle 211 performs swinging relative to the attitude control module 220 along the fourth degree of freedom.

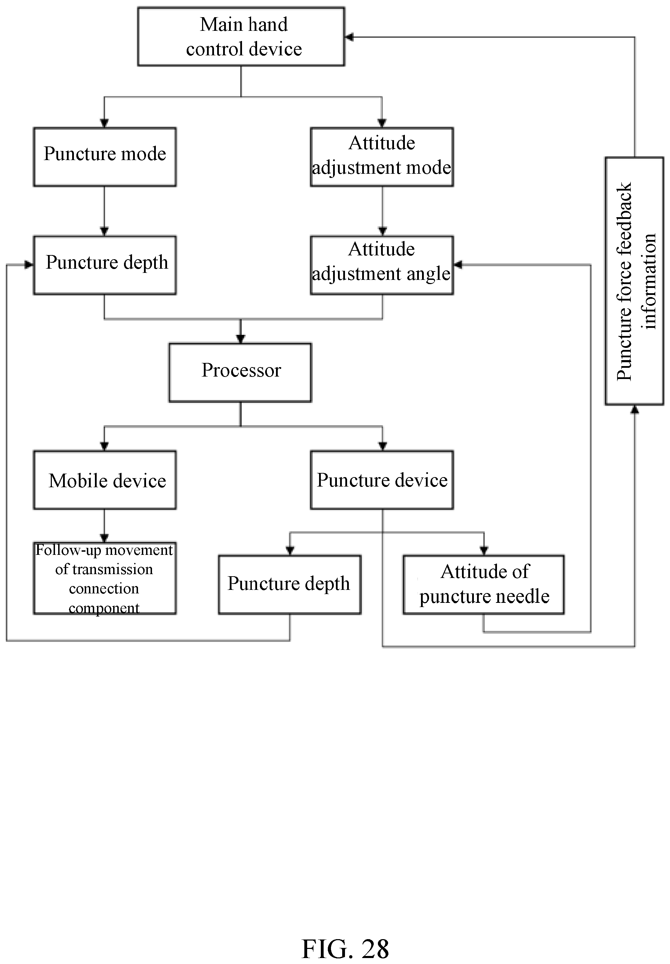

To more clearly illustrate a working flow of the robot, FIG. 28 is a flowchart illustrating the working flow of the robot. As shown in FIG. 28, when the operator controls the main hand control device 100 to perform a puncture operation or an attitude adjustment operation (respectively corresponding to the puncture mode and the attitude adjustment mode), corresponding motion information will be respectively generated, such as motion distance information of movement of the rod structure 111 relative to the attitude adjustment execution component 120 along its own axial direction (i.e., puncture depth) and rotation angle information of the rod structure 111 relative to the attitude adjustment execution component 120 (i.e., attitude adjustment angle). The puncture depth and the attitude adjustment angle may be respectively obtained based on the first position detection module 112 and an attitude adjustment angle sensor (such as the second angle sensor 1213 and the third angle sensor 1223), and transmitted to the processor through a first signal transmission component, wherein the processor controls the puncture device 200 to perform corresponding operations based on the corresponding motion information. When the main hand control device 100 is in the puncture mode, the transmission connection component 320 of the mobile device 300 remains stationary, and the puncture needle drive module 210 of the puncture device 200 (such as the rotary drive mechanism 212 and the advance-retreat drive mechanism 213) drives the puncture needle 211 to perform corresponding puncture operations. Furthermore, the second position detection module 2139 is capable of detecting a position of the puncture needle 211 along its axial direction in real time, to make a puncture depth of the puncture needle 211 consistent with the movement distance of the rod structure 111 along its own axial direction. When the main hand control device 100 is in the attitude adjustment mode, the transmission connection component 320 of the mobile device 300 generates a follow-up movement to make the needle tip of the puncture needle 211 remain stationary at the preset position in the medical image. The attitude control module 220 of the puncture device 200 (such as the first attitude adjustment drive mechanism 2235 and the second attitude adjustment drive mechanism 2236) drives the first coupling rod 2233 and the second coupling rod 2234 to perform corresponding rotation to achieve attitude adjustment of the puncture needle 211. Moreover, the sixth angle sensor and the seventh angle sensor 22364 are capable of detecting rotation angles of the first coupling rod 2233 and the second coupling rod 2234 in real time, to make the attitude adjustment angle of the puncture needle 211 consistent with the rotation angle of the rod structure 111 relative to the attitude adjustment execution component 120. In addition, when the puncture device 200 is performing a puncture operation, the first force sensor 2124 disposed on the puncture needle 211 may acquire puncture force feedback information, and transmit it back to the force feedback component 130 through a signal transmission component (such as the first signal transmission component and a second signal transmission component). The force feedback component 130 then outputs a resistance corresponding to puncture resistance to the rod structure 111 through the resistance transmission member 113, to achieve a master-slave puncture force feedback function.

The advantageous effects of the robot provided in the present disclosure may include, but are not limited to: (1) the puncture needle execution component of the main hand control device of the robot and the puncture needle drive module of the puncture device are both provided with corresponding detection components or instruments (such as an angle detection component and a position detection module), which are beneficial to achieve closed-loop control of puncture operations in a master-slave mode, so as to make the master-slave puncture motion control more accurate, thereby improving puncture accuracy and puncture success rate; (2) the attitude adjustment execution component of the main hand control device of the robot and the attitude control module of the puncture device employ similar structures and configurations (e.g., some structures are parallel configurations), and have the same degrees of freedom, which are beneficial to achieve master-slave attitude adjustment motion control. In addition, the attitude adjustment execution component and the attitude control module are both provided with corresponding angle sensors, which are beneficial to achieve closed-loop control of attitude adjustment operations in the master-slave mode, so as to make the master-slave attitude adjustment motion control more accurate, thereby improving puncture accuracy and the success rate of puncture surgery.

Basic concepts have been described above. Obviously, for those skilled in the art, the detailed disclosure above is merely by way of example, and does not constitute a limitation on the present disclosure. Although not explicitly described herein, those skilled in the art may make various modifications, improvements, and revisions to the present disclosure. Such modifications, improvements, and revisions are suggested in the present disclosure. Therefore, such modifications, improvements, and revisions still fall within the spirit and scope of the exemplary embodiments of the present disclosure.

## Claims

1. A main hand control device (100) for a robot, comprising:
a puncture needle execution component (110), the puncture needle execution component (110) including a rod structure (111); and
an attitude adjustment execution component (120), the attitude adjustment execution component (120) being configured to obtain an attitude of the rod structure (111); wherein the rod structure is capable of moving relative to the attitude adjustment execution component along an axial direction of the rod structure (111).

2. The main hand control device (100) of claim 1, wherein the main hand control device (100) further comprises a force feedback component (130), and the force feedback component (130) is configured to apply a puncture motion resistance along the axial direction of the rod structure (111) to the rod structure (111).

3. The main hand control device (100) of claim 2, wherein the puncture needle execution component (110) further includes a resistance transmission member (113) disposed on the rod structure (111), and the force feedback component (130) applies the puncture motion resistance to the rod structure (111) via the resistance transmission member (113).

4. The main hand control device (100) of claim 3, wherein the force feedback component (130) comprises a force output unit (131) and a force transmission unit (132), wherein the force transmission unit (132) is drivingly connected to the resistance transmission member (113), and the force output unit (131) outputs the puncture motion resistance based on puncture force feedback information during puncturing of a puncture needle (211).

5. The main hand control device (100) of claim 4, wherein the resistance transmission member (113) is fixedly connected to the rod structure (111), the force feedback component (130) further includes a first brake (1324), the first brake (1324) is fixedly connected to the attitude adjustment execution component (120), the first brake (1324) is drivingly connected to the force transmission unit (132), and the first brake (1324) is configured to restrict relative movement of the rod structure (111) along the axial direction of the rod structure (111) by controlling the force transmission unit (132) to be fixed relative to the resistance transmission member (113).

6. The main hand control device (100) of claim 4, wherein the force output unit (131) includes a force feedback motor (1311), the force transmission unit (132) includes a gear (1322) connected to an output end of the force feedback motor (1311), the resistance transmission member (113) includes a rack (1131) meshing with the gear (1322), and the rack (1131) is disposed on the rod structure (111) and an arrangement direction of the rack (1131) is parallel to the axial direction of the rod structure (111).

7. The main hand control device (100) of claim 4, wherein the force output unit (131) includes a force feedback motor (1311), the force transmission unit (132) includes a pulley (1326) connected to an output end of the force feedback motor (1311), the resistance transmission member (113) includes a mounting seat (1132), a first connection rope (1133), and a second connection rope (1134), the mounting seat (1132) is provided with two mounting portions, and the two mounting portions are spaced apart along a direction parallel to the axial direction of the rod structure (111); and
one end of the first connection rope (1133) is connected to one of the mounting portions, the first connection rope (1133) is wound around the pulley (1326), and another end of the first connection rope (1133) is fixed on the pulley (1326); one end of the second connection rope (1134) is connected to the other of the mounting portions, the second connection rope (1134) is wound around the pulley (1326), and another end of the second connection rope (1134) is fixed on the pulley (1326); and the first connection rope (1133) and the second connection rope (1134) are wound on the pulley (1326) in opposite directions.

8. The main hand control device (100) of claim 6 or 7, wherein the force feedback component (130) further includes a first angle sensor (1323), and the first angle sensor (1323) is configured to detect a rotation angle of an output shaft of the force feedback motor (1311).

9. The main hand control device (100) of any one of claims 1-8, wherein the puncture needle execution component (110) further includes a first position detection module (112), and the first position detection module (112) is configured to obtain a position of the rod structure (111) along the axial direction of the rod structure (111).

10. The main hand control device (100) of claim 9, wherein the first position detection module (112) includes a first linear scale (1121) and a first linear scale reading member (1122), the first linear scale (1121) is disposed on the rod structure (111) along the axial direction of the rod structure (111), and the first linear scale reading member (1122) is disposed on the attitude adjustment execution component (120).

11. The main hand control device (100) of any one of claims 1-10, wherein the rod structure (111) is capable of rotating about the axial direction of the rod structure (111).

12. The main hand control device (100) of claim 11, wherein the rotating of the rod structure (111) about the axial direction of the rod structure (111) is configured to control a puncture needle(211) of a puncture device to correspondingly rotate about an axis of the puncture needle (211).

13. The main hand control device (100) of any one of claims 1-10, wherein the main hand control device (100) comprises a rotation control member, and the rotation control member is configured to control a puncture needle of a puncture device to rotate about an axial direction of the puncture needle (211).

14. The main hand control device (100) of claim 12, wherein the puncture needle execution component (110) includes an angle detection component (114), and the angle detection component (114) is configured to obtain a rotation angle of the rod structure (111) rotating about the axial direction of the rod structure (111).

15. The main hand control device (100) of claim 14, wherein the angle detection component (114) includes an encoder (1141) and an encoder reading member (1142); and
the encoder (1141) is disposed on the rod structure (111) and rotates synchronously with the rod structure (111), and the encoder reading member (1142) is disposed on the attitude adjustment execution component (120).

16. The main hand control device (100) of any one of claims 1-15, wherein the rod structure (111) is disposed on the attitude adjustment execution component (120), and the rod structure (111) has a puncture degree of freedom and an attitude adjustment degree of freedom relative to the attitude adjustment execution component (120); and
the puncture degree of freedom is a degree of freedom allowing a position change of the rod structure (111) along the axial direction of the rod structure (111), and the attitude adjustment degree of freedom is a degree of freedom allowing a change in the axial direction of the rod structure (111).

17. The main hand control device (100) of claim 16, wherein the attitude adjustment execution component (120) is configured to apply an attitude adjustment resistance under the attitude adjustment degree of freedom to the rod structure (111) based on attitude adjustment force feedback information during attitude adjustment of the puncture needle (211).

18. The main hand control device (100) of claim 16 or 17, wherein the attitude adjustment execution component (120) includes a first connecting rod (1211), a first rotating ring (1212), a second connecting rod (1221), a second rotating ring (1222), a second angle sensor (1213), a third angle sensor (1223), and a base (123);
the first rotating ring (1212) and the second rotating ring (1222) are coaxial and arranged surrounding the rod structure (111), one end of the first connecting rod (1211) is rotatably connected to the first rotating ring (1212), another end of the first connecting rod (1211) is rotatably connected to the base (123), and the second angle sensor (1213) is further disposed at the another end of the first connecting rod (1211);
one end of the second connecting rod (1221) is rotatably connected to the second rotating ring (1222), another end of the second connecting rod (1221) is rotatably connected to the base (123), and the third angle sensor (1223) is further disposed at the another end of the second connecting rod (1221); and
the second angle sensor (1213) is configured to detect an angle of rotation of the another end of the first connecting rod (1211) relative to the base (123), and the third angle sensor (1223) is configured to detect an angle of rotation of the another end of the second connecting rod (1221) relative to the base (123).

19. The main hand control device (100) of claim 18, wherein the attitude adjustment execution component (120) includes a first motor and a second motor (1228), the first motor is configured to apply a torque to the first connecting rod (1211), and the second motor (1228) is configured to apply a torque to the second connecting rod (1221).

20. The main hand control device (100) of claim 19, wherein the first motor is configured to apply an attitude adjustment resistance torque to the first connecting rod (1211) based on attitude adjustment force feedback information during attitude adjustment of the puncture needle; and the second motor (1228) is configured to apply an attitude adjustment resistance torque to the second connecting rod (1221) based on attitude adjustment force feedback information during attitude adjustment of the puncture needle.

21. The main hand control device (100) of claim 19, wherein the attitude adjustment execution component (120) further includes a first damper and a second damper (125); the first damper is configured to provide a motion resistance hindering rotation of the first connecting rod (1211) relative to the base (123) based on a position of the rod structure (111) along the axial direction of the rod structure (111), and the second damper (125) is configured to provide a motion resistance hindering rotation of the second connecting rod (1221) relative to the base (123) based on the position of the rod structure (111) along the axial direction of the rod structure (111).

22. The main hand control device (100) of claim 21, wherein,
when a stroke of the rod structure (111) along a first direction increases, a force arm of a deflection force applied by an operator on the rod structure (111) decreases;
when a stroke of the rod structure (111) along a second direction increases, the force arm of the deflection force applied by the operator on the rod structure (111) increases; and the first direction is opposite to the second direction;
the motion resistance provided by at least one of the first damper and the second damper (125) decreases as the stroke of the rod structure (111) along the first direction increases; and
the motion resistance provided by at least one of the first damper and the second damper (125) increases as the stroke of the rod structure (111) along the second direction increases.

23. The main hand control device (100) of claim 21, wherein the attitude adjustment execution component (120) further includes a first reducer and a second reducer (1229);
the first motor is connected to the first damper through the first reducer, and the second motor (1228) is connected to the second damper (125) through the second reducer (1229); and
the first motor is configured to output a torque to overcome an output damping of the first damper, the second motor (1228) is configured to output a torque to overcome an output damping of the second damper (125), the first reducer is configured to increase an output torque of the first damper, and the second reducer (1229) is configured to increase an output torque of the second damper (125).

24. The main hand control device (100) of claim 21, wherein the attitude adjustment execution component (120) further includes a second brake and a third brake (1227), an output shaft of the second brake is drivingly connected to the first connecting rod (1211) through the first damper, the second brake is configured to restrict rotation of the first connecting rod (1211) relative to the base (123), an output shaft of the third brake (1227) is drivingly connected to the second connecting rod (1221) through the second damper (125), and the third brake (1227) is configured to restrict rotation of the second connecting rod (1221) relative to the base (123).

25. The main hand control device (100) of claim 18, wherein the attitude adjustment execution component (120) further includes a passive degree-of-freedom connection component (126), the passive degree-of-freedom connection component (126) is rotatably connected to the first rotating ring (1212) and the second rotating ring (1222), and the passive degree-of-freedom connection component (126) is slidably connected to the rod structure (111).

26. The main hand control device (100) of any one of claims 1-25, wherein the main hand control device comprises at least one of a mode selection member, a degree-of-freedom selection member, and a quick switching member;
the mode selection member is configured to select the main hand control device (100) to be in a puncture mode or an attitude adjustment mode; in the puncture mode, the rod structure (111) has a puncture degree of freedom relative to the attitude adjustment execution component (120), and in the attitude adjustment mode, the rod structure (111) has an attitude adjustment degree of freedom relative to the attitude adjustment execution component (120);
the attitude adjustment degree of freedom includes at least one of a first degree of freedom and a second degree of freedom;
the degree-of-freedom selection member controls an attitude adjustment degree of freedom of the puncture needle (211) relative to the puncture device (200) to be at least one of the first degree of freedom and the second degree of freedom; and
the quick switching member is used to control the main hand control device (100) to switch from the attitude adjustment mode to the puncture mode, and/or to control the main hand control device (100) to switch back from the puncture mode to the attitude adjustment mode.

27. The main hand control device (100) of claim 2, wherein the main hand control device comprises a release member, and the release member is configured to control the force feedback component (130) and the attitude adjustment execution component (120) to release an acting force on the rod structure (111).

28. The main hand control device (100) of any one of claims 1-27, wherein the rod structure (111) includes an operation portion (1111) and a sliding portion (1112), the operation portion (1111) is configured to receive user operation, the sliding portion (1112) is configured to move relative to the attitude adjustment execution component (120) along an axial direction of the sliding portion (1112), and the operation portion (1111) is located at an upper end of the rod structure (111).

29. The main hand control device (100) of claim 28, wherein the operation portion (1111) includes a plurality of operation regions (11111) and at least one enable button.

30. The main hand control device (100) of claim 29, wherein the plurality of operation regions (11111) includes a first control region (11112) and at least one second control region (11114), and the first control region (11112) and the at least one second control region (11114) are arranged back-to-back.

31. The main hand control device (100) of claim 30, wherein the at least one enable button includes a first enable button (11113) disposed on the first control region (11112); and/or, the at least one enable button further includes at least one second enable button (11115) disposed on the at least one second control region (11114).

32. The main hand control device (100) of claim 29, wherein the at least one enable button further includes a third enable button (11116) disposed on a top of the operation portion (1111).

33. The main hand control device (100) of claim 28, wherein a first limit structure (1113) and a second limit structure (1114) are respectively disposed at an end of the sliding portion (1112) close to the operation portion (1111) and an end of the sliding portion (1112) away from the operation portion (1111), and the first limit structure (1113) and the second limit structure (1114) are configured to limit a stroke range of movement of the rod structure (111) along the axial direction of the rod structure (111).

34. A puncture device (200) for a robot, comprising:
a puncture needle drive module (210), including an advance-retreat drive mechanism (213), the advance-retreat drive mechanism (213) being connected to a puncture needle (211); and
an attitude control module (220), the advance-retreat drive mechanism (213) being disposed on the attitude control module (220), and the advance-retreat drive mechanism (213) being configured to drive the puncture needle (211) to move relative to the attitude control module (220) along an axial direction of the puncture needle (211).

35. The puncture device (200) of claim 34, wherein the puncture needle drive module (210) includes a rotary drive mechanism (212); the rotary drive mechanism (212) is configured to be connected to a puncture needle (211); and the rotary drive mechanism (212) is in transmission connection with the advance-retreat drive mechanism (213); and
the rotary drive mechanism (212) is arranged on the attitude control module (220); and the rotary drive mechanism (212) is configured to drive the puncture needle (211) to rotate relative to the attitude control module (220) about an axial direction of the puncture needle (211).

36. The puncture device (200) of claim 35, wherein the rotary drive mechanism (212) includes a rotary drive motor (2121), a fourth angle sensor (2122), and a fourth brake (2123); the rotary drive motor (2121) is configured to drive the puncture needle (211) to rotate about an axial direction of the puncture needle (211); the fourth angle sensor (2122) is configured to detect a rotation angle of an output shaft of the rotary drive motor (2121); the fourth brake (2123) is arranged between the puncture needle (211) and the rotary drive motor (2121); and the fourth brake (2123) is configured to restrict the puncture needle (211) from rotating about the axial direction thereof.

37. The puncture device (200) of claim 34, wherein the advance-retreat drive mechanism (213) includes an advance-retreat drive motor (2131), a fifth angle sensor (2132), and a fifth brake (2133); the advance-retreat drive motor (2131) is configured to drive the puncture needle (211) to move along the axial direction of the puncture needle (211); the fifth angle sensor (2132) is configured to detect a rotation angle of an output shaft of the advance-retreat drive motor (2131); the fifth brake (2133) is arranged between the puncture needle (211) and the advance-retreat drive motor (2131); and the fifth brake (2133) is configured to restrict the puncture needle (211) from moving along the axial direction of the puncture needle (211).

38. The puncture device (200) of claim 37, wherein the advance-retreat drive mechanism (213) further includes a linear rail (2134), a slider (2135), and a mounting seat (2136);
the linear rail (2134) is arranged parallel to the puncture needle (211); the slider (2135) is in transmission connection with the advance-retreat drive motor (2131); and the slider (2135) is slidably connected to the linear rail (2134); and
the mounting seat (2136) is fixedly connected to the slider (2135); and the puncture needle (211) is mounted on the mounting seat (2136).

39. The puncture device (200) of claim 38, wherein the advance-retreat drive mechanism (213) further includes a second position detection module (2139); the second position detection module (2139) is configured to acquire a position of the puncture needle (211) in the axial direction of the puncture needle (211); the second position detection module (2139) includes a second linear scale (21391) and a second linear scale reading member (21392); the second linear scale (21391) is arranged on the linear rail (2134) along an axial direction of the linear rail (2134); and the second linear scale reading member (21392) is arranged on the slider (2135).

40. The puncture device (200) of claim 34, wherein the puncture device (200) further comprises a first force sensor (2124); the first force sensor (2124) is arranged between the puncture needle (211) and the puncture needle drive module (210); and the first force sensor (2124) is configured to acquire puncture force feedback information during puncture of the puncture needle (211); and
the puncture force feedback information is used to determine a puncture motion resistance output by a main hand control device (100) for controlling the puncture device (200).

41. The puncture device (200) of claim 34, wherein the attitude control module (220) includes an attitude adjustment base (222) and an attitude adjustment drive module (223); the attitude adjustment drive module (223) is arranged on the attitude adjustment base (222); the puncture needle drive module (210) is mounted to the attitude adjustment base (222) through the attitude adjustment drive module (223); and the attitude adjustment drive module (223) drives the puncture needle (211) to adjust an attitude of the puncture needle (211) relative to the attitude adjustment base (222).

42. The puncture device (200) of claim 41, wherein the attitude adjustment drive module (223) includes a first passive ring (2231), a second passive ring (2232), a first coupling rod (2233), a second coupling rod (2234), a first attitude adjustment drive mechanism (2235), and a second attitude adjustment drive mechanism (2236); the first passive ring (2231) and the second passive ring (2232) are coaxial and rotatably connected to the attitude adjustment base (222); and the puncture needle (211) passes through the first passive ring (2231) and the second passive ring (2232) along an axial direction of the first passive ring (2231); and
the first passive ring (2231) is rotatably connected to one end of the first coupling rod (2233); the other end of the first coupling rod (2233) is rotatably connected to the attitude adjustment base (222); the first attitude adjustment drive mechanism (2235) is further arranged at the other end of the first coupling rod (2233); the second passive ring (2232) is rotatably connected to one end of the second coupling rod (2234); the other end of the second coupling rod (2234) is rotatably connected to the attitude adjustment base (222); and the second attitude adjustment drive mechanism (2236) is further arranged at the other end of the second coupling rod (2234).

43. The puncture device (200) of claim 42, wherein the first attitude adjustment drive mechanism (2235) includes a sixth brake, a first reducer, a first attitude adjustment drive motor, and a sixth angle sensor; the sixth brake, the first reducer, the first attitude adjustment drive motor, and the sixth angle sensor are arranged at the other end of the first coupling rod (2233); and the sixth angle sensor is configured to detect a rotation angle of an output shaft of the first attitude adjustment drive motor; and/or,
the second attitude adjustment drive mechanism (2236) includes a seventh brake (22361), a second reducer (22362), a second attitude adjustment drive motor (22363), and a seventh angle sensor (22364); the seventh brake (22361), the second reducer (22362), the second attitude adjustment drive motor (22363), and the seventh angle sensor (22364) are arranged at the other end of the second coupling rod (2234); and the seventh angle sensor (22364) is configured to detect a rotation angle of an output shaft of the second attitude adjustment drive motor (22363).

44. The puncture device (200) of claim 41, wherein the puncture device (200) further comprises a second force sensor; the second force sensor is arranged between the puncture needle (211) and the attitude adjustment drive module (223); and the second force sensor is configured to acquire attitude adjustment force feedback information during attitude adjustment of the puncture needle (211); and
the attitude adjustment force feedback information is used to determine an attitude adjustment resistance output by the main hand control device (100).

45. The puncture device (200) of any one of claims 34 to 44, wherein a guiding structure (221) is arranged on the attitude control module (220); and the puncture needle (211) passes through the guiding structure (221).

46. The puncture device (200) of any one of claims 34 to 45, wherein the puncture device (200) further comprises at least one of an instrument verification control member and a manual switching control;
the instrument verification control member is configured to control the puncture device (200) after clamping the puncture needle (211) to enter an attitude adjustment state or a puncture state; and
the manual switching control is configured to control the puncture needle (211) to be manually released from the puncture device.

47. A robot, comprising the main hand control device (100) of claim 1 and the puncture device (200) of claim 34, wherein the puncture needle drive module (210) of the puncture device (200) drives the puncture needle (211) to move in response to a puncture execution signal from the puncture needle execution component (110) of the main hand control device (100).

48. The robot of claim 47, wherein the puncture needle execution component (110) includes a first position detection module (112); the first position detection module (112) is configured to acquire a position of the rod structure (111) in the axial direction of the rod structure (111); and the puncture execution signal includes the position of the rod structure (111) in the axial direction of the rod structure (111); and
the advance-retreat drive mechanism (213) includes an advance-retreat drive motor (2131); and the advance-retreat drive motor (2131) is configured to drive the puncture needle (211) to move a corresponding distance along the axial direction of the puncture needle (211) based on the position of the rod structure (111) in the axial direction of the rod structure (111).

49. The robot of claim 47 or 48, wherein the rod structure (111) is capable of rotating about the axial direction of the rod structure (111);
the puncture needle execution component (110) includes an angle detection component (114); the angle detection component (114) is configured to acquire a rotation angle of the rod structure (111) rotating about the axial direction of the rod structure (111); and the puncture execution signal includes the rotation angle; and
the rotary drive mechanism (212) includes a rotary drive motor (2121); and the rotary drive motor (2121) is configured to drive the puncture needle (211) to rotate a corresponding angle about the axial direction of the puncture needle (211) based on the rotation angle.

50. The robot of any one of claims 47 to 49, wherein the puncture device further includes a first force sensor (2124); the first force sensor (2124) is arranged between the puncture needle drive module (210) and the puncture needle; and the first force sensor (2124) is configured to acquire puncture force feedback information during puncture of the puncture needle (211);
the main hand control device (100) further includes a force feedback component (130); and the force feedback component (130) is configured to apply a puncture motion resistance along the axial direction of the rod structure (111) to the rod structure (111); and
the puncture motion resistance is determined based on the puncture force feedback information.

51. The robot of any one of claims 47 to 50, wherein the attitude control module (220) includes an attitude adjustment base (222) and an attitude adjustment drive module (223); the attitude adjustment drive module (223) is arranged on the attitude adjustment base (222); and the puncture needle drive module (210) is mounted to the attitude adjustment base (222) through the attitude adjustment drive module (223); and
the attitude adjustment drive module (223) adjusts an attitude of the puncture needle (211) relative to the attitude adjustment base (222) in response to an attitude adjustment signal from the attitude adjustment execution module (120) of the main hand control device (100).

52. The robot of claim 51, wherein
the attitude adjustment execution module (120) includes a first connecting rod (1211), a first rotating ring (1212), a second connecting rod (1221), a second rotating ring (1222), a base (123), a second angle sensor (1213), and a third angle sensor (1223); and
the first rotating ring (1212) and the second rotating ring (1222) are coaxial and arranged around the rod structure (111); one end of the first connecting rod (1211) is rotatably connected to the first rotating ring (1212); the other end of the first connecting rod (1211) is rotatably connected to the base (123); the second angle sensor (1213) is further arranged at the other end of the first connecting rod (1211); one end of the second connecting rod (1221) is rotatably connected to the second rotating ring (1222); the other end of the second connecting rod (1221) is rotatably connected to the base (123); and the third angle sensor (1223) is further arranged at the other end of the second connecting rod (1221).

53. The robot of claim 52, wherein a puncture device (200) further comprises a second force sensor, and the second force sensor is configured to obtain the attitude adjustment force feedback information during attitude adjustment of the puncture needle (211); and
the attitude adjustment drive module (223) is configured to apply the attitude adjustment resistance to a rod structure (111), and the attitude adjustment resistance is determined based on the attitude adjustment force feedback information.

54. The robot of claim 53, wherein
the attitude adjustment execution component (120) comprises a first motor and a second motor (1228), wherein the first motor is configured to apply attitude adjustment resistance to a first connecting rod (1211), and the second motor (1228) is configured to apply the attitude adjustment resistance to a second connecting rod (1221); the attitude adjustment resistance applied by the first motor to the first connecting rod (1211) and the attitude adjustment resistance applied by the second motor (1228) to the second connecting rod (1221) are both determined based on attitude adjustment force feedback information.

55. The robot of claim 52, wherein the attitude adjustment drive module (223) comprises a first passive ring (2231), a second passive ring (2232), a first coupling rod (2233), a second coupling rod (2234), a first attitude adjustment drive mechanism (2235), and a second attitude adjustment drive mechanism (2236), wherein the first passive ring (2231) and the second passive ring (2232) are coaxial and rotationally connected to an attitude adjustment base (222); and the puncture needle (211) passes through the first passive ring (2231) and the second passive ring (2232) along the axial direction of the first passive ring (2231);
the first passive ring (2231) is rotationally connected to an end of the first coupling rod (2233), an opposite end of the first coupling rod (2233) is rotationally connected to the attitude adjustment base (222), and the opposite end of the first coupling rod (2233) is further provided with the first attitude adjustment drive mechanism (2235); the second passive ring (2232) is rotationally connected to an end of the second coupling rod (2234), an opposite end of the second coupling rod (2234) is rotationally connected to the attitude adjustment base (222), and the opposite end of the second coupling rod (2234) is further provided with the second attitude adjustment drive mechanism (2236);
the attitude adjustment drive module (223) responds to an attitude adjustment signal of the attitude adjustment execution component (120) of the main hand control device (100) to adjust the attitude of the puncture needle (211) relative to the attitude adjustment base (222), wherein:
the first attitude adjustment drive mechanism (2235) drives the first coupling rod (2233) to rotate by a corresponding angle relative to the attitude adjustment base (222) based on an angle of rotation of the opposite end of the first connecting rod (1211) relative to a base (1241); and,
the second attitude adjustment drive mechanism (2236) drives the second coupling rod (2234) to rotate a corresponding angle relative to the attitude adjustment base (222) based on an angle of the other end of the second coupling rod (2234) rotating relative to the base (1241).

56. The robot of any one of claims 46 to 55, further comprising a mobile device (300), wherein the mobile device (300) is connected to the puncture device (200); and the mobile device (300) drives the puncture device (200) to move in a plurality of degrees of freedom.
